# EUROPEAN PATENT APPLICATION

(11) **EP 1 624 064 A2**
(43) Date of publication of application: **08.02.2006**
(21) Application number: 05019268.1
(22) Date of filing: 27.07.1999
(51) Int. Cl.: C12N 15/31, C07K 14/315, C07K 16/12, A61K 31/70, A61K 39/09, G01N 33/53, G01N 33/68, C12Q 1/68

(54) **Nucleic acids and proteins from streptococcus pneumoniae**

(30) Priority: 27.07.1998 GB 9816336; 19.03.1999 US 125329 P
(62) Divisional of application: 99934990.5
(71) Applicant: MICROBIAL TECHNICS LIMITED, Cambridge, CB2 1QA (GB)
(72) Inventor: Le Page, Richard William Falla, Cambridge CB2 1QP (GB); Wells, Jeremy Mark, Waterbeech, Cambridge CB5 9PB (GB); Hanniffy, Sean Bosco, Cambridge CB2 1QP (GB); Hansbro, Philip Michael, CBVT Dis. Immun. Microbio, Newcastle, NSW 2300 (AU)
(74) Representative: Chapman, Paul William

(57) **Abstract**

Novel proteins from *Streptococcus pneumoniae* are described, together with nucleic acid sequences encoding them. Their use in vaccines and in screening methods is also described.

## Description

The present invention relates to proteins derived from *Streptococcus pneumoniae,* nucleic acid molecules encoding such proteins, the use of the nucleic acid and/or proteins as antigens/immunogens and in detection/diagnosis, as well as methods for screening the proteins/nucleic acid sequences as potential anti-microbial targets.

*Streptococcus pneumoniae,* commonly referred to as the pneumococcus; is an important pathogenic organism. The continuing significance of *Streptoccocus pneumoniae* infections in relation to human disease in developing and developed countries has been authoritatively reviewed (Fiber, G.R., *Science,* **265**: 1385-1387 (1994)). That indicates that on a global scale this organism is believed to be the most common bacterial cause of acute respiratory infections, and is estimated to result in 1 million childhood deaths each year, mostly in developing countries (Starisfield, S.K., *Pediatr. Infect. Dis.,* 6: 622 (1987)). In the USA it has been suggested (Breiman *et al, Arch. Intern. Med. ,* **150**: 1401 (1990)) that the pneumococcus is still the most common cause of bacterial pneumonia, and that disease rates are particularly high in young children, in the elderly, and in patients with predisposing conditions such as asplenia, heart, lung and kidney disease, diabetes, alcoholism, or with immunosupressive disorders, especially AIDS. These groups are at higher risk of pneumococcal septicaemia and hence meningitis and therefore have a greater risk of dying from pneumococcal infection. The pneumococcus is also the leading cause of otitis media and sinusitis, which remain prevalent infections in children in developed countries, and which incur substantial costs.

The need for effective preventative strategies against pneumococcal infection is highlighted by the recent emergence of penicillin-resistant pneumococci. It has been reported that 6.6% of pneumoccal isolates in 13 US hospitals in 12 states were found to be resistant to penicillin and some isolates were also resistant to other antibiotics including third generation cyclosporins (Schappert, S.M., *Vital and Health Statistics of the Centres for Disease Control*/*National Centre for Health Statistics,* **214**:1 (1992)). The rates of penicillin resistance can be higher (up to 20%) in some hospitals (Breiman *et al,* J. Am. Med. Assoc., **271:** 1831 (1994)). Since the development of penicillin resistance among pneumococci is both recent and sudden, coming after decades during which penicillin remained an effective treatment, these findings are regarded as alarming.

For the reasons given above, there are therefore compelling grounds for considering improvements in the means of preventing, controlling, diagnosing or treating pneumococcal diseases.

Various approaches have been taken in order to provide vaccines for the prevention of pneumococcal infections. Difficulties arise for instance in view of the variety of serotypes (at least 90) based on the structure of the polysaccharide capsule surrounding the organism. Vaccines against individual serotypes are not effective against other serotypes and this means that vaccines must include polysaccharide antigens from a whole range of serotypes in order to be effective in a majority of cases. An additional problem arises because it has been found that the capsular polysaccharides (each of which determines the serotype and is the major protective antigen) when purified and used as a vaccine do not reliably induce protective antibody responses in children under two years of age, the age group which suffers the highest incidence of invasive pneumococcal infection and meningitis.

A modification of the approach using capsule antigens relies on conjugating the polysaccharide to a protein in order to derive an enhanced immune response, particularly by giving the response T-cell dependent character. This approach has been used in the development of a vaccine against *Haemophilus influenzae,* for instance. There are, however, issues of cost concerning both the multi-polysaccharide vaccines and those based on conjugates.

A third approach is to look for other antigenic components which offer the potential to be vaccine candidates. This is the basis of the present invention. Using a specially developed bacterial expression system, we have been able to identify a group of protein antigens from pneomococcus which are associated with the bacterial envelope or which are secreted.

Thus, in a first aspect the present invention provides a *Streptococcus pneumoniae* protein or polypeptide having a sequence selected from those shown in table 1.

In a second aspect, the present invention provides a *Streptococcus pneumoniae* protein or polypeptide having a sequence selected from those shown in table 2.

A protein or polypeptide of the present invention may be provided in substantially pure form. For example, it may be provided in a form which is substantially free of other proteins.

As discussed herein, the proteins and polypeptides of the invention are useful as antigenic material. Such material can be "antigenic" and/or "immunogenic". Generally, "antigenic" is taken to mean that the protein or polypeptide is capable of being used to raise antibodies or indeed is capable of inducing an antibody response in a subject. "Immunogenic" is taken to mean that the protein or polypeptide is capable of eliciting a protective immune response in a subject. Thus, in the latter case, the protein or polypeptide may be capable of not only generating an antibody response but, in addition, a non-antibody based immune response.

The skilled person will appreciate that homologues or derivatives of the proteins or polypeptides of the invention will also find use in the context of the present invention, ie as antigenic/immunogenic material. Thus, for instance proteins or polypeptides which include one or more additions, deletions, substitutions or the like are encompassed by the present invention. In addition, it may be possible to replace one amino acid with another of similar "type". For instance replacing one hydrophobic amino acid with another.
One can use a program such as the CLUSTAL program to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of identity analysis are contemplated in the present invention.

In the case of homologues and derivatives, the degree of identity with a protein or polypeptide as described herein is less important than that the homologue or derivative should retain the antigenicity or immunogenicity of the original protein or polypeptide. However, suitably, homologues or derivatives having at least 60% similarity (as discussed above) with the proteins or polypeptides described herein are provided. Preferably, homologues or derivatives having at least 70% similarity, more preferably at least 80% similarity are provided. Most preferably, homologues or derivatives having at least 90% or even 95% similarity are provided.

In an alternative approach, the homologues or derivatives could be fusion proteins, incorporating moieties which render purification easier, for example by effectively tagging the desired protein or polypeptide. It may be necessary to remove the "tag" or it may be the case that the fusion protein itself retains sufficient antigenicity to be useful.

In an additional aspect of the invention there are provided antigenic/immunogenic fragments of the proteins or polypeptides of the invention, or of homologues or derivatives thereof.

For fragments of the proteins or polypeptides described herein, or of homologues or derivatives thereof, the situation is slightly different. It is well known that is possible to screen an antigenic protein or polypeptide to identify epitopic regions, ie those regions which are responsible for the protein or polypeptide's antigenicity or immunogenicity. Methods for carrying out such screening are well known in the art. Thus, the fragments of the present invention should include one or more such epitopic regions or be sufficiently similar to such regions to retain their antigenic/immunogenic properties. Thus, for fragments according to the present invention the degree of identity is perhaps irrelevant, since they may be 100% identical to a particular part of a protein or polypeptide, homologue or derivative as described herein. The key issue, once again, is that the fragment retains the antigenic/immunogenic properties.

Thus, what is important for homologues, derivatives and fragments is that they possess at least a degree of the antigenicity/immunogenicity of the protein or polypeptide from which they are derived.

Gene cloning techniques may be used to provide a protein of the invention in substantially pure form. These techniques are disclosed, for example, in J. Sambrook *et al Molecular Cloning* 2nd Edition, Cold Spring Harbor Laboratory Press (1989). Thus, in a third aspect, the present invention provides a nucleic acid molecule comprising or consisting of a sequence which is:
(i) any of the DNA sequences set out in Table 1 or their RNA equivalents;
(ii) a sequence which is complementary to any of the sequences of (i);
(iii) a sequence which codes for the same protein or polypeptide, as those sequences of (i) or (ii);
(iv) a sequence which has substantial identity with any of those of (i), (ii) and (iii);
(v) a sequence which codes for a homologue, derivative or fragment of a protein as defined in Table 1.

In a fourth aspect the present invention provides a nucleic acid molecule comprising or consisting of a sequence which is:
(i) any of the DNA sequences set out in Table 2 or their RNA equivalents;
(ii) a sequence which is complementary to any of the sequences of (i);
(iii) a sequence which codes for the same protein or polypeptide, as those sequences of (i) or (ii);
(iv) a sequence which has substantial identity with any of those of (i), (ii) and (iii); or
(v) a sequence which codes for a homologue, derivative or fragment of a protein as defined in Table 2.

The nucleic acid molecules of the invention may include a plurality of such sequences, and/or fragments. The skilled person will appreciate that the present invention can include novel variants of those particular novel nucleic acid molecules which are exemplified herein. Such variants are encompassed by the present invention. These may occur in nature, for example because of strain variation. For example, additions, substitutions and/or deletions are included. In addition, and particularly when utilising microbial expression systems, one may wish to engineer the nucleic acid sequence by making use of known preferred codon usage in the particular organism being used for expression. Thus, synthetic or non-naturally occurring variants are also included within the scope of the invention.

The term "RNA equivalent" when used above indicates that a given RNA molecule has a sequence which is complementary to that of a given DNA molecule (allowing for the fact that in RNA "U" replaces "T" in the genetic code).

When comparing nucleic acid sequences for the purposes of determining the degree of homology or identity one can use programs such as BESTFIT and GAP (both from the Wisconsin Genetics Computer Group (GCG) software package) BESTFIT, for example, compares two sequences and produces an optimal alignment of the most similar segments. GAP enables sequences to be aligned along their whole length and finds the optimal alignment by inserting spaces in either sequence as appropriate. Suitably, in the context of the present invention when discussing identity of nucleic acid sequences, the comparison is made by alignment of the sequences along their whole length.

Preferably, sequences which have substantial identity have at least 50% sequence identity, desirably at least 75% sequence identity and more desirably at least 90 or at least 95% sequence identity with said sequences. In some cases the sequence identity may be 99 % or above.

Desirably, the term "substantial identity" indicates that said sequence has a greater degree of identity with any of the sequences described herein than with prior art nucleic acid sequences.

It should however be noted that where a nucleic acid sequence of the present invention codes for at least part of a novel gene product the present invention includes within its scope all possible sequence coding for the gene product or for a novel part thereof.

The nucleic acid molecule may be in isolated or recombinant form. It may be incorporated into a vector and the vector may be incorporated into a host. Such vectors and suitable hosts form yet further aspects of the present invention.

Therefore, for example, by using probes based upon the nucleic acid sequences provided herein, genes in *Streptococcus pneumoniae* can be identified. They can then be excised using restriction enzymes and cloned into a vector. The vector can be introduced into a suitable host for expression.

Nucleic acid molecules of the present invention may be obtained from *S.pneumoniae* by the use of appropriate probes complementary to part of the sequences of the nucleic acid molecules. Restriction enzymes or sonication techniques can be used to obtain appropriately sized fragments for probing.

Alternatively PCR techniques may be used to amplify a desired nucleic acid sequence. Thus the sequence data provided herein can be used to design two primers for use in PCR so that a desired sequence, including whole genes or fragments thereof, can be targeted and then amplified to a high degree.

Typically primers will be at least 15-25 nucleotides long.

As a further alternative chemical synthesis may be used. This may be automated. Relatively short sequences may be chemically synthesised and ligated together to provide a longer sequence.

There is another group of proteins from *S.pneumoniae* which have been identified using the bacterial expression system described herein. These are known proteins from *S.pneumoniae,* which have not previously been identified as antigenic proteins. The amino acid sequences of this group of proteins, together with DNA sequences coding for them are shown in Table 3. These proteins, or homologues, derivatives and/or fragments thereof also find use as antigens/immunogens. Thus, in another aspect the present invention provides the use of a protein or polypeptide having a sequence selected from those shown in Tables 1-3, or homologues, derivatives and/or fragments thereof, as an immunogen/antigen.

In yet a further aspect the present invention provides an immunogenic/antigenic composition comprising one or more proteins or polypeptides selected from those whose sequences are shown in Tables 1-3, or homologues or derivatives thereof, and/or fragments of any of these. In preferred embodiments, the immunogenic/antigenic composition is a vaccine or is for use in a diagnostic assay.

In the case of vaccines suitable additional excipients, diluents, adjuvants or the like may be included. Numerous examples of these are well known in the art.

It is also possible to utilise the nucleic acid sequences shown in Tables 1-3 in the preparation of so-called DNA vaccines. Thus, the invention also provides a vaccine composition comprising one or more nucleic acid sequences as defined herein. DNA vaccines are described in the art (see for instance, Donnelly *et al, Ann. Rev. Immunol.,* **15**:617-648 (1997)) and the skilled person can use such art described techniques to produce and use DNA vaccines according to the present invention.

As already discussed herein the proteins or polypeptides described herein, their homologues or derivatives, and/or fragments of any of these, can be used in methods of detecting/diagnosing *S.pneumoniae.* Such methods can be based on the detection of antibodies against such proteins which may be present in a subject. Therefore the present invention provides a method for the detection/diagnosis of *S.pneumoniae* which comprises the step of bringing into contact a sample to be tested with at least one protein, or homologue, derivative or fragment thereof, as described herein. Suitably, the sample is a biological sample, such as a tissue sample or a sample of blood or saliva obtained from a subject to be tested.

In an alternative approach, the proteins described herein, or homologues, derivatives and/or fragments thereof, can be used to raise antibodies, which in turn can be used to detect the antigens, and hence *S.pneumaniae.* Such antibodies form another aspect of the invention. Antibodies within the scope of the present invention may be monoclonal or polyclonal.

Polyclonal antibodies can be raised by stimulating their production in a suitable animal host (e.g. a mouse, rat, guinea pig, rabbit, sheep, goat or monkey) when a protein as described herein, or a homologue, derivative or fragment thereof, is injected into the animal. If desired, an adjuvant may be administered together with the protein. Well-known adjuvants include Freund's adjuvant (complete and incomplete) and aluminium hydroxide. The antibodies can then be purified by virtue of their binding to a protein as described herein.

Monoclonal antibodies can be produced from hybridomas. These can be formed by fusing myeloma cells and spleen cells which produce the desired antibody in order to form an immortal cell line. Thus the well-known Kohler & Milstein technique (*Nature* **256** (1975)) or subsequent variations upon this technique can be used.

Techniques for producing monoclonal and polyclonal antibodies that bind to a particular polypeptide/protein are now well developed in the art. They are discussed in standard immunology textbooks, for example in Roitt *et al, Immunology* second edition (1989), Churchill Livingstone, London.

In addition to whole antibodies, the present invention includes derivatives thereof which are capable of binding to proteins etc as described herein. Thus the present invention includes antibody fragments and synthetic constructs. Examples of antibody fragments and synthetic constructs are given by Dougall *et al* in *Tibtech* **12** 372-379 (September 1994).

Antibody fragments include, for example, Fab, F(ab')₂ and Fv fragments. Fab fragments (These are discussed in Roitt *et al* [*supra*]). Fv fragments can be modified to produce a synthetic construct known as a single chain Fv (scFv) molecule. This includes a peptide linker covalently joining Vₕ and Vₗ regions, which contributes to the stability of the molecule. Other synthetic constructs that can be used include CDR peptides. These are synthetic peptides comprising antigen-binding determinants. Peptide mimetics may also be used. These molecules are usually conformationally restricted organic rings that mimic the structure of a CDR loop and that include antigen-interactive side chains.

Synthetic constructs include chimaeric molecules. Thus, for example, humanised (or primatised) antibodies or derivatives thereof are within the scope of the present invention. An example of a humanised antibody is an antibody having human framework regions, but rodent hypervariable regions. Ways of producing chimaeric antibodies are discussed for example by Morrison *et al* in PNAS, **81**, 6851-6855 (1984) and by Takeda *et al* in Nature. **314,** 452-454 (1985).

Synthetic constructs also include molecules comprising an additional moiety that provides the molecule with some desirable property in addition to antigen binding. For example the moiety may be a label (e.g. a fluorescent or radioactive label). Alternatively, it may be a pharmaceutically active agent.

Antibodies, or derivatives thereof, find use in detection/diagnosis of *S.pneumoniae.* Thus, in another aspect the present invention provides a method for the detection/diagnosis of *S.pneumoniae* which comprises the step of bringing into contact a sample to be tested and antibodies capable of binding to one or more proteins described herein, or to homologues, derivatives and/or fragments thereof.

In addition, so-called "Affibodies" may be utilised. These are binding proteins selected from combinatorial libraries of an alpha-helical bacterial receptor domain (Nord *et al*,) Thus, Small protein domains, capable of specific binding to different target proteins can be selected using combinatorial approaches.

It will also be clear that the nucleic acid sequences described herein may be used to detect/diagnose *S.pneumoniae.* Thus, in yet a further aspect, the present invention provides a method for the detection/diagnosis of *S.pneumoniae* which comprises the step of bringing into contact a sample to be tested with at least one nucleic acid sequence as described herein. Suitably, the sample is a biological sample, such as a tissue sample or a sample of blood or saliva obtained from a subject to be tested. Such samples may be pre-treated before being used in the methods of the invention. Thus, for example, a sample may be treated to extract DNA. Then, DNA probes based on the nucleic acid sequences described herein (ie usually fragments of such sequences) may be used to detect nucleic acid from *S.pneumoniae.*

In additional aspects, the present invention provides:
(a) a method of vaccinating a subject against *S.pneumoniae* which comprises the step of administering to a subject a protein or polypeptide of the invention, or a derivative, homologue or fragment thereof, or an immunogenic composition of the invention;
(b) a method of vaccinating a subject against *S.pneumoniae* which comprises the step of administering to a subject a nucleic acid molecule as defined herein;
(c) a method for the prophylaxis or treatment of *S.pneumoniae* infection which comprises the step of administering to a subject a protein or polypeptide of the invention, or a derivative, homologue or fragment thereof, or an immunogenic composition of the invention;
(d) a method for the prophylaxis or treatment of *S.pneumoniae* infection which comprises the step of administering to a subject a nucleic acid molecule as defined herein;
(e) a kit for use in detecting/diagnosing *S.pneumoniae* infection comprising one or more proteins or polypeptides of the invention, or homologues, derivatives or fragments thereof, or an antigenic composition of the invention; and
(f) a kit for use in detecting/diagnosing *S.pneumoniae* infection comprising one or more nucleic acid molecules as defined herein.

Given that we have identified a group of important proteins, such proteins are potential targets for anti-microbial therapy. It is necessary, however, to determine whether each individual protein is essential for the organism's viability. Thus, the present invention also provides a method of determining whether a protein or polypeptide as described herein represents a potential anti-microbial target which comprises antagonising, inhibiting or otherwise interfering with the function or expression of said protein and determining whether *S.pneumoniae* is still viable.

A suitable method for inactivating the protein is to effect selected gene knockouts, ie prevent expression of the protein and determine whether this results in a lethal change. Suitable methods for carrying out such gene knockouts are described in Li *et al , P.N.A.S*., **94**:13251-13256 (1997) and Kolkman *et al ,* **178**:3736-3741 (1996).

In a final aspect the present invention provides the use of an agent capable of antagonising, inhibiting or otherwise interfering with the function or expression of a protein or polypeptide of the invention in the manufacture of a medicament for use in the treatment or prophylaxis of *S.pneumoniae* infection.

As mentioned above, we have used a bacterial expression system as a means of identifying those proteins which are surface associated, secreted or exported and thus, would find use as antigens.

The information necessary for the secretion/export of proteins has been extensively studied in bacteria. In the majority of cases, protein export requires a signal peptide to be present at the N-terminus of the precursor protein so that it becomes directed to the translocation machinery on the cytoplasmic membrane. During or after translocation, the signal peptide is removed by a membrane associated signal peptidase. Ultimately the localization of the protein (i.e. whether it be secreted, an integral membrane protein or attached to the cell wall) is determined by sequences other than the leader peptide itself.

We are specifically interested in surface located or exported proteins as these are likely to be antigens for use in vaccines, as diagnostic reagents or as targets for therapy with novel chemical entities. We have therefore developed a screening vector-system in *Lactococcus lactis* that permits genes encoding exported proteins to be identified and isolated. We provide below a representative example showing how given novel surface associated proteins from *Streptococcus pneumoniae* have been identified and characterized. The screening vector incorporates the staphylococcal nuclease gene *nuc* lacking its own export signal as a secretion reporter. Staphylococcal nuclease is a naturally secreted heat-stable, monomeric enzyme which has been efficiently expressed and secreted in a range of Gram positive bacteria (Shortle, *Gene*, 22:181-189 (1983); Kovacevic *et al., J. Bacteriol.,* 162:521-528 (1985); Miller *et al., J*. *Bacteriol.,* **169**:3508-3514 (1987); Liebl *et al., J. Bacteriol.,* **174**:1854-1861 (1992); Le Loir *et al., J. Bacteriol.,* **176**:5135-5139 (1994); Poquet *et al., J. Bacteriol*., **180**:1904-1912 (1998)).

Recently, Poquet *et al.* ((1998), *supra)* have described a screening vector incorporating the *nuc* gene lacking its own signal leader as a reporter to identify exported proteins in Gram positive bacteria, and have applied it to *L. lactis.* This vector (pFUN) contains the pAMβ1 replicon which functions in a broad host range of Gram-positive bacteria in addition to the ColE1 replicon that promotes replication in *Escherichia coli* and certain other Gram negative bacteria. Unique cloning sites present in the vector can be used to generate transcriptional and translational fusions between cloned genomic DNA fragments and the open reading frame of the truncated nuc gene devoid of its own signal secretion leader. The *nuc* gene makes an ideal reporter gene because the secretion of nuclease can readily be detected using a simple and sensitive plate test: Recombinant colonies secreting the nuclease develop a pink halo whereas control colonies remain white (Shortle, (1983), *supra;* Le Loir *et al.,* (1994), *supra).*

Thus, the invention will now be described with reference to the following representative example, which provides details of how the proteins, polypeptides and nucleic acid sequences described herein identified as antigenic targets.

We describe herein the construction of three reporter vectors and their use in L. *lactis* to identify and isolate genomic DNA fragments from *Streptococcus pneumoniae* encoding secreted or surface associated proteins. The invention will now be described with reference to the examples, which should not be construed as in any way limiting the invention. The examples refer to the figures in which:

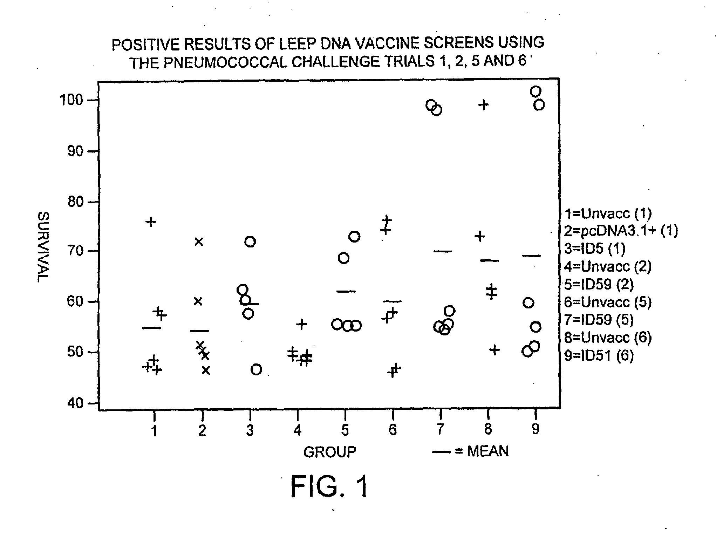

Figure 1: shows the results of a number of DNA vaccine trials; and
Figure 2: shows the results of further DNA vaccine trials.

### EXAMPLE 1

### (i) Construction of the pTREP1-nuc series of reporter vectors

### (a) Construction of expression plasmid pTREP1

The pTREP1 plasmid is a high-copy number (40-80 per cell) theta-replicating gram positive plasmid, which is a derivative of the pTREX plasmid which is itself a derivative of the previously published pIL253 plasmid. pIL253 incorporates the broad Gram-positive host range replicon of pAMβ1 (Simon and Chopin, *Biochimie,* 70:559-567 (1988)) and is non-mobilisable by the L *lactis* sex-factor. pIL253 also lacks the tra function which is necessary for transfer or efficient mobilisation by conjugative parent plasmids exemplified by pIL501. The Enterococcal pAMβ1 replicon has previously been transferred to various species including *Streptococcus, Lactobacillus* and *Bacillus* species as well as *Clostridium acetobutylicum,* (Oultram and Klaenhammer, FEMS *Microbiological Letters*, **27**:129-134 (1985); Gibson *et al.,* (1979); LeBlanc *et al*., *Proceedings of the Natianal Academy of Science USA*, **75**:3484-3487 (1978)) indicating the potential broad host range utility. The pTREP1 plasmid represents a constitutive transcription vector.

The pTREX vector was constructed as follows. An artificial DNA fragment containing a putative RNA stabilising sequence, a translation initiation region (TIR), a multiple cloning site for insertion of the target genes and a transcription terminator was created by annealing 2 complementary oligonucleotides and extending with Tfl DNA polymerase. The sense and anti-sense oligonucleotides contained the recognition sites for NheI and BamHI at their 5' ends respectively to facilitate cloning. This fragment was cloned between the XbaI and BamHI sites in pUC19NT7, a derivative of pUC19 which contains the T7 expression cassette from pLET1 (Wells *et al*, *J. Appl. Bacteriol*., **74**:629-636 (1993)) cloned between the EcoRI and HindIII sites. The resulting construct was designated pUCLEX. The complete expression cassette of pUCLEX was then removed by cutting with HindIII and blunting followed by cutting with EcoRI before cloning into EcoRI and SacI (blunted) sites of pIL253 to generate the vector pTREX (Wells and Schofield, *In Current advances in metabolism, genetics and applications-NATO ASI Series, H* 98:37-62 (1996)). The putative RNA stabilising sequence and TIR are derived from the *Escherichia coli* T7 bacteriophage sequence and modified at one nucleotide position to enhance the complementarity of the Shine Dalgarno (SD) motif to the ribosomal 16s RNA of *Lactococcus lactis* (Schofield et *al.* pers. coms. University of Cambridge Dept. Pathology.).

*A Lactococcus lactis* MG1363 chromosomal DNA fragment exhibiting promoter activity which was subsequently designated P7 was cloned between the EcoRI and BgIII sites present in the expression cassette, creating pTREX7. This active promoter region had been previously isolated using the promoter probe vector pSB292 (Waterfield *et al, Gene,* **165**:9-15 (1995)). The promoter fragment was amplified by PCR using the Vent DNA polymerase according to the manufacturer.

The pTREP1 vector was then constructed as follows. An artificial DNA fragment which included a transcription terminator, the forward pUC sequencing primer, a promoter multiple -cloning site region and a universal translation stop sequence was created by annealing two overlapping partially complementary synthetic oligonucleotides together and extending with sequenase according to manufacturers instructions. The sense and anti-sense (pTREP_{F} and pTREP_{R}) oligonucleotides contained the recognition sites for EcoRV and BamHI at their 5' ends respectively to facilitate cloning into pTREX7. The transcription terminator was that of the *Bacillus penicillinase* gene, which has been shown to be effective in Lactococcus (Jos *et al., Applied and Environmental Microbiology,* **50**:540-542 (1985)). This was considered necessary as expression of target genes in the pTREX vectors was observed to be leaky and is thought to be the result of cryptic promoter activity in the origin region (Schofield *et al.* pers. coms. University of Cambridge Dept. Pathology.). The forward pUC primer sequencing was included to enable direct sequencing of cloned DNA fragments. The translation stop sequence which encodes a stop codon in 3 different frames was included to prevent translational fusions between vector genes and cloned DNA fragments. The pTREX7 vector was first digested with EcoRI and blunted using the 5' - 3' polymerase activity of T4 DNA polymerase (NEB) according to manufacturer's instructions. The EcoRI digested and blunt ended pTREX7 vector was then digested with Bgl II thus removing the P7 promoter. The artificial DNA fragment derived from the annealed synthetic oligonucleotides was then digested with EcoRV and Bam HI and cloned into the EcoRI(blunted)-Bgl II digested pTREX7 vector to generate pTREP. A *Lactococcus lactis* MG1363 chromosomal promoter designated P1 was then cloned between the EcoRI and BglII sites present in the pTREP expression cassette forming pTREP1. This promoter was also isolated using the promoter probe vector pSB292 and characterised by Waterfield *et al.,* (1995), *supra.* The P1 promoter fragment was originally amplified by PCR using vent DNA polymerase according to manufacturers instructions and cloned into the pTREX as an EcoRI-BgIII DNA fragment. The EcoRI-BgIII P1 promoter containing fragment was removed from pTREX1 by restriction enzyme digestion and used for cloning into pTREP (Schofield *et al.* pers. coms. University of Cambridge, Dept. Pathology.).

### (b) PCR amplification of the S. aureus nuc gene.

The nucleotide sequence of the *S*. *aureus nuc* gene (EMBL database accession number V01281) was used to design synthetic oligonucleotide primers for PCR amplification. The primers were designed to amplify the mature form of the nuc gene designated nucA which is generated by proteolytic cleavage of the N-terminal 19 to 21 amino acids of the secreted propeptide designated Snase B (Shortle, (1983), *supra).* Three sense primers (nucS1, nucS2 and nucS3, Appendix 1) were designed, each one having a blunt-ended restriction endonuclease cleavage site for EcoRV or SmaI in a different reading frame with respect to the nuc gene. Additionally BglII and BamHI were incorporated at the 5' ends of the sense and anti-sense primers respectively to facilitate cloning into BamHI and BgIII cut pTREP1. The sequences of all the primers are given in Appendix 1. Three nuc gene DNA fragments encoding the mature form of the nuclease gene (NucA) were amplified by PCR using each of the sense primers combined with the anti-sense primer described above. The nuc gene fragments were amplified by PCR using *S*. *aureus* genomic DNA template, Vent DNA Polymerase (NEB) and the conditions recommended by the manufacturer. An initial denaturation step at 93 °C for 2 min was followed by 30 cycles of denaturation at 93 °C for 45 sec, annealing at 50 °C for 45 seconds, and extension at 73 °C for 1 minute and then a final 5 min extension step at 73 °C. The PCR amplified products were purified using a Wizard clean up column (Promega) to remove unincorporated nucleotides and primers.

### (c) Construction of the pTREP1-nuc vectors

The purified nuc gene fragments described in section b were digested with Bgl II and BamHI using standard conditions and ligated to BamHI and BgIII cut and dephosphorylated pTREP1 to generate the pTREP1-nuc1, pTREP1-nuc2 and pTREP1-nuc3 series of reporter vectors. General molecular biology techniques were carried out using the reagents and buffer supplied by the manufacture or using standard conditions(Sambrook and Maniatis, (1989), *supra).* In each of the pTREP1-nuc vectors the expression cassette comprises a transcription terminator, lactococcal promoter P1, unique cloning sites (BgIII, EcoRV or SmaI) followed by the mature form of the nuc gene and a second transcription terminator. Note that the sequences required for translation and secretion of the nuc gene were deliberately excluded in this construction. Such elements can only be provided by appropriately digested foreign DNA fragments (representing the target bacterium) which can be cloned into the unique restriction sites present immediately upstream of the *nuc* gene.

In possessing a promoter, the pTREP1-nuc vectors differ from the pFUN vector described by Poquet *et al.* (1998), *supra,* which was used to identify *L. lactis* exported proteins by screening directly for Nuc activity directly in L. *lactis.* As the pFUN vector does not contain a promoter upstream of the *nuc* open reading frame the cloned genomic DNA fragment must also provide the signals for transcription in addition to those elements required for translation initiation and secretion of Nuc. This limitation may prevent the isolation of genes that are distant from a promoter for example genes which are within polycistronic operons. Additionally there can be no guarantee that promoters derived from other species of bacteria will be recognised and functional in *L*. *lactis.* Certain promoters may be under stringent regulation in the natural host but not in *L. lactis.* In contrast, the presence of the P1 promoter in the pTREP1-nuc series of vectors ensures that promoterless DNA fragments (or DNA fragments containing promoter sequences not active in *L. lactis*) will still be transcribed.

### (d) Screening for secreted proteins in S. pneumoniae

Genomic DNA isolated from *S. pneumoniae* was digested with the restriction enzyme Tru9I. This enzyme which recognises the sequence 5'- TTAA -3' was used because it cuts A/T rich genomes efficiently and can generate random genomic DNA fragments within the preferred size range (usually averaging 0.5 - 1.0 kb). This size range was preferred because there is an increased probability that the P1 promoter can be utilised to transcribe a novel gene sequence. However, the P1 promoter may not be necessary in all cases as it is possible that many Streptococcal promoters are recognised *in L. lactis.* DNA fragments of different size ranges were purified from partial Tru9I digests of *S. pneumoniae* genomic DNA. As the Tru 9I restriction enzyme generates staggered ends the DNA fragments had to be made blunt ended before ligation to the EcoRV or SmaI cut pTREP1-nuc vectors. This was achieved by the partial fill-in enzyme reaction using the 5'-3' polymerase activity of Klenow enzyme. Briefly Tru9I digested DNA was dissolved in a solution (usually between 10-20 µl in total) supplemented with T4 DNA ligase buffer (New England Biolabs; NEB) (1X) and 33 µM of each of the required dNTPs, in this case dATP and dTTP. Klenow enzyme was added (1 unit Klenow enzyme (NEB) per µg of DNA) and the reaction incubated at 25°C for 15 minutes. The reaction was stopped by incubating the mix at 75°C for 20 minutes. EcoRV or SmaI digested pTREP-nuc plasmid DNA was then added (usually between 200-400 ng). The mix was then supplemented with 400 units of T4 DNA ligase (NEB) and T4 DNA ligase buffer (IX) and incubated overnight at 16°C. The ligation mix was precipitated directly in 100% Ethanol and 1/10 volume of 3M sodium acetate (pH 5.2) and used to transform *L. lactis* MG1363 (Gasson, 1983). Alternatively, the gene cloning site of the pTREP-nuc vectors also contains a BgIII site which can be used to clone for example Sau3AI digested genomic DNA fragments.
*L. lactis* transformant colonies were grown on brain heart infusion agar and nuclease secreting (Nuc⁺) clones were detected by a toluidine blue-DNA-agar overlay (0.05 M Tris pH 9.0, 10 g of agar per litre, 10 g of NaCl per liter, 0.1 mM CaCl2, 0.03% wt/vol. salmon sperm DNA and 90 mg of Toluidine blue O dye) essentially as described by Shortle, 1983, *supra* and Le Loir *et al*., 1994, *supra).* The plates were then incubated at 37°C for up to 2 hours. Nuclease secreting clones develop an easily identifiable pink halo. Plasmid DNA was isolated from Nuc⁺ recombinant *L. lactis* clones and DNA inserts were sequenced on one strand using the NucSeq sequencing primer described in Appendix 1, which sequences directly through the DNA insert.

### Isolation of Genes Encoding Exported Proteins from S. pneumoniae

A large number of gene sequences putatively encoding exported proteins in *S*. *pneumoniae* have been identified using the nuclease screening system. These have now been further analysed to remove artefacts. The sequences identified using the screening system have been analysed using a number of parameters.
1. All putative surface proteins were analysed for leader/signal peptide sequences using the software programs Sequencher (Gene Codes Corporation) and DNA Strider (Marck, *Nucleic Acids Res.,* **16**:1829-1836 (1988)). Bacterial signal peptide sequences share a common design. They are characterised by a short positively charged N-terminus (N region) immediately preceding a stretch of hydrophobic residues (central portion-h region) followed by a more polar C-terminal portion which contains the cleavage site (c-region). Computer software is available which allows hydropathy profiling of putative proteins and which can readily identify the very distinctive hydrophobic portion (h-region) typical of leader peptide sequences. In addition, the sequences were checked for the presence of or absence of a potential ribosomal binding site (Shine-Dalgarno motif) required for translation initiation of the putative nuc reporter fusion protein.
2. All putative surface protein sequences were also matched with all of the protein/DNA sequences using the publicly databases [OWL-proteins inclusive of SwissProt and GenBank translations]. This allows us to identify sequences similar to known genes or homologues of genes for which some function has been ascribed. Hence it has been possible to predict a function for some of the genes identified using the LEEP system and to unequivocally establish that the system can be used to identify and isolate gene sequences of surface associated proteins. We should also be able to confirm that these proteins are indeed surface related and not artifacts. The LEEP system has been used to identify novel gene targets for vaccine and therapy.
3. Some of the genes identified proteins did not possess a typical leader peptide sequence and did not show homology with any DNA/protein sequences in the database. Indeed these proteins may indicate the primary advantage of our screening method, i.e. the isolation of atypical surface-related proteins, which may have been missed in all previously described screening protocols or approaches based on sequence homology searches.

In all cases, only partial gene sequences were initially obtained. Full length genes were obtained in all cases by reference to the TIGR *S.pneumoniae* database (www@tigr.org). Thus, by matching the originally obtained partial sequences with the database, we were able to identify the full length gene sequences. In this way, as described herein, three groups of genes were clearly identified, ie a group of genes encoding previously unidentified *S.pneumoniae* proteins, a second group exhibiting some homology with known proteins from a variety of sources and a third group which encoded known *S.pneumoniae* proteins, which were, however, not known as antigens.

### Example 2: Vaccine trials

### pcDNA3.1 + as a DNA vaccine vector

### pcDNA3.1+

The vector chosen for use as a DNA vaccine vector was pcDNA3.1 (Invitrogen) (actually pcDNA3.1 +, the forward orientation was used in all cases but may be referred to as pcDNA3.1 here on). This vector has been widely and successfully employed as a host vector to test vaccine candidate genes to give protection against pathogens in the literature (Zhang, *et al*., Kurar and Splitter, Anderson *et al*.). The vector was designed for high-level stable and non-replicative transient expression in mammalian cells. pcDNA3.1 contains the ColE1 origin of replication which allows convenient high-copy number replication and growth in *E*. *coli.* This in turn allows rapid and efficient cloning and testing of many genes. The pcDNA3.1 vector has a large number of cloning sites and also contains the gene encoding ampicillin resistance to aid in cloning selection and the human cytomegalovirus (CMV) immediate-early promoter/enhancer which permits efficient, high-level expression of the recombinant protein. The CMV promoter is a strong viral promoter in a wide range of cell types including both muscle and immune (antigen presenting) cells. This is important for optimal immune response as it remains unknown as to which cells types are most important in generating a protective response *in vivo.* A T7 promoter upstream of the multiple cloning site affords efficient expression of the modified insert of interest and which allows *in vitro* transcription of a cloned gene in the sense orientation.

Zhang, D., Yang, X., Berry, J. Shen, C., McClarty, G. and Brunham, R.C. (1997) "DNA vaccination with the major outer-membrane protein genes induces acquired immunity to *Chlamydia trachomatis* (mouse pneumonitis) infection". *Infection and Immunity,* **176**, 1035-40.

Kurar, E. and Splitter, G.A. (1997) "Nucleic acid vaccination of *Brucella abortus* ribosomal *L7*/*L12* gene elicits immune response". *Vaccine,* **15,** 1851-57.

Anderson, R., Gao, X.-M., Papakonstantinopoulou, A., Roberts, M. and Dougan, G. (1996) "Immune response in mice following immunisation with DNA encoding fragment C of tetanus toxin". *Infection and Immunity,* **64**, 3168-3173.

### Preparation of DNA vaccines

Oligonucleotide primers were designed for each individual gene of interest derived using the LEEP system. Each gene was examined thoroughly, and where possible, primers were designed such that they targeted that portion of the gene thought to encode only the mature portion of the gene protein. It was hoped that expressing those sequences that encode only the mature portion of a target gene protein, would facilitate its correct folding when expressed in mammalian cells. For example, in the majority of cases primers were designed such that putative N-terminal signal peptide sequences would not be included in the final amplification product to be cloned into the pcDNA3.1 expression vector. The signal peptide directs the polypeptide precursor to the cell membrane via the protein export pathway where it is normally cleaved off by signal peptidase I (or signal peptidase II if a lipoprotein). Hence the signal peptide does not make up any part of the mature protein whether it be displayed on the surface of the bacteria surface or secreted. Where an N-terminal leader peptide sequence was not immediately obvious, primers were designed to target the whole of the gene sequence for cloning and ultimately, expression in pcDNA3.1.

Having said that, however, other additional features of proteins may also affect the expression and presentation of a soluble protein. DNA sequences encoding such features in the genes encoding the proteins of interest were excluded during the design of oligonucleotides. These features included:
1. LPXTG cell wall anchoring motifs,
2. LXXC ipoprotein attachment sites.
3. Hydrophobic C-terminal domain.
4. Where no N-terminal signal peptide or LXXC was present the start codon was excluded.
5. Where no hydrophobic C-terminal domain or LPXTG motif was present the stop codon was removed.

Appropriate PCR primers were designed for each gene of interest and any and all of the regions encoding the above features was removed from the gene when designing these primers. The primers were designed with the appropriate enzyme restriction site followed by a conserved Kozak nucleotide sequence (in most cases(NB except in occasional instances for example ID59) GCCACC was used. The Kozak sequence facilitates the recognition of initiator sequences by eukaryotic ribosomes) and an ATG start codon upstream of the insert of the gene of interest. For example the forward primer using a BamH1 site the primer would begin GCGGGATCCGCCACCATG followed by a small section of the 5' end of the gene of interest. The reverse primer was designed to be compatible with the forward primer and with a Not1 restriction site at the 5' end in most cases (this site is TTGCGGCCGC) (NB except in occasional instances for example ID59 where a Xho1 site was used instead of Not1).

### PCR primers

The following PCR primers were designed and used to amplify the truncated genes of interest.

ID5

Forward Primer 5'
CGGATCCGCCACCATGGGTCTAATTGAAGACTTAAAAAATCAA 3'
Reverse Primer 5' TTGCGGCCGCCAATGCTAGACTAAACACAAGACTCA 3'

ID59

Forward Primer 5' CGCGGATCCATGAAAAAAATCTATTCATTTTTAGCA 3'
Reverse Primer 5' CCCTCGAGGGCTACTTCCGATACATTTTAAACTGTAGG
3'

ID51

Forward Primer 5' CGGATCCGCCACCATGAGTCATGTCGCTGCAAATG 3'
Reverse Primer 5' TTGCGGCCGCATACCAAACGCTGACATCTACG 3'

ID29

Forward Primer 5' CGGATCCGCCACCATGCAAAAAGAGCGGTATGGTTATG
3'
Reverse Primer 5' TTGCGGCCGCACCCCCATTCTTAATCCCTT 3'

ID50

Forward Primer 5'
CGGATCCGCCACCATGGAGGTATGTGAAATGTCACGTAAA 3'
Reverse Primer 5' TTGCGGCCGCTTTACAAAGTCAAGCAAAGCC 3'

### Cloning

The insert along with the flanking features described above was amplified using PCR against a template of genomic DNA isolated from type 4 S. *pneumoniae* strain 11886 obtained from the National Collection of Type Cultures. The PCR product was cut with the appropriate restriction enzymes and cloned in to the multiple cloning site of pcDNA3.1 using conventional molecular biological techniques. Suitably mapped clones of the genes of interested were cultured and the plasmids isolated on a large scale (> 1.5 mg) using Plasmid Mega Kits (Qiagen). Successful cloning and maintenance of genes was confirmed by restriction mapping and sequencing ~700 base pairs through the 5' cloning junction of each large scale preparation of each construct.

### Strain validation

A strain of type 4 was used in cloning and challenge methods which is the strain from which the S. *pneumoniae* genome was sequenced. A freeze dried ampoule of a homogeneous laboratory strain of type 4 S. *pneumoniae* strain NCTC 11886 was obtained from the National Collection of Type Strains. The ampoule was opened and the cultured..re suspended with 0.5 ml of tryptic soy broth (0.5% glucose, 5% blood). The suspension was subcultured into 10 ml tryptic soy broth (0.5% glucose, 5% blood) and incubated statically overnight at 37°C. This culture was streaked on to 5% blood agar plates to check for contaminants and confirm viability and on to blood agar slopes and the rest of the culture was used to make 20% glycerol stocks. The slopes were sent to the Public Health Laboratory Service where the type 4 serotype was confirmed.

A glycerol stock of NCTC 11886 was streaked on a 5% blood agar plate and incubated overnight in a CO2 gas jar at 37°C. Fresh streaks were made and optochin sensitivity was confirmed.

### Pneumococcal challenge

A standard inoculum of type 4 S. *pneumoniae* was prepared and frozen down by passaging a culture of pneumococcus 1x through mice, harvesting from the blood of infected animals, and grown up to a predetermined viable count of around 10⁹ cfu/ml in broth before freezing down. The preparation is set out below as per the flow chart.

Streak pneumococcal culture and confirm identity
|
V
Grow over-night culture from 4-5 colonies on plate above
|
V
Animal passage pneumococcal culture
(i.p. injection of cardiac bleed to harvest)
|
V
Grow over-night culture from animal passaged pneumococcus
|
V
Grow day culture (to pre-determined optical density) from over-night of animal
passage and freeze down at -70°C - This is standard minimum
|
V
Thaw one aliquot of standard inoculum to viable count
|
V
Use standard inoculum to determine effective dose (called Virulence Testing)

|
V
All subsequent challenges - use standard inoculum to effective dose

An aliquot of standard inoculum was diluted 500x in PBS and used to inoculate the mice.

Mice were lightly anaesthetised using halothane and then a dose of 1.4 x 10⁵ cfu of pneumococcus was applied to the nose of each mouse. The uptake was facilitated by the normal breathing of the mouse, which was left to recover on its back.

### S. pneumoniae Vaccine trials

Vaccine trials in mice were carried out by the administration of DNA to 6 week old CBA/ca mice (Harlan, UK). Mice to be vaccinated were divided into groups of six and each group was immunised with recombinant pcDNA3.1 + plasmid DNA containing a specific target-gene sequence of interest. A total of 100 *µ*g of DNA in Dulbecco's PBS (Sigma) was injected intramuscularly into the tibialis anterior muscle of both legs (50 *µ*l in each leg). A boost was carried using the same procedure 4 weeks later. For comparison, control groups were included in all vaccine trials. These control groups were either unvaccinated animals or those administered with non-recombinant pcDNA3.1+ DNA (sham vaccinated) only, using the same time course described above. 3 weeks after the second immunisation, all mice groups were challenged intra-nasally with a lethal dose of S. *pneumoniae* serotype 4 (strain NCTC 11886). The number of bacteria administered was monitored by plating serial dilutions of the inoculum on 5% blood agar plates. A problem with intranasal immunisations is that in some mice the inoculum bubbles out of the nostrils, this has been noted in results table and taken account of in calculations. A less obvious problem is that a certain amount of the inoculum for each mouse may be swallowed.. It is assumed that this amount will be the same for each mouse and will average out over the course of inoculations. However, the sample sizes that have been used are small and this problem may have significant effects in some experiments. All mice remaining after the challenge were killed 3 or 4 days after infection. During the infection process, challenged mice were monitored for the development of symptoms associated with the onset of S. *pneumoniae* induced-disease. Typical symptoms in an appropriate order included piloerection, an increasingly hunched posture, discharge from eyes, increased lethargy and reluctance to move. The latter symptoms usually coincided with the development of a moribund state at which stage the mice were culled to prevent further suffering. These mice were deemed to be very close to death, and the time of culling was used to determine a survival time for statistical analysis. Where mice were found dead, the survival time was taken as the last time point when the mouse was monitored alive.

### Interpretation of Results

A positive result was taken as any DNA sequence that was cloned and used in challenge experiments as described above which gave protection against that challenge. Protection was taken as those DNA sequences that gave statistically significant protection (to a 95% confidence level (p < 0.05)) and also those which were marginal or close to significant using Mann-Whitney or which show some protective features for example there were one or more outlying mice or because the time to the first death was prolonged. It is acceptable to allow marginal or non-significant results to be considered as potential positives when it is considered that the clarity of some of the results may be clouded by the problems associated with the administration of intranasal infections.

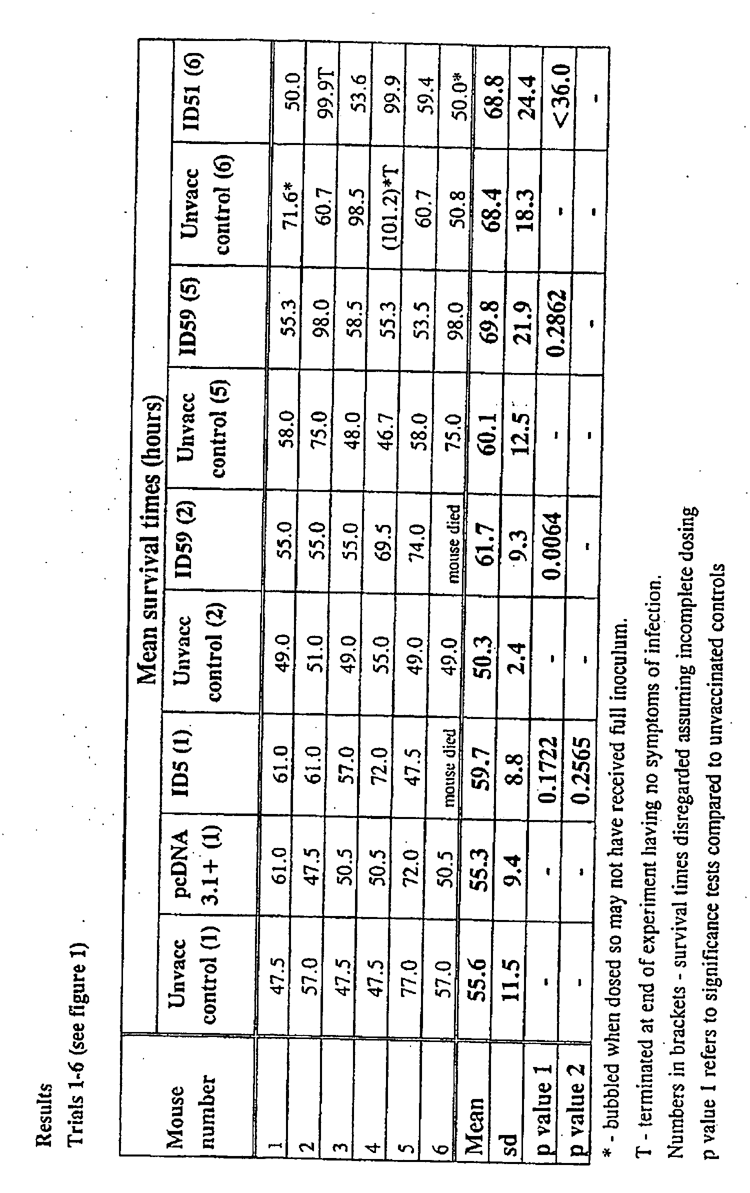

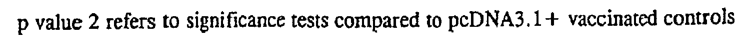

### Statistical Analyses.

Trial 1 - None of the other groups had significantly longer survival times than the controls. The survival times of the unvaccinated and pcDNA3.1 control groups were not significantly different. One of the mice from ID5 was an outlying result and the mean survival times for ID5 were extended but not significantly so.
Trial 2 - The group vaccinated with ID59 had significantly longer survival times than the unvaccinated control group.
Trial 5 - The group vaccinated with ID59 again survived for an average of almost 10 hours longer than the controls but the results were not quite statistically significant.
Trial 6 - The group vaccinated with ID51 did not have survival times significantly higher than unvaccinated controls (p= <36.0), however, there were 2 outlying mice in the vaccinated group.

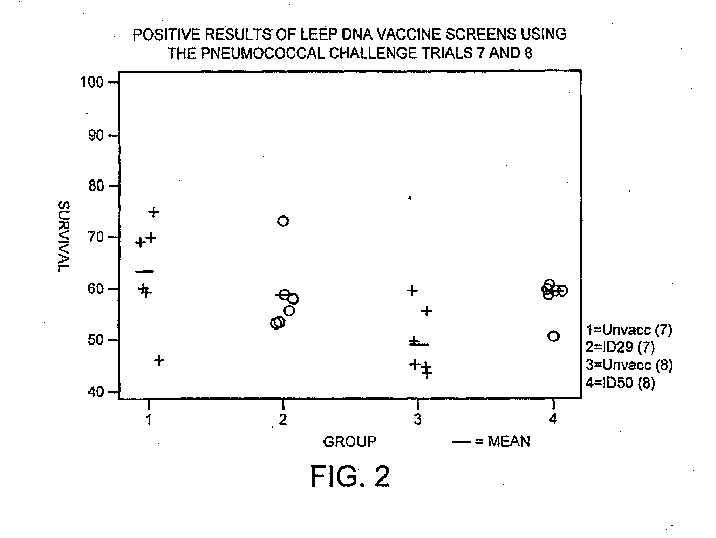

### Vaccine trials 7 and 8 (See figure 2)

| **Mouse number** | **Mean survival times (hours)** | | | |
|---|---|---|---|---|
| | **Unvacc control (7)** | **ID29 (7)** | **Unvacc control (8)** | **ED50 (8)** |
| 1 | 59.6 | 73.1 | 45.1 | 60.6 |
| 2 | 47.2 | 54.8 | 50.8 | 60.6 |
| 3 | 59.6 | 59.3 | 60.4 | 51.1 |
| 4 | 70.9 | 54.8* | 55.2 | 60.6 |
| 5 | 68.6* | 59.3 | 45.1 | 60.6 |
| 6 | 76.0 | 54.8 | 45.1 | 60.6 |
| **Mean** | **63.6** | **59.35** | **50.2** | **59.1** |
| **sd** | **10.3** | **7.1** | **6.4** | **3.9** |
| **p value 1** | **-** | **< 39.0** | **-** | **0.0048** |

| | | | | |
|---|---|---|---|---|
| * - bubbled when dosed so may not have received full inoculum. | | | | |
| T - terminated at end of experiment having no symptoms of infection. | | | | |

Numbers in brackets - survival times disregarded assuming incomplete dosing p value 1 refers to significance tests compared to unvaccinated controls

### Statistical Analyses.

Trial 7 - The ID29 vaccinated group showed prolonged times to the first death. T Trial 8 - The group vaccinated with ID50 survived significantly longer than unvaccinated controls.

Appendix I - Oligonucleotide primers

nucS1
Bgl II Eco RV
5'- cgagatctgatatctcacaaacagataacggcgtaaatag -3'

nucS2
Bgl II Sma I
5'- gaagatcttcccgggatcacaaacagataacggcgtaaatag -3'

nucS3
Bgl II Eco RV
5'- cgagatctgatatccatcacaaacagataacggcgtaaatag -3'

nucR
Bam HI
5'- cgggatccttatggacctgaatcagcgttgtc -3'

NucSeq
5'- ggatgctttgtttcaggtgtatc -3'

pTREPF
5'- catgatatcggtacctcaagctcatatcattgtccggcaatggtgtgggctttttttgttttagcggataa
caatttcacac -3'

pTREPR
5'- gcggatcccccgggcttaattaatgtttaaacactagtcgaagatctcgcgaattctcctgtgtgaaatt
gttatccgcta -3'

pUCF
5'- cgccagggttttcccagtcacgac -3'

VR
5'- tcagggggcggagcctatg -3'

V1
5'- tcgtatgttgtgtggaattgtg -3'

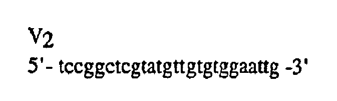

TABLE 1

ID4 1200 bp

ATGAGAAATATGTGGGTTGTAATCAAGGAAACCTATCTTCGACATGTCGAGTCATGGAGTTTCTTCTTTATGGTGA
TTTCGCCGTTCCTCTTTTTAGGAATCTCTGTAGGAATTGGGCATCTCCAAGGTTCTTCTATGGCTAAAAATAATAA
AGTGGCAGTAGTGACAACAGTGCCATCTGTAGCAGAAGGACTGAAGAATGTAAATGGTGTTAACTTCGACTATAA
AGACGAAGCAAGTGCCAAAGAAGCAATTAAAGAAGAAAAATTAAAAGGTTATTTGACCATTGATCAAGAAGATA
GTGTTCTAAAGGCAGTTTATCATGGCGAAACATCGCTTGAAAATGGAATTAAATTTGAGGTTACAGGTACACTCA
ATGAACTGCAAAATCAGCTTAATCGTTCAACTGCTTCCTTGTCTCAAGAGCAGGAAAAACGCTTAGCGCAGACAA
TTCAATTCACAGAAAAGATTGATGAAGCCAAGGAAAATAAAAAGTTTATTCAAACAATTGCAGCAGGTGCCTTAG
GATTCTTTCTTTATATGATTCTGATTACCTATGCGGGTGTAACAGCTCAGGAAGTTGCCAGTGAAAAAGGCACCAA
AATTATGGAAGTCGTTTTTTCTAGCATAAGGGCAAGTCACTATTTCTATGCGCGGATGATGGCTCTGTTTCTAGTG
ATTTTAACGCATATTGGGATCTATGTTGTAGGTGGTCTGGCTGCCGTTTTGCTCTTTAAAGATTTGCCATTCTTGGC
TCAGTCTGGTATTTTGGATCACTTGGGAGATGCTATCTCACTGAATACCTTGCTCTTTATTTTGATCAGTCTTTTCA
TGTACGTAGTCTTGGCAGCCTTCCTAGGATCTATGGTTTCTCGTCCTGAGGACTCAGGGAAAGCCTTGTCGCCTTT
GATGATTTTGATTATGGGTGGTTTTTTTGGAGTGACAGCTCTAGGTGCAGCTGGTGACAATCTCCTCTTGAAGATT
GGTTCTTATATTCCCTTTATTTCGACCTTCTTTATGCCGTTTCGAACGATTAATGACTATGCGGGGGGAGCAGAAG
CATGGATTTCACTTGCTATTACAGTGATTTTTGCGGTGGTAGCAACAGGATTTATCGGACGCATGTATGCTAGTCT
CGTTCTTCAAACGGATGATTTAGGGATTTGGAAAACCTTTAAACGTGCCTTATCTTATAAATAG

MRNMWVVIKETYLRHVESWSFFFMVISPFLFLGISVGIGHLQGSSMAKNNKVAVVTTVPSVAEGLKNVNGVNFDYKD
EASAKEAIKEEKLKGYLTIDQEDSVLKAVYHGETSLENGIKFEVTGTLNELQNQLNRSTASLSQEQEKRLAQTIQFTEKI
DEAKENKKFIQTIAAGALGFFLYMILITYAGVTAQEVASEKGTKIMEVVFSSIRASHYFYARMMALFLVILTHIGIYVVG
GLAAVLLFKDLPFLAQSGILDHLGDAISLNTLLFILISLFMYVVLAAFLGSMVSRPEDSGKALSPLMILIMGGFFGVTALG
AAGDNLLLKIGSYIPFISTFFMPFRTINDYAGGAEAWISLAITVIFAVVATGFIGRMYASLVLQTDDLGIWKTFKRALSYK
Z

ID5 1125 bp

CCTGGGAAAGTCTTGAAAATTATGATAGAATGGTGGAAGGAAAAATTCAGGAGAGTAGTAGTGACTCAAAATGTT
GAAAGTCTTCTCGTATCCATTGTAATCAGTGCATACAATGAAGAAAAATATCTGCCTGGTCTAATTGAAGACTTAA
AAAATCAAACCTATCCTAAAGAGGATATTGAAATTCTATTTATAAATGCTATGTCCACAGATGGGACCACAGCTA
TCATTCAGCAATTTATAAAGGAAGATACAGAGTTTAACTCAATTAGATTGTATAACAATCCTAAGAAAAATCAAG
CTAGTGGTTTTAACCTGGGAGTTAAACATTCTGTAGGGGACCTTATTTTAAAAATTGATGCTCATTCAAAAGTTAC
TGAGACTTTTGTAATGAACAATGTGGCTATTATTCAACAAGGTGAATTTGTCTGTGGGGGGCCTAGACCGACGATT
GTCGAAGGAAAAGGAAAATGGGCAGAGACCTTGCATCTTGTTGAGGAAAATATGTTTGGCAGTAGCATTGCCAAT
TATCGAAATAGTTCTGAGGATAGATATGTTTCTTCTATTTTTCATGGAATGTATAAACGAGAGGTTTTCCAGAAGG
TTGGTTTAGTAAATGAGCAACTTGGCCGAACTGAAGATAATGATATTCATTATAGAATTCGAGAATATGGTTATAA
AATCCGCTATAGCCCAAGTATTCTATCTTATCAGTATATTCGACCAACATTCAAGAAAATGCTGCATCAAAAGTAT
TCAAATGGTTTGTGGATTGGCTTGACAAGTCATGTTCAGTTTAAGTGTTTATCATTATTTCACTATGTTCCTTGTTT
ATTTGTTTTGAGTCTTGTGTTTAGTCTAGCATTGTTACCGATCACATTCGTATTCATAACTTTACTATTAGGTGCCT
ATTTTCTACTTTTGTCATTACTCACTTTGCTGACTTTATTAAAACATAAAAATGGATTTCTAATTGTGATGCCCTTT
ATTTTATTTTCCATTCACTTTGCTTATGGCCTTGGGACGATTGTAGGTTTAATTAGAGGATTTAAATGGAAGAAGG
AGTACAAGAGAACAATAATTTATTTGGATAAAATAAGCCAAATAAATCAAAATATGCTATAA

PGKVLKIMIEWWKEKFRRVVVTQNVESLLVSIVISAYNEEKYLPGLIEDLKNQTYPKEDIEILFINAMSTDGTTAIIQQFIK
EDTEFNSIRLYNNPKKNQASGFNLGVKHSVGDLILKIDAHSKVTETFVMNNVAIIQQGEFVCGGPRPTIVEGKGKWAET
LHLVEENMFGSSIANYRNSSEDRYVSSIFHGMYKREVFQKVGLVNEQLGRTEDNDIHYRIREYGYKIRYSPSILSYQYIRP
TFKKMLHQKYSNGLWIGLTSHVQFKCLSLFHYVPCLFVLSLVFSLALLPITFVFITLLLGAYFLLLSLLTLLTLLKHKNGF
LIVMPFILFSIHFAYGLGTIVGLIRGFKWKKEYKRTIIYLDKISQINQNMLZ

ID11 696 bp

ATGATGAAAGAACAAAATACGATAGAAATCGATGTATTTCAATTAGTTAAAAGCTTGTGGAAACGCAAGCTAATG
ATTTTAATAGTGGCACTTGTGACAGGTGCGGGGGCTTTTGCATATAGCACTTTTATTGTTAAGCCAGAATATACGA
GTACCACGCGAATTTACGTAGTGAATCGCAATCAAGGAGACAAGCCGGGGTTGACAAATCAGGATTTGCAGGCAG
GAACTTATCTGGTAAAAGACTACCGTGAGATTATCCTTTCGCAGGATGTTTTGGAGGAAGTTGTTTCTGATTTGAA
ACTAGATTTGACGCCAAAAGGTTTGGCTAATAAAATTAAAGTGACAGTACCAGTTGATACCCGTATTGTCTCTATT
TCAGTTAATGATCGAGTTCCTGAAGAGGCAAGCCGTATCGCTAACTCTTTGAGAGAAGTAGCTGCTCAAAAAATT
ATCAGTATTACTCGTGTTTCTGACGTGACAACACTGGAGGAGGCAAGGCCGGCGATATCCCCGTCTTCGCCAAAT
ATTAAACGCAATACACTAATTGGTTTTTTGGCAGGGGTGATTGGAACTAGTGTTATAGTTCTTCATCTTGAACTTTT

GGATACTCGTGTGAAACGTCCGGAAGATATCGAAAATACATTGCAGATGACACTTTTGGGAGTTGTGCCAAACTT
GGGTAAGTTGAAATAG

MMKEQNTIEIDVFQLVKSLWKRKLMILIVALVTGAGAFAYSTFIVKPEYTSTTRIYVVNRNQGDKPGLTNQDLQAGTYL
VKDYREIILSQDVLEEVVSDLKLDLTPKGLANKIKVTVPVDTRIVSISVNDRVPEEASRIANSLREVAAQKIISITRVSDVT
TLEEARPAISPSSPNIKRNTLIGFLAGVIGTSVIVLHLELLDTRVKRPEDIENTLQMTLLGVVPNLGKLKZ

ID19 555 bp

ATGGTAAAAGTAGCAGTTATATTAGCTCAGGGCTTTGAAGAAATTGAAGCCTTGACAGTTGTAGATGTCTTGCGTC
GAGCCAATATCACATGTGATATGGTTGGTTTTGAAGAGCAAGTAACGGGTTCGCATGCAATCCAAGTAAGAGCAG
ATCATGTCTTTGATGGAGATTTATCAGACTATGATATGATTGTTCTTCCTGGAGGTATGCCTGGTTCTGCACATTTA
CGTGATAATCAGACCTTGATTCAAGAATTGCAAAGCTTCGAGCAAGAAGGGAAGAAACTAGCAGCCATTTGTGCG
GCACCAATTGCCCTCAATCAAGCAGAGATATTGAAAAATAAGCGATACACTTGTTATGACGGCGTTCAAGAGCAA
ATCCTTGATGGTCACTACGTCAAGGAAACAGTAGTGGTAGATGGTCAGTTGACAACCAGTCGGGGTCCTTCAACA
GCCCTTGCCTTTGCCTACGAGTTGGTGGAGCAACTAGGAGGGGACGCAGAGAGTTTACGAACAGGAATGCTCTAT
CGAGATGTCTTTGGTAAAAATCAGTAA

MVKVAVILAQGFEEIEALTVVDVLRRANITCDMVGFEEQVTGSHAIQVRADHVFDGDLSDYDMIVLPGGMPGSAHLR
DNQTLIQELQSFEQEGKKLAAICAAPIALNQAEILKNKRYTCYDGVQEQILDGHYVKETVVVDGQLTTSRGPSTALAFA
YELVEQLGGDAESLRTGMLYRDVFGKNQZ

ID27 306 bp

GTGGTAGGGATGGTAGAACCAAACCTAGAAAGCCTTATAAAAGATCTTTACAATCATGCTCGACATGATTTGAGT
GAAGATTTAGTTGCTGCTCTCCTAGAGACTACTAAAAAACTGCCTACTACAAATGAGCAATTGCAGGCAGTTCGTC
TCTCAGGCCTGGTCAATCGTGAATTGCTCCTAAATCCCAAACATCCAGCACCTGAGTTGCTCAACTTGGCTCGCTT
TGTCAAAAGAGAAGAAGCCAAGTACAGAGGAACTGCGACTTCTGCGCTTATGTATGAGGAACTCTTTAAAATGCT
TTGA

MVGMVEPNLESLIKDLYNHARHDLSEDLVAALLETTKKLPTTNEQLQAVRLSGLVNRELLLNPKHPAPELLNLARFVK
REEAKYRGTATSALMYEELFKMLZ

ID29 945 bp

TTGTTCTTAAAAAAGGAAAGAGAGGTAATCAGCATGCGTAAATGGACAAAAGGATTTCTCATCTTTGGTGTGGTG
ACTACCGTTATCGGCTTTATCCTGCTTTTTGTAGGTATCCAATCTGACGGGA[?]TAAGAGCCTACTTTCCATGTCCAA
AGAACCTGTCTATGATAGCCGTACGGAAAAGCTAACCTTTGGCAAGGAAGTCGAAAACCTAGAAATTACTCTCCA
CCAACACACGCTCACCATCACAGACTCTTTCGATGATCAAATCCACATTTCTTACCATCCATCTCTTTCTGCTCAC
CATGATCTTATCACCAATCAGAACGATAGAACTCTGAGTCTCACTGATAAGAAACTGTCTGAAACTCCGTTTCTCT
CTTCTGGAATTGGTGGGATTCTTCATATCGCAAGTAGCTACTCTAGTCGTTTTGAAGAAGTTATTCTCCGACTACC
AAAAGGGAGAACTCTAAAAGGGATCAACATCTCAGCCAATCGCGGACAAACCACCATCATAAATGCTAGCCTTGA
AAATGCGACCCTCAATACAAACAGCTATATCCTCCGAATTGAAGGAAGTCGTATCAAAAACAGTAAACTCACAAC
GCCCAATATCGTTAATATCTTTGATACAGTTCTTACAGATAGTCAGCTAGAGTCAACAGAGAATCACTTCCACGCT
GAAAATATCCAAGTCCATGGCAAGGTTGAACTGACTGCCAAAGATTATCTCAGAATCATCCTAGACCAGAAAGAA
AGCCAACGAATTAACTGGGACATCTCAAGCAACTATGGTTCTATCTTCCAATTCACAAGAGAAAAGCCTGAATCA
AGAGGTACGGAATTAAGCAACCCTTACAAAACTGAAAAAACCGATGTCAAGGATCAACTCATTGCGAGATCTGAT
GATAATATTGATCTAATATCCACACCAAGCAGACGTTGA

MFLKKEREVISMRKWTKGFLIFGVVTTVIGFILLFVGIQSDGIKSLLSMSKEPVYDSRTEKLTFGKEVENLEITLHQHTLTI
TDSFDDQIHISYHPSLSAHHDLITNQNDRTLSLTDKKLSETPFLSSGIGGILHIASSYSSRFEEVILRLPKGRTLKGINISANR
GQTTIINASLENATLNTNSYILRIEGSRIKNSKLTTPNIVNIFDTVLTDSQLESTENHFHAENIQVHGKVELTAKDYLRIILD
QKESQRINWDISSNYGSIFQFTREKPESRGTELSNPYKTEKTDVKDQLIARSDDNIDLISTPSRRZ

ID30 879 bp

ATGAAACAAGAATGGTTTGAAAGTAATGATTTTGTAAAAACAACAAGCAAGAACAAGCCTGAAGAGCAAGCTCA
AGAGGTTGCAGACAAGGCTGAAGAAACGATAGCCGATCTCGATACACCAATTGAAAAAAATACTCAGTTAGAGG
AGGAAGTCCCTCAAGCTGAAGTCGAATTGGAAAGCCAGCAAGAAGAGAAAATTGAAGCTCCTGAAGACAGTGAA
GCGAGAACAGAAATAGAAGAAAAGAAGGCATCTAATTCTACTGAAGAAGAGCCAGACCTTTCTAAAGAAACAGA
AAAAGTCACTATAGCTGAAGAGAGCCAAGAAGCTCTTCCTCAGCAAAAAGCAACCACGAAAGAGCCACTTCTTAT
CAGTAAATCTTTAGAAAGTCCTTATATCCCCGACCAAGCTCCAAAATCTAGGGATAAATGGAAAGAGCAAGTGCT
TGATTTTTGGTCTTGGCTAGTGGAAGCGATCAAATCTCCTACAAGTAAGTTGGAAACAAGTATCACACACAGTTAC
ACAGCCTTTCTCTTGCTCATTCTGTTTTCTGCATCTTCCTTTTTCTTTAGTATCTATCACATCAAACATGCTTACTAT
GGACATATAGCAAGCATTAACAGTCGCTTCCCTGAGCAGCTAGCTCCTTTAACTCTTTTTTCTATCATCTCTATCCT

AGTAGCGACAACACTCTTCTTCTTTTCATTCCTCTTGGGTAGTTTCGTTGTGAGACGATTTATCCACCAGGAAAAG
GACTGGACGCTAGACAAGGTTCTCCAACAATATAGTCAACTCTTGGCAATTCCAATCTCCTCACTGCTATTGCTAG
TTTCTTTGCTTTCTTTGATAGCCTACGATTTACAGCCCTCTTGTGTGTGA

MKQEWFESNDFVKTTSKNKPEEQAQEVADKAEETIADLDTPIEKNTQLEEEVPQAEVELESQQEEKIEAPEDSEARTEIE
EKKASNSTEEEPDLSKETEKVTIAEESQEALPQQKATTKEPLLISKSLESPYIPDQAPKSRDKWKEQVLDFWSWLVEAIKS
PTSKLETSITHSYTAFLLLILFSASSFFFSIYHIKHAYYGHIASINSRFPEQLAPLTLFSIISILVATTLFFFSFLLGSFVVRRFIH
QEKDWTLDKVLQQYSQLLAIPISSLLLLVSLLSLIAYDLQPSCVZ

ID105 990 bp

ATGCAACTCGCTTCTTCGGTCTACTCATTGTTCGTCTGGTACAATTTGTTCTTAAAAAAGGAAAGAGAGGTAATCA
GCATGCGTAAATGGACAAAAGGATTTCTCATCTTTGGTGTGGTGACTACCGTTATCGGCTTTATCCTGCTTTTTGTA
GGTATCCAATCTGACGGGATTAAGAGCCTACTTTCCATGTCCAAAGAACCTGTCTATGATAGCCGTACGGAAAAG
CTAACCTTTGGCAAGGAAGTCGAAAACCTAGAAATTACTCTCCACCAACACACGCTCACCATCACAGACTCTTTC
GATGATCAAATCCACATTTCTTACCATCCATCTCTTTCTGCTCACCATGATCTTATCACCAATCAGAACGATAGAA
CTCTGAGTCTCACTGATAAGAAACTGTCTGAAACTCCGTTTCTCTCTTCTGGAATTGGTGGATTCTTCATATCGC
AAGTAGCTACTCTAGTCGTTTTGAAGAAGTTATTCTCCGACTACCAAAAGGGAGAACTCTAAAAGGGATCAACAT
CTCAGCCAATCGCGGACAAACCACCATCATAAATGCTAGCCTTGAAAATGCGACCCTCAATACAAACAGCTATAT
CCTCCGAATTGAAGGAAGTCGTATCAAAAACAGTAAACTCACAACGCCCAATATCGTTAATATCTTTGATACAGTT
CTTACAGATAGTCAGCTAGAGTCAACAGAGAATCACTTCCACGCTGAAAATATCCAAGTCCATGGCAAGGTTGAA
CTGACTGCCAAAGATTATCTCAGAATCATCCTAGACCAGAAAGAAAGCCAACGAATTAACTGGGACATCTCAAGC
AACTATGGTTCTATCTTCCAATTCACAAGAGAAAAGCCTGAATCAAGAGGTACGGAATTAAGCAACCCTTACAAA
ACTGAAAAAACCGATGTCAAGGATCAACTCATTGCGAGATCTGATGATAATATTGATCTAATATCCACACCAAGC
AGACGTTGA

MQLASSVYSLFVWYNLFLKKEREVISMRKWTKGFLIFGVVTTVIGFILLFVGIQSDGIKSLLSMSKEPVYDSRTEKLTFG
KEVENLEITLHQHTLTITDSFDDQIHISYHPSLSAHHDLITNQNDRTLSLTDKKLSETPFLSSGIGGFFISQVATLVVLKKLFSDYQKGELZKGINISANRGQTTIINASLENATLNTNSYILRIEGSRIKNSKLTTPNIVNIFDTVLTDSQLESTENHFHAENIQV
HGKVELTAKDYLRIILDQKESQRINWDISSNYGSIFQFTREKPESRGTELSNPYKTEKTDVKDQLIARSDDNIDLISTPSRR
Z

ID107 -78bp

ATGATATGTAAAATGAAGCAGGGAGGGAGCAGGGCGTGCTGGGGATGGAGAGTGGGGGAGGGACGCTGCTATTT
TAATC

MICKMKQGGSRACWGWRVGEGRCYFN

ID109 714 bp

CGATAAAGAGGCCTTGAGTAATCTCAATTTGCAGATTGAAAATGGAGAGATTATGGGCTTGATTGGTCATAATGG
GGCTGGAAAATCGACCACTATAAAATCCCTAGTCAGTATCATTTCACCCAGCAGTGGTCGTATTTTGGTAGACGGT
CAGGAGTTATCGGAAAATCGCTTGGCTATTAAACGAAAGATTGGCTACGTAGCAGACTCGCCTGACTTATTTTTAC
GCTTAACGGCCAATGAATTTTGGGAATTGATCGCCTCATCCTATGATCTGAGTAGATCTGACTTGGAGGCTAGTCT
AGCTAGGCTATTGAACGTTTTTGATTTTGCTGAAAATCGCTATCAGGTTATTGAAACTCTTTCTCACGGAATGCGT
CAGAAAGTCTTTGTCATCGGAGCACTCTTGTCTGATCCCGATATTTGGGTTTTGGACGAACCCTTGACTGGTTTGG
ATCCCCAGGCTGCCTTTGATTTGAAACAGATGATGAAGGAACATGCACAAAAAGGGAAGACAGTCTTGTTTTCAA
CTCATGTCCTAGAGGTGGCAGAGCAAGTCTGTGATCGGATTGCCATTTTGAAAAAGGGGCATTTGATTTATTGTGG
TAAGGTAGAGGACTTGAGGAAAGACCACCCAGACCAGTCTTTGGAAAGTATCTACCTTAGTCTTGCTGGTAGAAA
AGAGGAGGTTGCGGATGCGTCTCAAGGTCATTAA

DKEALSNLNLQIENGEIMGLIGHNGAGKSTTIKSLVSIISPSSGRILVDGQELSENRLAIKRKIGYVADSPDLFLRLTANEF
WELIASSYDLSRSDLEASLARLLNVFDFAENRYQVIETLSHGMRQKVFVIGALLSDPDIWVLDEPLTGLDPQAAFDLKQ
MMKEHAQKGKTVLFSTHVLEVAEQVCDRIAILKKGHLIYCGKVEDLRKDHPDQSLESIYLSLAGRKEEVADASQGHZ

ID112 360 bp

ATGGCTTTGTTTTCAGAGAGAGGAGCAGTACGGAAGACACCAATGGCAAGTCCAATAATGAGACCTATGATGGTT
CCGACGATAGAGATTAAAAGAGTGATACCAGCACCACGCAAGAGTTGTTGCCAGTTTTCAGAAAGAATTTTAGCA
ACTTGGCTAAAGAAACTACTGCTAGTCTCTTCAGTTGTTGTAGCTTCGGCAGGTTGTTCCTTGATCATACGATCCA
TCAAGGCAACTTGGTCATCTTTTGAAATGGTTTCAATGCTGGCATTGATTTGGCTAATACGATTGTCATTTTTACGA
AGCCCGATAGCGATAGCTGTATCTTCTTCCCCAGTTTTGAAACCAGGTTCTACTTGA

MALFSERGAVRKTPMASPIMRPMMVPTIEIKRVIPAPRKSCCQFSERILATWLKKLLLVSSVVVASAGCSLIIRSIKATWSS
FEMVSMLALIWLIRLSFLRSPIAIAVSSSPVLKPGSTZ

ID 128 - 3.43

ATGAAATTTAGTAAAAAATATATAGCAGCTGGATCAGCTGTTATCGTATC
CTTGAGTCTATGTGCCTATGCACTAAACCAGCATCGTTCGCAGGAAAATA
AGGACAATAATCGTGTCTCTTATGTGGATGGCAGCCAGTCAAGTCAGAAA
AGTGAAAACTTGACACCAGACCAGGTTAGCCAGAAAGAAGGAATTCAGGC
TGAGCAAATTGTAATCAAAATTACAGATCAGGGCTATGTAACGTCACACG
GTGACCACTATCATTACTATAATGGGAAAGTTCCTTATGATGCCCTCTTT
AGTGAAGAACTCTTGATGAAGGATCCAAACTATCAACTTAAAGACGCTGA
TATTGTCAATGAAGTCAAGGGTGGTTATATCATCAAGGTCGATGGAAAAT
ATTATGTCTACCTGAAAGATGCAGCTCATGCTGATAATGTTCGAACTAAA
GATGAAATCAATCGTCAAAAACAAGAACATGTCAAAGATAATGAGAAGGT
TAACTCTAATGTTGCTGTAGCAAGGTCTCAGGGACGATATACGACAAATG
ATGGTTATGTCTTTAATCCAGCTGATATTATCGAAGATACGGGTAATGCT
TATATCGTTCCTCATGGAGGTCACTATCACTACATTCCCAAAAGCGATTT
ATCTGCTAGTGAATTAGCAGCAGCTAAAGCACATCTGGCTGGAAAAAATA
TGCAACCGAGTCAGTTAAGCTATTCTTCAACAGCTAGTGACAATAACACG
CAATCTGTAGCAAAAGGATCAACTAGCAAGCCAGCAAATAAATCTGAAAA
TCTCCAGAGTCTTTTGAAGGAACTCTATGATTCACCTAGCGCCCAACGTT
ACAGTGAATCAGATGGCCTGGTCTTTGACCCTGCTAAGATTATCAGTCGT
ACACCAAATGGAGTTGCGATTCCGCATGGCGACCATTACCACTTTATTCC
TTACAGCAAGCTTTCTGCCTTAGAAGAAAAGATTGCCAGAATGGTGCCTA
TCAGTGGAACTGGTTCTACAGTTTCTACAAATGCAAAACCTAATGAAGTA
GTGTCTAGTCTAGGCAGTCTTTCAAGCAATCCTTCTTCTTTAACGACAAG
TAAGGAGCTCTCTTCAGCATCTGATGGTTATATTTTTAATCCAAAAGATA
TCGTTGAAGAAACGGCTACAGCTTATATTGTAAGACATGGTGATCATTTC
CATTACATTCCAAAATCAAATCAAATTGGGCAACCGACTCTTCCAAACAA
TAGTCTAGCAACACCTTCTCCATCTCTTCCAATCAATCCAGGAACTTCAC
ATGAGAAACATGAAGAAGATGGATACGGATTTGATGCTAATCGTATTATC
GCTGAAGATGAATCAGGTTTTGTCATGAGTCACGGAGACCACAATCATTA
TTTCTTCAAGAAGGACTTGACAGAAGAGCAAATTAAGGTGCGCAAAAACA
TTTAG

MKFSKKYIAAGSAVIVSLSLCAYALNQHRSQENKDNNRVSYVDGSQSSQK
SENLTPDQVSQKEGIQAEQIVIKITDQGYVTSHGDHYHYYNGKVPYDALF
SEELLMKDPNYQLKDADIVNEVKGGYIIKVDGKYYVYLKDAAHADNVRTK
DEINRQKQEHVKDNEKVNSNVAVARSQGRYTTNDGYVFNPADIIEDTGNA
YIVPHGGHYHYIPKSDLSASELAAAKAHLAGKNMQPSQLSYSSTASDNNT
QSVAKGSTSKPANKSENLQSLLKELYDSPSAQRYSESDGLVFDPAKIISR
TPNGVAIPHGDHYHFIPYSKLSALEEKIARMVPISGTGSTVSTNAKPNEV
VSSLGSLSSNPSSLTTSKELSSASDGYIFNPKDIVEETATAYIVRHGDHF
HYIPKSNQIGQPTLPNNSLATPSPSLPINPGTSHEKHEEDGYGFDANRII
AEDESGFVMSHGDHNHYFFKKDLTEEQIKVRKNI*

TABLE 2

ID2 840 bp

ATGGGAATTGCTCTAGAAAATGTGAATTTTACATATCAAGAAGGTACTCCCTTAGCTTCAGCAGCTTTGTCGGATG
TTTCTTTGACGATTGAAGATGGCTCTTATACAGCTTTAATTGGGCACACAGGTAGTGGTAAATCAACTATTTTACA
ACTCTTAAATGGTTTATTGGTGCCAAGTCAAGGGAGTGTGAGGGTTTTTGATACCTTAATCACCTCGACTTCTAAA
AATAAAGATATTCGTCAAATTAGAAAACAGGTTGGCTTGGTATTTCAGTTTGCTGAAAATCAGATTTTTGAAGAAA
CGGTTTTGAAGGACGTTGCTTTTGGACCGCAAAATTTTGGAGTTTCTGAAGAAGATGCTGTGAAGACTGCGCGTGA
GAAACTGGCTCTGGTTGGAATTGATGAATCACTTTTTGATCGTAGTCCGTTTGAGCTGTCAGGGGGACAAATGAGA
CGTGTTGCCATTGCAGGCATACTTGCCATGGAGCCAGCTATATTAGTCTTAGATGAGCCAACAGCTGGTCTAGATC
CTCTAGGGAGAAAAGAGTTGATGACCCTGTTCAAAAAACTCCACCAGTCAGGGATGACCATCGTCTTGGTAACGC
ATTTGATGGATGATGTTGCTGAATATGCGAATCAAGTCTATGTAATGGAAAAGGGACGTTTAGTAAAGGGGGGCA
AACCAAGTGATGTCTTTCAAGACGTTGTTTTTATGGAAGAAGTTCAGTTGGGAGTACCTAAAATTACGGCCTTTTG
TAAACGATTGGCTGATAGAGGCGTGTCATTTAAACGATTACCGATTAAGATAGAGGAGTTCAAGGAGTCGCTAAA
TGGATAG

MGIALENVNFTYQEGTPLASAALSDVSLTIEDGSYTALIGHTGSGKSTILQLLNGLLVPSQGSVRVFDTLITSTSKNKDIR
QIRKQVGLVFQFAENQIFEETVLKDVAFGPQNFGVSEEDAVKTAREKLALVGIDESLFDRSPFELSGGQMRRVAIAGILA
MEPAILVLDEPTAGLDPLGRKELMTLFKKLHQSGMTIVLVTHLMDDVAEYANQVYVMEKGRLVKGGKPSDVFQDVV
FMEEVQLGVPKITAFCKRLADRGVSFKRLPIKIEEFKESLNGZ

ID 3 6360 bp

TACCCGGTAGTCTTAGCAGACACATCTAGCTCTGAAGATGCTTTAAACATCTCTGATAAAGAAAAAGTAGCAGAA
AATAAAGAGAAACATGAAAATATCCATAGTGCTATGGAAACTTCACAGGATTTTAAAGAGAAGAAAACAGCAGTC
ATTAAGGAAAAAGAAGTTGTTAGTAAAAATCCTGTGATAGACAATAACACTAGCAATGAAGAAGCAAAAATCAA
AGAAGAAAATTCCAATAAATCCCAAGGAGATTATACGGACTCATTTGTGAATAAAAACACAGAAAATCCCAAAAA
AGAAGATAAAGTTGTCTATATTGCTGAATTTAAAGATAAAGAATCTGGAGAAAAAGCAATCAAGGAACTATCCAG
TCTTAAGAATACAAAAGTTTTATATACTTATGATAGAATTTTTAACGGTAGTGCCATAGAAACAACTCCAGATAAC
TTGGACAAAATTAAACAAATAGAAGGTATTTCATCGGTTGAAAGGGCACAAAAAGTCCAACCCATGATGAATCAT
GCCAGAAAGGAAATTGGAGTTGAGGAAGCTATTGATTACCTAAAGTCTATCAATGCTCCGTTTGGGAAAAATTTT
GATGGTAGAGGTATGGTCATTTCAAATATCGATACTGGAACAGATTATAGACATAAGGCTATGAGAATCGATGAT
GATGCCAAAGCCTCAATGAGATTTAAAAAAGAAGACTTAAAAGGCACTGATAAAAATTATTGGTTGAGTGATAAA
ATCCCTCATGCGTTCAATTATTATAATGGTGGCAAAATCACTGTAGAAAAATATGATGATGGAAGGGATTATTTTG
ACCCACATGGGATGCATATTGCAGGGATTCTTGCTGGAAATGATACTGAACAAGACATCAAAAACTTTAACGGCA
TAGATGGAATTGCACCTAATGCACAAATTTTCTCTTACAAAATGTATTCTGACGCAGGATCTGGGTTTGCGGGTGA
TGAAACAATGTTTCATGCTATTGAAGATTCTATCAAACACAACGTTGATGTTGTTTCGGTATCATCTGGTTTTACA
GGAACAGGTCTTGTAGGTGAGAAATATTGGCAAGCTATTCGGGCATTAAGAAAAGCAGGCATTCCAATGGTTGTC
GCTACGGGTAACTATGCGACTTCTGCTTCAAGTTCTTCATGGGATTTAGTAGCAAATAATCATCTGAAAATGACCG
ACACTGGAAATGTAACACGAACTGCAGCACATGAAGATGCGATAGCGGTCGCTTCTGCTAAAAATCAAACAGTTG
AGTTTGATAAAGTTAACATAGGTGGAGAAAGTTTTAAATACAGAAATATAGGGGCCTTTTTCGATAAGAGTAAAA
TCACAACAAATGAAGATGGAACAAAAGCTCCTAGTAAATTAAAATTTGTATATATAGGCAAGGGGCAAGACCAAG
ATTTGATAGGTTTGGATCTTAGGGGCAAAATTGCAGTAATGGATAGAATTTATACAAAGGATTTAAAAAATGCTTT
TAAAAAAGCTATGGATAAGGGTGCACGCGCCATTATGGTTGTAAATACTGTAAATTACTACAATAGAGATAATTG
GACAGAGCTTCCAGCTATGGGATATGAAGCGGATGAAGGTACTAAAAGTCAAGTGTTTTCAATTTCAGGAGATGA
TGGTGTAAAGCTATGGAACATGATTAATCCTGATAAAAAAACTGAAGTCAAAAGAAATAATAAAGAAGATTTTAA
AGATAAATTGGAGCAATACTATCCAATTGATATGGAAAGTTTTAATTCCAACAAACCGAATGTAGGTGACGAAAA
AGAGATTGACTTTAAGTTTGCACCTGACACAGACAAAGAACTCTATAAAGAAGATATCATCGTTCCAGCAGGATC
TACATCTTGGGGGCCAAGAATAGATTTACTTTTAAAACCCGATGTTTCAGCACCTGGTAAAAATATTAAATCCACG
CTTAATGTTATTAATGGCAAATCAACTTATGGCTATATGTCAGGAACTAGTATGGCGACTCCAATCGTGGCAGCTT
CTACTGTTTTGATTAGACCGAAATTAAAGGAAATGCTTGAAAGACCTGTATTGAAAAATCTTAAGGGAGATGACA
AAATAGATCTTACAAGTCTTACAAAAATTGCCCTACAAAATACTGCGCGACCTATGATGGATGCAACTTCTTGGA
AAGAAAAAAGTCAATACTTTGCATCACCTAGACAACAGGGAGCAGGCCTAATTAATGTGGCCAATGCTTTGAGAA
ATGAAGTTGTAGCAACTTTCAAAAACACTGATTCTAAAGGTTTGGTAAACTCATATGGTTCCATTTCTCTTAAAGA
AATAAAAGGTGATAAAAAATACTTTACAATCAAGCTTCACAATACATCAAACAGACCTTTGACTTTTAAAGTTTCA
GCATCAGCGATAACTACAGATTCTCTAACTGACAGATTAAAACTTGATGAAACATATAAAGATGAAAAATCTCCA
GATGGTAAGCAAATTGTTCCAGAAATTCACCCAGAAAAAGTCAAAGGAGCAAATATCACATTTGAGCATGATACT
TTCACTATAGGCGCAAATTCTAGCTTTGATTTGAATGCGGTTATAAATGTTGGAGAGGCCAAAAACAAAAATAAA
TTTGTAGAATCATTTATTCATTTTGAGTCAGTGGAAGCGATGGAAGCTCTAAACTCCAGCGGGAAGAAAATAAAC
TTCCAACCTTCTTTGTCGATGCCTCTAATGGGATTTGCTGGGAATTGGAACCACGAACCAATCCTTGATAAATGGG
CTTGGGAAGAAGGGTCAAGATCAAAAACACTGGGAGGTTATGATGATGATGGTAAACCGAAAATTCCAGGAACCT
TAAATAAGGGAATTGGTGGAGAACATGGTATAGATAAATTTAATCCAGCAGGAGTTATACAAAATAGAAAAGATA
AAAATACAACATCCCTGGATCAAAATCCAGAATTATTTGCTTTCAATAACGAAGGGATCAACGCTCCATCATCAA
GTGGTTCTAAGATTGCTAACATTTATCCTTTAGATTCAAATGGAAATCCTCAAGATGCTCAACTTGAAAGAGGATT

AACACCTTCTCCACTTGTATTAAGAAGTGCAGAAGAAGGATTGATTTCAATAGTAAATACAAATAAAGAGGGAGA
AAATCAAAGAGACTTAAAAGTCATTTCGAGAGAACACTTTATTAGAGGAATTTTAAATTCTAAAAGCAATGATGC
AAAGGGAATCAAATCATCTAAACTAAAAGTTTGGGGTGACTTGAAGTGGGATGGACTCATCTATAATCCTAGAGG
TAGAGAAGAAAATGCACCAGAAAGTAAGGATAATCAAGATCCTGCTACTAAGATAAGAGGTCAATTTGAACCGAT
TGCGGAAGGTCAATATTTCTATAAATTTAAATATAGATTAACTAAAGATTACCCATGGCAGGTTTCCTATATTCCT
GTAAAAATTGATAACACCGCCCCTAAGATTGTTTCGGTTGATTTTTCAAATCCTGAAAAAATTAAGTTGATTACAA
AGGATACTTATCATAAGGTAAAAGATCAGTATAAGAATGAAACGCTATTTGCGAGAGATCAAAAAGAACATCCTG
AAAAATTTGACGAGATTGCGAACGAAGTTTGGTATGCTGGCGCCGCTCTTGTTAATGAAGATGGAGAGGTTGAAA
AAAATCTTGAAGTAACTTACGCAGGTGAGGGTCAAGGAAGAAATAGAAAACTTGATAAAGACGGAAATACCATTT
ATGAAATTAAAGGTGCGGGAGATTTAAGGGGAAAAATCATTGAAGTCATTGCATTAGATGGTTCTAGCAATTTCA
CAAAGATTCATAGAATTAAATTTGCTAATCAGGCTGATGAAAAGGGGATGATTTCCTATTATCTAGTAGATCCTGA
TCAAGATTCATCTAAATATCAAAAGCTTGGCGAGATTGCAGAATCTAAATTTAAAAATTTAGGAAATGGAAAAGA
GGGTAGTCTAAAAAAAGATACAACTGGGGTAGAACATCATCATCAAGAAAATGAAGAGTCTATTAAAGAAAAAT
CTAGTTTTACTATTGATAGAAATATTTCAACAATTAGAGACTTTGAAAATAAAGACTTAAAGAAACTCATTAAAAA
GAAATTTAGAGAAGTTGATGATTTTACAAGTGAAACTGGTAAGAGAATGGAGGAATACGATTATAAATACGATGA
TAAAGGAAATATAATAGCCTACGATGATGGGACTGATCTAGAATATGAAACTGAGAAACTTGACGAAATCAAATC
AAAAATTTATGGTGTTCTAAGTCCGTCTAAAGATGGACACTTTGAAATTCTTGGAAAGATAAGTAATGTTTCTAAA
AATGCCAAGGTATATTATGGGAATAACTATAAATCTATAGAAATCAAAGCGACCAAGTATGATTTCCACTCAAAA
ACGATGACATTTGATCTATACGCTAATATTAATGATATTGTGGATGGATTAGCTTTTGCAGGAGATATGAGATTAT
TTGTTAAAGATAATGATCAGAAAAAAGCTGAAATTAAAATTAGAATGCCTGAAAAAATTAAGGAAACTAAATCAG
AATATCCCTATGTATCAAGTTATGGGAATGTCATAGAATTAGGGGAAGGAGATCTTTCAAAAAACAAACCAGACA
ATTTAACTAAAATGGAATCTGGTAAAATCTATTCTGATTCAGAAAAACAACAATATCTGTTAAAGGATAATATCAT
TCTAAGAAAAGGCTATGCACTAAAAGTGACTACCTATAATCCTGGAAAAACGGATATGTTAGAAGGAAATGGAGT
CTATAGCAAGGAAGATATAGCAAAAATACAAAAGGCCAATCCTAATCTAAGAGCCCTTTCAGAAACAACAATTTA
TGCTGATAGTAGAAATGTTGAAGATGGAAGAAGTACCCAATCTGTATTAATGTCGGCTTTGGACGGCTTTAACATT
ATAAGGTATCAAGTGTTTACATTTAAAATGAACGATAAAGGGGAAGCTATCGATAAAGACGGAAATCTTGTGACA
GATTCTTCTAAACTTGTATTATTTGGTAAGGATGATAAAGAATACACTGGAGAGGATAAGTTCAATGTAGAAGCTA
TAAAAGAAGATGGCTCCATGTTATTTATTGATACCAAACCAGTAAACCTTTCAATGGATAAGAACTACTTTAATCC
ATCTAAATCTAATAAAATTTATGTACGAAATCCAGAATTTTATTTAAGAGGTAAGATTTCTGATAAGGGTGGTTTT
AACTGGGAATTGAGAGTTAATGAATCGGTTGTAGATAATTATTTAATCTACGGAGATTTACACATTGATAACACTA
GAGATTTTAATATTAAGCTGAATGTTAAAGACGGTGACATCATGGACTGGGGAATGAAAGACTATAAAGCAAACG
GATTTCCAGATAAGGTAACAGATATGGATGGAAATGTTTATCTTCAAACTGGCTATAGCGATTTGAATGCTAAAGC
AGTTGGAGTCCACTATCAGTTTTTATATGATAATGTTAAACCCGAAGTAAACATTGATCCTAAGGGAAATACTAGT
ATCGAATATGCTGATGGAAAATCTGTAGTCTTTAACATCAATGATAAAAGAAATAATGGATTCGATGGTGAGATT
CAAGAACAACATATTTATATAAATGGAAAAGAATATACATCATTTAATGATATTAAACAAATAATAGACAAGACA
CTAAACATTAAGATTGTTGTAAAAGATTTTGCAAGAAATACAACCGTAAAAGAATTCATTTTAAATAAAGATACG
GGAGAGGTAAGTGAATTAAAACCTCATAGGGTAACTGTGACCATTCAAAATGGAAAAGAAATGAGTTCAACGATA
GTGTCGGAAGAAGATTTTATTTTACCTGTTTATAAGGGTGAATTAGAAAAAGGATACCAATTTGATGGTTGGGAAA
TTTCTGGTTTCGAAGGTAAAAAAGACGCTGGCTATGTTATTAATCTATCAAAAGATACCTTTATAAAACCTGTATT
CAAGAAAATAGAGGAGAAAAAGGAGGAAGAAAATAAACCTACTTTTGATGTATCGAAAAAGAAAGATAACCCAC
AAGTAAACCATAGTCAATTAAATGAAAGTCACAGAAAAGAGGATTTACAAAGAGAAGAGCATTCACAAAAATCT
GATTCAACTAAGGATGTTACAGCTACAGTTCTTGATAAAAACAATATCAGTAGTAAATCAACTACTAACAATCCT
AATAAGTTGCCAAAAACTGGAACAGCAAGCGGAGCCCAGACACTATTAGCTGCCGGAATAATGTTTATAGTAGGA
ATTTTTCTTGGATTGAAGAAAAAAAATCAAGATTAA

YPVVLADTSSSEDALNISDKEKVAENKEKHENIHSAMETSQDFKEKKTAVIKEKEVVSKNPVIDNNTSNEEAKIKEENSN
KSQGDYTDSFVNKNTENPKKEDKVVYIAEFKDKESGEKAIKELSSLKNTKVLYTYDRIFNGSAIETTPDNLDKIKQIEGIS
SVERAQKVQPMMNHARKEIGVEEAIDYLKSINAPFGKNFDGRGMVISNIDTGTDYRHKAMRIDDDAKASMRFKKEDL
KGTDKNYWLSDKIPHAFNYYNGGKITVEKYDDGRDYFDPHGMHIAGILAGNDTEQDIKNFNGIDGIAPNAQIFSYKMY
SDAGSGFAGDETMFHAIEDSIKHNVDVVSVSSGFTGTGLVGEKYWQAIRALRKAGIPMVVATGNYATSASSSSWDLVA
NNHLKMTDTGNVTRTAAHEDAIAVASAKNQTVEFDKVNIGGESFKYRNIGAFFDKSKITTNEDGTKAPSKLKFVYIGK
GQDQDLIGLDLRGKIAVMDRIYTKDLKNAFKKAMDKGARAIMVVNTVNYYNRDNWTELPAMGYEADEGTKSQVFSI
SGDDGVKLWNMINPDKKTEVKRNNKEDFKDKLEQYYPIDMESFNSNKPNVGDEKEIDFKFAPDTDKELYKEDIIVPAG
STSWGPRIDLLLKPDVSAPGKNIKSTLNVINGKSTYGYMSGTSMATPIVAASTVLIRPKLKEMLERPVLKNLKGDDKIDL
TSLTKIALQNTARPMMDATSWKEKSQYFASPRQQGAGLINVANALRNEVVATFKNTDSKGLVNSYGSISLKEIKGDKK
YFTIKLHNTSNRPLTFKVSASAITTDSLTDRLKLDETYKDEKSPDGKQIVPEIHPEKVKGANITFEHDTFTIGANSSFDLN
AVINVGEAKNKNKFVESFIHFESVEAMEALNSSGKKINFQPSLSMPLMGFAGNWNHEPILDKWAWEEGSRSKTLGGYD
DDGKPKIPGTLNKGIGGEHGIDKFNPAGVIQNRKDKNTTSLDQNPELFAFNNEGINAPSSSGSKIANIYPLDSNGNPQDA
QLERGLTPSPLVLRSAEEGLISIVNTNKEGENQRDLKVISREHFIRGILNSKSNDAKGIKSSKLKVWGDLKWDGLIYNPRG
REENAPESKDNQDPATKIRGQFEPIAEGQYFYKFKYRLTKDYPWQVSYIPVKIDNTAPKIVSVDFSNPEKIKLITKDTYHK
VKDQYKNETLFARDQKEHPEKFDEIANEVWYAGAALVNEDGEVEKNLEVTYAGEGQGRNRKLDKDGNTIYEIKGAG
DLRGKIIEVIALDGSSNFTKIHRIKFANQADEKGMISYYLVDPDQDSSKYQKLGEIAESKFKNLGNGKEGSLKKDTTGVE
HHHQENEESIKEKSSFTIDRNISTIRDFENKDLKKLIKKKFREVDDFTSETGKRMEEYDYKYDDKGNIIAYDDGTDLEYE
TEKLDEIKSKIYGVLSPSKDGHFEILGKISNVSKNAKVYYGNNYKSIEIKATKYDFHSKTMTFDLYANINDIVDGLAFAG
DMRLFVKDNDQKKAEIKIRMPEKIKETKSEYPYVSSYGNVIELGEGDLSKNKPDNLTKMESGKIYSDSEKQQYLLKDNII

LRKGYALKVTTYNPGKTDMLEGNGVYSKEDIAKIQKANPNLRALSETTIYADSRNVEDGRSTQSVLMSALDGFNIIRYQ
VFTFKMNDKGEAIDKDGNLVTDSSKLVLFGKDDKEYTGEDKFNVEAIKEDGSMLFIDTKPVNLSMDKNYFNPSKSNKI
YVRNPEFYLRGKISDKGGFNWELRVNESVVDNYLIYGDLHIDNTRDFNIKLNVKDGDIMDWGMKDYKANGFPDKVTD
MDGNVYLQTGYSDLNAKAVGVHYQFLYDNVKPEVNIDPKGNTSIEYADGKSVVFNINDKRNNGFDGEIQEQHIYINGK
EYTSFNDIKQIIDKTLNIKIVVKDFARNTTVKEFILNKDTGEVSELKPHRVTVTIQNGKEMSSTIVSEEDFILPVYKGELEK
GYQFDGWEISGFEGKKDAGYVINLSKDTFIKPVFKKIEEKKEEENKPTFDVSKKKDNPQVNHSQLNESHRKEDLQREEH
SQKSDSTKDVTATVLDKNNISSKSTTNNPNKLPKTGTASGAQTLLAAGIMFIVGIFLGLKKKNQDZ

ID6 597 bp

CTTGAATTAAATAAAAAACGTCATGCGACTAAGCATTTTACTGATAAGCTTGTTGATCCCAAAGATGTGCGTACGG
CTATCGAAATTGCAACCTTAGCGCCAAGCGCCCACAACAGCCAGCCTTGGAAATTTGTGGTGGTACGTGAGAAAA
ATGCTGAACTGGCAAAGTTAGCTTATGGTTCCAATTTTGAACAGGTATCATCAGCGCCTGTAACCATTGCCTTGTT
TACAGATACGGACTTAGCCAAACGTGCTCGTAAGATTGCCCGTGTTGGTGGTGCTAATAACTTTTCTGAAGAGCAA
CTTCAATATTTTATGAAAAATCTGCCAGCTGAGTTTGCCCGTTACAGTGAGCAACAAGTCAGCGACTACCTAGCTC
TCAATGCAGGTTTGGTTGCCATGAACTTGGTTCTTGCATTGACAGACCAAGGAATTGGTTCTAACATTATTCTTGG
TTTTGACAAATCAAAAGTTAATGAAGTTTTGGAAATCGAAGACCGTTTCCGCCCAGAACTCTTGATCACAGTGGGT
TATACAGACGAAAAATTGGAACCAAGCTACCGCTTGCCAGTAGATGAAATCATCGAGAAAAGATAG

LELNKKRHATKHFTDKLVDPKDVRTAIEIATLAPSAHNSQPWKFVVVREKNAELAKLAYGSNFEQVSSAPVTIALFTDT
DLAKRARKIARVGGANNFSEEQLQYFMKNLPAEFARYSEQQVSDYLALNAGLVAMNLVLALTDQGIGSNIILGFDKSK
VNEVLEIEDRFRPELLITVGYTDEKLEPSYRLPVDEIIEKRZ

ID7 1401 bp

ATGACAGCAATTGATTTTACAGCAGAAGTAGAAAAACGCAAAGAAGACCTCTTGGCTGACTTGTTTAGCCTTTTG
GAAATCAATTCAGAACGTGATGACAGCAAGGCTGATGCCCAGCATCCATTTGGGCCTGGTCCAGTAAAAGCCTTG
GAGAAATTCCTTGAAATCGCAGACCGCGATGGCTACCCAACTAAGAATGTTGATAACTATGCAGGACATTTTGAG
TTTGGTGATGGAGAAGAAGTTCTCGGAATCTTTGCCCATATGGATGTGGTGCCTGCTGGTAGCGGTTGGGACACAG
ACCCTTACACACCAACTATCAAAGATGGTCGCCTTTATGCGCGCGGGGCTTCGGACGATAAGGGTCCTACAACAG
CTTGTTACTATGGTTTGAAAATCATCAAAGAATTGGGTCTTCCAACTTCTAAGAAAGTTCGCTTCATCGTTGGAAC
AGACGAAGAATCAGGCTGGGCAGACATGGACTACTACTTTGAGCACGTAGGACTTGCCAAACCAGATTTCGGTTT
CTCACCAGATGCTGAATTTCCAATCATCAATGGTGAAAAAGGAAATATCACGGAATACCTCCACTTTGCAGGAGA
AAATACAGGTGTTGCCCGTCTTCACAGCTTTACAGGTGGTTTACGTGAAAATATGGTACCAGAATCAGCAACAGC
AGTCGTTTCAGGTGACTTGGCTGACTTGCAAGCTAAACTAGATGCCTTTGTTGCAGAACACAAACTTAGAGGAGA
ACTCCAAGAAGAAGCTGGCAAATACAAGGTGACGATCATTGGTAAATCAGCCCACGGTGCTATGCCTGCTTCAGG
TGTCAATGGCGCAACTTACCTTGCCCTCTTCCTCAGCCAGTTTGGCTTTGCTGGTCCAGCCAAAGACTACCTTGAC
ATCGCAGGTAAAATTCTCTTGAACGATCATGAGGGTGAAAATCTTAAGATTGCTCATGTGGATGAAAAGATGGGT
GCTCTTTCTATGAATGCCGGCGTCTTCCACTTCGATGAAACAAGTGCTGATAATACCATTGCCCTCAACATCCGCT
ATCCAAAAGGAACAAGTCCAGAACAAATCAAGTCAATCCTTGAAAACTTGCCAGTTGTTTCTGTTAGCCTGTCTGA
ACACGGTCACACGCCTCACTATGTGCCAATGGAAGATCCACTTGTGCAAACCTTGTTGAATATCTATGAAAAACA
AACTGGCTTTAAAGGTCATGAACAAGTCATCGGTGGTGGAACCTTTGGTCGCTTGCTAGAACGCGGAGTTGCCTA
CGGTGCTATGTTCCCAGACTCGATTGATACCATGCACCAAGCCAATGAATTTATCGCCTTGGATGATCTTTTCCGA
GCAGCAGCAATTTATGCCGAAGCTATTTACGAATTGATCAAATAA

MTAIDFTAEVEKRKEDLLADLFSLLEINSERDDSKADAQHPFGPGPVKALEKFLEIADRDGYPTKNVDNYAGHFEFGD
GEEVLGIFAHMDVVPAGSGWDTDPYTPTIKDGRLYARGASDDKGPTTACYYGLKIIKELGLPTSKKVRFIVGTDEESGW
ADMDYYFEHVGLAKPDFGFSPDAEFPIINGEKGNITEYLHFAGENTGVARLHSFTGGLRENMVPESATAVVSGDLADL
QAKLDAFVAEHKLRGELQEEAGKYKVTIIGKSAHGAMPASGVNGATYLALFLSQFGFAGPAKDYLDIAGKILLNDHEG
ENLKIAHVDEKMGALSMNAGVFHFDETSADNTIALNIRYPKGTSPEQIKSILENLPVVSVSLSEHGHTPHYVPMEDPLVQ
TLLNIYEKQTGFKGHEQVIGGGTFGRLLERGVAYGAMFPDSIDTMHQANEFIALDDLFRAAAIYAEAIYELIKZ

ID8 1617 bp

GTGTATACTATTATAAAATCAAATATAAAAAAATTTAGTTTATTAACGATATTTATTGTTGCTGGTCAATTATTGCT
AATTTATGCAGCAACTATTAATGCTCTGGTGTTGAATGAATTAATTGCGATGAATTTAGAGCGGTTTTTGAAATTG
TCAATCTACCAAATGATTGTCTGGTGTGGGATAATATTCCTTGACTGGGTAGTGAAAAATTATCAGGTTGAAGTGA
TCCAAGAGTTTAATCTAGAGATTCGAAATAGAGTTGCCACAGACATCTCTAACTCTACCTATCAAGAATTTCATAG
TAAATCATCAGGAACATATCTTTCGTGGCTAAATAATGATGTTCAGACTTTAAATGATCAGGCGTTTAAACAACTT
TTTTTAGTAATAAAAGGAATTTCTGGTACTATATTTGCAGTTGTGACTCTTAATCACTATCATTGGTCATTGACTGT
AGCCACCTTGTTTTCATTAATGATTATGCTACTTGTACCAAAAATCTTTGCATCGAAAATGCGAGAAGTTAGTCTA
AATTTAACTAACCAAAATGAAGCTTTTTTAAAATCTAGTGAGACTATATTGAATGGATTTGATGTGTTAGCGTCCT
TGAATCTTTTATATGTATTGCCTAAGAAAATTAAAGAAGCAGGAATTTTATTAAAGATGGTTATACAAAGAAAGA
CAACTGTAGAAACGTTAGCAGGCGCTATTAGCTTCTTTCTCAATATTTTTTTTCAGATATCTCTCGTTTTTTTAACA
GGCTATCTTGCAATAAAAGGAATAGTGAAAATTGGTACTATTGAAGCAATAGGAGCACTAACAGGTGTTATTTTT

ACAGCGCTAGGTGAATTAGGAGGTCAATTATCCTCTATTATTGGTACGAAGCCTATTTTTTTAAAATTGTATTCAA
TTAATCCAATTGAGTCAAATAAAATGAATGATATCGAACCAAATGAGGTGAATAGAGATTTTCCGTTATATGAAG
CAAAAAATATTTGCTATAAGTATGGAGATAAAGAAATATTAAAAAACTTAAATTTTTGTTTTCAACGTAATGAAAA
GTATTTAATTTTAGGTGAAAGTGGAAGCGGGAAATCTACATTATTAAAATTATTGAATGGCTTTTTGAGAGATTAT
AGTGGAGAATTGCGATTCTGCGGGGATGATATAAAAAAAACCTCCTATTTAAATATGGTTTCGAATGTTCTATATG
TAGATCAAAAAGCTTATTTGTTTGAAGGTACGATTAGAGATAATATTTTATTGGAAGAAAATTATACTGATGAAGA
AATACTACAGTCTTTAGAGCAAGTTGGTTTGAGTGTAAAAGATTTTCCTAATAACATTTTAGATTATTATGTTGGT
GATGATGGGAGATTACTGTCAGGAGGGCAGAAACAAAAAATTACTTTAGCTAGAGGGCTAATTAGAAATAAGAA
AATAGTATTAATTGACGAGGGAACTTCTGCTATCGATAGGAGAACTTCGTTAGCGATTGAACGTAAGATATTAGA
TAGAGAGGATTTGACTGTCATTATTGTTACCCATGCTCCGCATCCGGAACTTAAACAATATTTTACTAAGATATAT
CAATTTCCAAAGGATTTTATTTAA

MYTIIKSNIKKFSLLTIFIVAGQLLLIYAATINALVLNELIAMNLERFLKLSIYQMIVWCGIIFLDWVVKNYQVEVIQEFNL
EIRNRVATDISNSTYQEFHSKSSGTYLSWLNNDVQTLNDQAFKQLFLVIKGISGTIFAVVTLNHYHWSLTVATLFSLMIM
LLVPKIFASKMREVSLNLTNQNEAFLKSSETILNGFDVLASLNLLYVLPKKIKEAGILLKMVIQRKTTVETLAGAISFFLNI
FFQISLVFLTGYLAIKGIVKIGTIEAIGALTGVIFTALGELGGQLSSIIGTKPIFLKLYSINPIESNKMNDIEPNEVNRDFPLYE
AKNICYKYGDKEILKNLNFCFQRNEKYLILGESGSGKSTLLKLLNGFLRDYSGELRFCGDDIKKTSYLNMVSNVLYVDQ
KAYLFEGTIRDNILLEENYTDEEILQSLEQVGLSVKDFPNNILDYYVGDDGRLLSGGQKQKITLARGLIRNKKIVLIDEGT
SAIDRRTSLAIERKILDREDLTVIIVTHAPHPELKQYFTKIYQFPKDFIZ

ID9 705 bp

ATAACAGTTAAACAGATTATGGACGAAATAGCCGTTTCAGATATGACTGCAAGGCGCTATTTACAGGAATTAGCT
GATAAAGATTTGCTGATTCGTGTGCATGGTGGAGCTGAAAAACTTCGAACCAACTCCCTTTTGACTAATGAGCGAT
CAAATATTGAAAAACAAGCCCTCCAAACGGCAGAAAAACAAGAAATAGCCCATTTTGCAGGCAGTCTAGTAGAA
GAAAGAGAAACTATTTTCATTGGACCAGGAACAACATTAGAGTTTTTTGCGCGTGAGTTGCCTATTGACAATATCC
GCGTCGTAACCAACAGTCTACCTGTTTTTCTGATTTTAAGCGAACGAAAATTAACAGATTTGATTTTAATAGGTGG
AAATTATCGCGATATTACAGGTGCTTTTGTTGGTACATTGACCCTACAAAATCTCTCTAATCTCCAATTTTCTAAA
GCTTTCGTTAGCTGTAATGGTATTCAAAACGGAGCTCTAGCTACTTTTAGCGAGGAAGAGGGAGAGGCTCAACGC
ATCGCTTTAAATAATTCTAATAAAAAATATTTACTCGCAGATCATAGCAAGTTCAATAAGTTTGATTTTTATACTTT
TTATAATGTATCAAATCTTGATACTATTGTTTCAGATTCTAAACTAAGTGATTCAATCCTTTTTAAGCTATCTAAAC
ACATTAAAGTCATCAAGCCTTAA

ITVKQIMDEIAVSDMTARRYLQELADKDLLIRVHGGAEKLRTNSLLTNERSNIEKQALQTAEKQEIAHFAGSLVEERETI
FIGPGTTLEFFARELPIDNIRVVTNSLPVFLILSERKLTDLILIGGNYRDITGAFVGTLTLQNLSNLQFSKAFVSCNGIQNGA
LATFSEEEGEAQRIALNNSNKKYLLADHSKFNKFDFYTFYNVSNLDTIVSDSKLSDSILFKLSKHIKVIKPZ

ID10 483 bp

ATGACTGAGTTTTCGTTAGATCTTCTTCTAGAAGCCATTAAACTAGCTCGTTGGACCTACTACTATCACTTGAAAC
AGCTAGACAAAACAGATAAAGACCAAGAGCTTAAAACTGAAATTCAATCCATCTTTATCGAACACAAGGGAAATT
ATGCTTATCGCCGGGTTCATTTAGAACTAAGAAATCGTGGTTATCTGGTAAATCATAAAAGAGTTCAAGGCTTGaT
GAAAGTACTCAATTTACAAGCTAAAATGCGAAAGAAACGAAAATATTCTTCTCATAAAGGAGACGTTGGTAAGAA
GGCAGAGAATCTCATTCAAGCCCAATTTGAAGGCTCTAAAACAATGGAAAAGTGCTACACAGATGTGACTGAATT
TGCCATTCCAGCAAGTACTCAAAAGCTTTACTTATCACCAGTTTTAGATGGCTTTAACAGCGAAATTATTGCTTTT
AATCTTTCTTGTTCGCCTAATTTAGAATAA

MTEFSLDLLLEAIKLARWTYYYHLKQLDKTDKDQELKTEIQSIFIEHKGNYAYRRVHLELRNRGYLVNHKRVQGLMK
VLNLQAKMRKKRKYSSHKGDVGKKAENLIQAQFEGSKTMEKCYTDVTEFAIPASTQKLYLSPVLDGFNSEIIAFNLSCS
PNLEZ

ID14 1266 bp

CCAGGATTTGGTACCGTTGCAAGTGGTGTGCCTTTCCTCCTAAAGGAAAATGGAGGAAAAATCAATCAATCAGCA
CATTCAGATATCAAAGTTGCTAAGGTATTGGTCAAGGATGAAGATGAAAAAAATCGCTTGCTTGCAGCAGGGAAT
GACTTTAACTTTGTAACCAATGTGGATGATATTTTATCAGACCAGGATATTACTATCGTAGTGGAATTGATGGGGC
GTATTGAGCCTGCTAAAACCTTTATCACTCGTGCCTTGGAAGCTGGAAAACACGTTGTTACTGCTAACAAGGACCT
TTTAGCTGTCCATGGCGCAGAATTGCTAGAAATCGCTCAAGCTAACAAGGTAGCACTTTACTACGAAGCAGCAGT
TGCTGGTGGGATTCCAATTCTTCGTACTTTAGCAAATTCCTTGGCTTCTGATAAAATTACGCGCGTGCTTGGAGTA
GTCAACGGAACTTCCAACTTCATGGTGACCAAGATGGTGGAAGAAGGCTGGTCTTACGATGATGCTCTTGCGGAA
GCACAACGTCTAGGATTTGCAGAAAGCGATCCGACGAATGACGTAGATGGGATTGATGCAGCCTACAAGATGGTT
ATTTTGAGCCAATTTGCCTTTGGCATGAAGATTGCCTTTGATGATGTAGCCCACAAGGGAATCCGCAATATCACAC
CAGAAGACGTAGCTGTAGCTCAAGAGCTTGGTTACGTAGTGAAATTGGTTGGTTCTATTGAGGAAACTTCTTCAGG
TATTGCTGCAGAAGTGACTCCAACCTTCCTACCTAAAGCGCACCCACTTGCTAGTGTGAATGGCGTAATGAACGCT
GTCTTTGTAGAATCTATCGGTATTGGTGAGTCTATGTACTACGGACCAGGTGCGGGTCAAAAACCAACTGCAACA

AGTGTTGTAGCTGATATTGTCCGTATCGTTCGTCGTTTGAATGATGGTACTATTGGCAAAGACTTCAACGAATATA
GCCGTGACTTGGTCTTGGCAAATCCTGAAGATGTCAAAGCAAACTACTATTTCTCAATCTTGGCTCTAGACTCAAA
AGGTCAGGTCTTGAAGTTGGCTGAAATCTTCAATGCTCAAGATATTTCCTTTAAGCAAATCCTTCAAGATGGCAAA
GAGGGTGACAAGGCGCGTGTCGTTATCATCACACACAAGATTAATAAAGCCCAGCTTGAAAATGTCTCAGCTGAA
TTGAAGAAGGTTTCAGAATTCGACCTCTTGAATACCTTCAAGGTGCTAGGAGAATAA

PGFGTVASGVPFLLKENGGKINQSAHSDIKVAKVLVKDEDEKNRLLAAGNDFNFVTNVDDILSDQDITIVVELMGRIEP
AKTFITRALEAGKHVVTANKDLLAVHGAELLEIAQANKVALYYEAAVAGGIPILRTLANSLASDKITRVLGVVNGTSNF
MVTKMVEEGWSYDDALAEAQRLGFAESDPTNDVDGIDAAYKMVILSQFAFGMKIAFDDVAHKGIRNITPEDVAVAQE
LGYVVKLVGSIEETSSGIAAEVTPTFLPKAHPLASVNGVMNAVFVESIGIGESMYYGPGAGQKPTATSVVADIVRIVRRL
NDGTIGKDFNEYSRDLVLANPEDVKANYYFSILALDSKGQVLKLAEIFNAQDISFKQILQDGKEGDKARVVIITHKINKA
QLENVSAELKKVSEFDLLNTFKVLGEZ

ID16 1725 bp

ATGAAACACCTATTATCTTACTTCAAACCCTACATCAAGGAATCAATTTTAGCCCCCTTGTTCAAGCTGTTAGAAG
CTGTTTTTGAGCTCTTGGTTCCCATGGTGATTGCTGGGATTGTTGACCAATCTTTACCTCAGGGAGATCAAGGTCA
TCTCTGGATGCAGATTGGCCTGCTCCTTATCTTTGCAGTAATTGGCGTTTTAGTGGCCTTGATAGCTCAATTTTACT
CAGCAAAGGCAGCAGTAGGTTCTGCTAAGGAATTGACAAACGATCTTTATCGTCATATTCTTTCCTTGCCCAAGGA
CAGCAGAGACCGTCTGACAACTTCTAGTTTGGTCACTCGCTTGACTTCGGATACCTACCAGATTCAGACTGGTATC
AATCAATTCCTGCGTCTCTTTTTACGAGCGCCCATTATCGTTTTTGGTGCCATTTTTATGGCTTATCGAATCTCAGC
TGAGTTGACTTTCTGGTTCTTAGTCTTGGTTGCCATTTTGACCATTGTCATTGTAGGGTTATCTCGATTGGTCAATC
CTTTCTACAGTAGTCTCAGAAAGAAAACGGACCAACTGGTTCAGGAAACGCGCCAGCAATTGCAAGGGATGCGGG
TTATTCGTGCTTTTGGTCAAGAAAAACGAGAGTTACAGATTTTTCAAACCCTTAACCAAGTTTATGCTAGATTACA
AGAAAAGACAGGTTTCTGGTCTAGTTTATTAACACCTCTGACCTATCTGATTGTCAATGGAACTCTTCTCGTTATT
ATCTGGCAAGGCTATATTTCAATTCAAGGAGGAGTGCTCAGTCAAGGTGCTCTCATTGCTCTTATCAATTACCTCT
TACAGATTTTGGTGGAATTGGTCAAGCTAGCCATGTTGATCAATTCCCTCAACCAGTCCTATATCTCAGTCAAGCG
AATCGAGGAAGTCTTTGTTGAGGCTCCAGAGGATATCCATTCAGAGTTAGAACAAAAGCAAGCTACCAGAGATAA
GGTTTTACAAGTCCAAGAATTGACCTTTACCTATCCTGATGCGGCCCAGCCTTCTCTGAGATACATTTCCTTTGAT
ATGACTCAAGGACAAATTCTAGGTATCATCGGGGGAACTGGTTCTGGTAAATCAAGCTTGGTGCAACTCTTACTTG
GACTTTATCCAGTAGACAAGGGGAACATTGACCTTTATCAAAATGGACGTAGTCCTCTTAATTTGGAGCAGTGGC
GGTCTTGGATTGCCTATGTACCTCAAAAGGTCGAACTCTTTAAAGGAACCATTCGTTCCAACTTGACTCTAGGTTT
CAATCAAGAAGTATCTGACCAGGAACTCTGGCAGGCCTTGGAGATTGCGCAAGCTAAGGATTTTGTCAGTGAAAA
GGAAGGACTCTTGGATGCTCTAGTTGAGGCAGGGGGGCGAAATTTCTCAGGTGGACAAAAACAAAGATTGTCTAT
CGCCCGAGCAGTCTTGCGCCAGGCTCCGTTTCTCATCCTAGATGATGCAACCTCGGCACTGGATACCATTACAGAG
TCCAAGCTCTTGAAAGCTATTAGAGAAAATTTTCCAAACACGAGCTTAATTTTGATCTCTCAACGAACCTCAACTT
TACAGATGGCGGACCAGATTCTCCTCTTGGAAAAAGGTGAGTTGCTAGCTGTTGGCAAGCACGATGACTTGATGA
AATCCAGCCAAGTCTATTGTGAAATCAATGCATCCCAACATGGAAAGGAGGACTAG

MKHLLSYFKPYIKESILAPLFKLLEAVFELLVPMVIAGIVDQSLPQGDQGHLWMQIGLLLIFAVIGVLVALIAQFYSAKA
AVGSAKELTNDLYRHILSLPKDSRDRLTTSSLVTRLTSDTYQIQTGINQFLRLFLRAPIIVFGAIFMAYRISAELTFWFLVL
VAILTIVIVGLSRLVNPFYSSLRKKTDQLVQETRQQLQGMRVIRAFGQEKRELQIFQTLNQVYARLQEKTGFWSSLLTPL
TYLIVNGTLLVIIWQGYISIQGGVLSQGALIALINYLLQILVELVKLAMLINSLNQSYISVKRIEEVFVEAPEDIHSELEQKQ
ATRDKVLQVQELTFTYPDAAQPSLRYISFDMTQGQILGIIGGTGSGKSSLVQLLLGLYPVDKGNIDLYQNGRSPLNLEQ
WRSWIAYVPQKVELFKGTIRSNLTLGFNQEVSDQELWQALEIAQAKDFVSEKEGLLDALVEAGGRNFSGGQKQRLSIA
RAVLRQAPFLILDDATSALDTITESKLLKAIRENFPNTSLILISQRTSTLQMADQILLLEKGELLAVGKHDDLMKSSQVYC
EINASQHGKEDZ

ID18 1224 bp

ATGAAACGTTCTCTCGACTCAAGAGTCGATTACAGTTTGCTCTTGCCAGTATTTTTTCTACTGGTCATCGGTGTGGT
GGCTATCTATATAGCCGTTAGTCATGATTATCCCAATAATATTCTGCCCATTTTAGGGCAGCAGGTCGCCTGGATT
GCCTTGGGGCTTGTGATTGGTTTTGTGGTCATGCTCTTTAATACAGAATTTCTTTGGAAGGTGACCCCCTTTCTATA
TATTTTAGGCTTGGGACTTATGATCTTGCCGATTGTATTTTATAATCCAAGCTTAGTTGCATCAACGGGTGCCAAA
AACTGGGTATCAATAAATGGAATTACCCTATTCCAACCGTCAGAATTTATGAAGATATCCTATATCCTCATGTTGG
CTCGTGTCATTGTCCAATTTACAAAGAAACATAAGGAATGGAGACGCACGGTTCCGCTGGACTTTTTGTTAATTTT
CTGGATGATTCTCTTTACCATTCCAGTCCTAGTTCTTTTAGCACTTCAAAGTGACTTGGGGACGGCTTTGGTTTTTG
TAGCCATTTTCTCAGGAATCGTTTTATTATCAGGGGTTTCTTGGAAAATTATTATCCCAGTATTTGTGACTGCTGTA
ACAGGAGTTGCTGGTTTCTTAGCTATCTTTATTAGCAAGGACGGACGAGCTTTTCTTCACCAGATTGGAATGCCGA
CCTACCAAATTAATCGGATTTTGGCTTGGCTCAATCCCTTTGAGTTTGCCCAAACAACGACTTACCAGCAGGCTCA
AGGGCAGATTGCCATTGGGAGTGGTGGCTTATTTGGTCAGGGATTTAATGCTTCGAATCTGCTTATCCCAGTTCGA
GAGTCAGATATGATTTTTACGGTTATTGCAGAAGATTTTGGCTTTATTGGCTCTGTCCTGGTTATTGCCCTCTATCT
CATGTTGATTTACCGTATGTTGAAGATTACTCTTAAATCAAATAACCAGTTCTACACTTATATTTCCACAGGTTTGA
TTATGATGTTGCTCTTCCACATCTTTGAGAATATCGGTGCTGTGACTGGACTACTTCCTTTGACGGGGATTCCCTTG
CCTTTCATTTCGCAAGGGGGATCAGCTATTATCAGTAATCTGATTGGTGTTGGTTTGCTTTTATCGATGAGTTACCA

GACTAATCTAGCTGAAGAAAAGAGCGGAAAAGTCCCATTCAAACGGAAAAAGGTTGTATTAAAACAAATTAAATA
A

MKRSLDSRVDYSLLLPVFFLLVIGVVAIYIAVSHDYPNNILPILGQQVAWIALGLVIGFVVMLFNTEFLWKVTPFLYILGL
GLMILPIVFYNPSLVASTGAKNWVSINGITLFQPSEFMKISYILMLARVIVQFTKKHKEWRRTVPLDFLLIFWMILFTIPVL
VLLALQSDLGTALVFVAIFSGIVLLSGVSWKIIIPVFVTAVTGVAGFLAIFISKDGRAFLHQIGMPTYQINRILAWLNPFEF
AQTTTYQQAQGQIAIGSGGLFGQGFNASNLLIPVRESDMIFTVIAEDFGFIGSVLVIALYLMLIYRMLKITLKSNNQFYTY
ISTGLIMMLLFHIFENIGAVTGLLPLTGIPLPFISQGGSAIISNLIGVGLLLSMSYQTNLAEEKSGKVPFKRKKVVLKQIKZ

ID22 987 bp

ATGGTGGCTAAGAAAAAAATCTTATTTTTTATGTGGTCTTTTTCTCTTGGAGGTGGTGCAGAGAAGATTCTATCAA
CCATTGTTTCAAATCTGGATCCAGAAAAGTATGATATTGATATTCTTGAAATGGAGCACTTTGACAAGGGATATGA
ATCTGTTCCAAAGCATGTACGCATTTTAAAATCCCTTCAAGATTATCGCCAAACCAGATGGTTACGAGCTTTTTTG
TGGAGAATGAGAATTTATTTTCCAAGACTGACTCGTCGTTTGCTTGTAAAAGATGATTATGATGTTGAAGTTTCTT
TTACCATTATGAATCCACCACTGTTGTTCTCTAAAAGAAGAGAAGTCAAGAAGATATCTTGGATTCATGGAAGTAT
TGAAGAACTTCTTAAGGATAGCTCTAAAAGAGAATCACATAGAAGCCAGTTGGATGCTGCGAATACAATTGTAGG
GATTTCAAAAAAGACCAGCAATTCTATCAAGGAAGTTTATCCAGATTATACTTCTAAATTACAGACAATCTACAAT
GGATATGATTTTCAGACTATTCTAGAAAAATCTCAAGAGAAGATCGATATCGAGATTGCTCCTCAAAGTATCTGTA
CTATCGGACGGATTGAGGAAAATAAGGGTTCTGACCGTGTAGTGGAAGTGATACGATTATTACACCAAGAGGGAA
AAAACTATCATCTCTATTTTATCGGGGCTGGTGATATGGAAGAGGAACTGAAAAAACGAGTCAAAGAGTATGGGA
TTGAGGACTATGTACATTTCCTTGGTTATCAAAAAAATCCTTATCAGTATCTATCTCAGACGAAAGTTCTTTTGTCT
ATGTCTAAACAAGAAGGTTTTCCTGGAGTGTATGTGGAGGCCTTGAGTCTGGGACTCCCTTTTATCTCTACGGACG
TTGGAGGGGCTGAGGAATTATCCCAAGAAGGACGATTTGGACAAATCATTGAGAGCAATCAAGAGGCAGCTCAG
GCGATTACTAATTACATGACTTCTGCCTCAAACTTTGATGTCGATGAGGCTAGCCAATTCATTCAACAATTTACAA
TTACAAAACAAATCGAACAAGTAGAAAAACTATTAGAGGAGTAG

MVAKKKILFFMWSFSLGGGAEKILSTIVSNLDPEKYDIDILEMEHFDKGYESVPKHVRILKSLQDYRQTRWLRAFLWRM
RIYFPRLTRRLLVKDDYDVEVSFTIMNPPLLFSKRREVKKISWIHGSIEELLKDSSKRESHRSQLDAANTIVGISKKTSNSIK
EVYPDYTSKLQTIYNGYDFQTILEKSQEKIDIEIAPQSICTIGRIEENKGSDRVVEVIRLLHQEGKNYHLYFIGAGDMEEEL
KKRVKEYGIEDYVHFLGYQKNPYQYLSQTKVLLSMSKQEGFPGVYVEALSLGLPFISTDVGGAEELSQEGRFGQIIESNQ
EAAQAITNYMTSASNFDVDEASQFIQQFTITKQIEQVEKLLEEZ

ID23 1434 bp

ATGGAAACTGCATTAATTAGTGTGATTGTGCCAGTCTATAATGTGGCGCAGTACCTAGAAAAATCGATAGCTTCCA
TTCAGAAGCAGACCTATCAAAATCTGGAAATTATTCTTGTTGATGATGGTGCAACAGATGAAAGTGGTCGCTTGTG
TGATTCAATCGCTGAACAAGATGACAGGGTGTCAGTGCTTCATAAAAAGAACGAAGGATTGTCGCAAGCACGAAA
TGATGGGATGAAGCAGGCTCACGGGGATTATCTGATTTTTATTGACTCAGATGATTATATCCATCCAGAAATGATT
CAGAGCTTATATGAGCAATTAGTTCAAGAAGATGCGGATGTTTCGAGCTGTGGTGTCATGAATGTCTATGCTAATG
ATGAAAGCCCACAGTCAGCCAATCAGGATGACTATTTTGTCTGTGATTCTCAAACATTTCTAAAGGAATACCTCAT
AGGTGAAAAAATACCTGGGACGATTTGCAATAAGCTAATCAAGAGACAGATTGCAACTGCCCTATCCTTTCCTAA
GGGGTTGATTTACGAAGATGCCTATTACCATTTTGATTTAATCAAGTTGGCCAAGAAGTATGTGGTTAATACTAAA
CCCTATTATTACTATTTCCATAGAGGGGATAGTATTACGACCAAACCCTATGCAGAGAAGGATTTAGCCTATATTG
ATATCTACCAAAAGTTTTATAATGAAGTTGTGAAAAACTATCCTGACTTGAAAGAGGTCGCTTTTTTCAGATTGGC
CTATGCCCACTTCTTTATTCTGGATAAGATGTTGCTAGATGATCAGTATAAACAGTTTGAAGCCTATTCTCAGATT
CATCGTTTTTTAAAAGGCCATGCCTTTGCTATTTCTAGGAATCCAATTTTCCGTAAGGGGAGAAGAATTAGTGCTT
TGGCCCTATTCATAAATATTTCCTTATATCGATTCTTATTACTGAAAAATATTGAAAAATCTAAAAAATTACATTA
G

METALISVIVPVYNVAQYLEKSIASIQKQTYQNLEIILVDDGATDESGRLCDSIAEQDDRVSVLHKKNEGLSQARNDGM
KQAHGDYLIFIDSDDYIHPEMIQSLYEQLVQEDADVSSCGVMNVYANDESPQSANQDDYFVCDSQTFLKEYLIGEKIPG
TICNKLIKRQIATALSFPKGLIYEDAYYHFDLIKLAKKYVVNTKPYYYYFHRGDSITTKPYAEKDLAYIDIYQKFYNEVV
KNYPDLKEVAFFRLAYAHFFILDKMLLDDQYKQFEAYSQIHRFLKGHAFAISRNPIFRKGRRISALALFINISLYRFLLLK
NIEKSKKLHZ

ID24 735bp

ATGAGAATCAAAGAGAAAACCAATAATATTAATGGAGGAATAAAAAATGTAAGTAAGCATTATGGTCATTCAATC
ATTCTCAAAGATATAAATTTTGCACTTAACAAGGGTGAAATTGTTGGTCTAGCAGGGAGAAATGGAGTTGGTAAG
AGTACGTTGATGAAAATTCTTGTTCAGAATAATCAACCGACTTCAGGTAATATTATAAGCAGTGATAATGTTGGGT
ATTTAATCGAAGAACCAAAATTATTTTTATCTAAAACAGGTTTAGAGAATTTAAAATATTTGTCAAATTTATATGG
TGTTGACTACAATCAAGAAAGATTTAGATGTTTGATCCAAGAGTTAGATTTGACTCAGTCTATTAATAAAAAAGTA
AAGACCTATTCTTTGGGTACAAAACAAAAATTAGCTTTGCTTCTAACTCTCGTTACGGAACCTGATATATTGATTT

TAGATGAACCGACTAATGGTTTAGATATTGAATCATCACAAATAGTTTTAGCGGTTCTAAAAAAATTAGCTTTACA
TGAAAATGTGGGAATTTTAATATCGAGTCATAAATTAGAAGACATTGAAGAAATTTGTGAGAGAGTTCTTTTCTTG
GAGAACGGGCTTTTGACATTTCAAAAAGTAGGAAAAGATAGTCATAATTTCTTGTTTGAGATAGCTTTTTCATCAG
CTACAGATAGAGACATTTTCATTACCAAACAAGAATTTTGGGATATTGTTTAG

MRIKEKTNNINGGIKNVSKHYGHSIILKDINFALNKGEIVGLAGRNGVGKSTLMKILVQNNQPTSGNIISSDNVGYLIEEP
KLFLSKTGLENLKYLSNLYGVDYNQERFRCLIQELDLTQSINKKVKTYSLGTKQKLALLLTLVTEPDILILDEPTNGLDIE
SSQIVLAVLKKLALHENVGILISSHKLEDIEEICERVLFLENGLLTFQKVGKDSHNFLFEIAFSSATDRDIFITKQEFWDIVZ

ID25 1704bp

ATGACTGAATTAGATAAACGTCACCGCAGTAGCATTTATGACAGCATGGTTAAATCACCTAACCGTGCTATGCTTC
GTGCGACTGGTATGACAGATAAGGACTTTGAAACATCGATTGTGGGAGTGATTTCGACTTGGGCGGAAAATACAC
CATGTAACATTCACTTGCATGATTTCGGGAAACTGGCTAAAGAAGGTGTCAAATCTGCAGGCGCTTGGCCTGTAC
AGTTTGGAACCATTACCGTAGCGGACGGGATCGCTATGGGAACGCCTGGTATGCGTTTCTCTCTAACATCTCGTGA
CATCATCGCGGACTCCATCGAGGCGGCTATGAGTGGTCACAACGTGGATGCCTTCGTCGCTATCGGTGGCTGTGA
CAAGAACATGCCTGGATCTATGATTGCTATTGCTAATATGGATATCCCAGCTATTTTCGCCTATGGTGGAACTATT
GCACCGGGAAATCTTGATGGTAAAGATATCGACTTGGTTTCTGTCTTTGAAGGTATCGGAAAATGGAACCACGGT
GACATGACAGCTGAGGACGTGAAACGTCTTGAATGTAATGCCTGCCCTGGCCCTGGTGGTTGTGGTGGTATGTAT
ACTGCTAATACCATGGCAACTGCTATCGAAGTTCTAGGGATGAGTTTGCCAGGGTCATCCTCTCACCCAGCTGAAT
CAGCTGATAAGAAAGAAGATATCGAAGCAGCAGGACGTGCTGTTGTTAAGATGTTGGAACTTGGTCTCAAACCAT
CAGATATCTTGACTCGTGAAGCCTTTGAAGATGCTATCACTGTAACGATGGCTCTCGGTGGTTCTACAAACGCCAC
TCTTCACTTGCTCGCCATTGCCCATGCCGCAAATGTTGACTTGTCACTTGAGGACTTCAATACGATTCAAGAACGT
GTGCCTCACTTGGCCGACTTGAAACCATCTGGTCAGTATGTCTTCCAAGACCTCTACGAAGTCGGTGGTGTCCCTG
CGGTTATGAAGTATTTGTTGGCAAATGGTTTCCTTCACGGAGATCGCATCACATGTACTGGTAAGACTGTAGCTGA
AAACTTGGCTGACTTTGCAGACTTGACTCCAGGCCAAAAAGTTATCATGCCACTTGAAAATCCAAAACGTGCGGA
TGGTCCGCTTATCATCTTGAACGGGAACCTTGCTCCTGACGGTGCAGTTGCCAAGGTATCAGGTGTTAAAGTGCGT
CGTCACGTTGGGCCAGCTAAGGTCTTTGACTCAGAAGAAGATGCGATTCAGGCCGTTCTGACAGATGAAATCGTT
GATGGCGATGTAGTCGTTGTTCGTTTTGTTGGACCTAAAGGTGGTCCTGGTATGCCTGAGATGCTATCACTTTCTTC
AATGATTGTTGGTAAAGGTCAGGGAGATAAGGTGGCCCTCTTGACGGACGGACGTTTCTCTGGTGGTACTTATGGT
CTGGTTGTTGGACATATCGCTCCTGAAGCTCAGGATGGTGGACCAATTGCCTATCTCCGTACCGGCGATATCGTTA
CGGTTGACCAAGATACCAAAGAAATTTCTATGGCCGTATCCGAAGAAGAACTTGAAAAACGCAAGGCAGAAACA
ACCTTGCCACCACTTTACAGCCGTGGTGTCCTCGGTAAATATGCCCACATCGTATCATCTGCTTCACGCGGAGCCG
TGACAGACTTCTGGAATATGGACAAGTCAGGTAAAAAATAA

MTELDKRHRSSIYDSMVKSPNRAMLRATGMTDKDFETSIVGVISTWAENTPCNIHLHDFGKLAKEGVKSAGAWPVQFG
TITVADGIAMGTPGMRFSLTSRDIIADSIEAAMSGHNVDAFVAIGGCDKNMPGSMIAIANMDIPAIFAYGGTIAPGNLDG
KDIDLVSVFEGIGKWNHGDMTAEDVKRLECNACPGPGGCGGMYTANTMATAIEVLGMSLPGSSSHPAESADKKEDIE
AAGRAVVKMLELGLKPSDILTREAFEDAITVTMALGGSTNATLHLLAIAHAANVDLSLEDFNTIQERVPHLADLKPSGQ
YVFQDLYEVGGVPAVMKYLLANGFLHGDRITCTGKTVAENLADFADLTPGQKVIMPLENPKRADGPLIILNGNLAPDG
AVAKVSGVKVRRHVGPAKVFDSEEDAIQAVLTDEIVDGDVVVVRFVGPKGGPGMPEMLSLSSMIVGKGQGDKVALLT
DGRFSGGTYGLVVGHIAPEAQDGGPIAYLRTGDIVTVDQDTKEISMAVSEEELEKRKAETTLPPLYSRGVLGKYAHIVSS
ASRGAVTDFWNMDKSGKKZ

ID26 274bp

ATGTTATAATAAAAATAAAGAATTTAAGGAGAAATACAATATGTCAATTTTTATTGGAGGAGCATGGCCATATGC
AAACGGTTCGTTACATATTGGTCACGCGGCAGCGCTTTTACCGGGGGATATTCTTGCAAGATACTATCGTCAGAA
GGGAGAGGAAGTTTTATATGTTTCTGGAAGTGATTGTAATGGAACCCCTATTTCTATCAGAGCTAAAAAAGAAAA
TAAGTCTGTGAAAGAAATTGCTGATTTTTATCATAAGGAATTTAATCCA

CYNKNKEFKEKYNMSIFIGGAWPYANGSLHIGHAAALLPGDILARYYRQKGEEVLYVSGSDCNGTPISIRAKKENKSVK
EIADFYHKEFNP

ID28 1065bp

ATGACAACATTATTTTCAAAAATTAAAGAAGTAACAGAACTTGCTGCAGTCTCAGGTCATGAAGCGCCTGTCCGT
GCTTATCTTCGTGAAAAGTTGACACCGCATGTGGATGAAGTGGTGACAGATGGCTTGGGTGGTATTTTTGGTATCA
AACATTCAGAAGCTGTGGATGCACCGCGCGTCTTGGTCGCTTCTCATATGGACGAAGTTGGTTTTATGGTCAGCGA
AATCAAGCCAGATGGTACCTTCCGTGTCGTAGAAATCGGTGGCTGGAACCCCATGGTGGTTAGCAGCCAACGTTT
CAAACTCTTGACTCGTGATGGTCATGAAATTCCTGTGATTTCAGGTTCTGTTCCTCCGCATTTGACTCGTGGAAAG
GGGGGACCAACCATGCCAGCCATTGCCGATATCGTTTTTGATGGTGGTTTTGCGGACAAGGCTGAGGCAGAAAGT
TTTGGCATCCGTCCTGGTGATACCATTGTACCAGATAGTTCTGCAATTTTGACAGCCAATGAAAAAAATATCATCT
CAAAAGCTTGGGATAACCGCTACGGTGTCCTCATGGTAAGCGAGCTAGCTGAAGCTTTATCGGGTCAAAAACTCG
GCAATGAACTCTATCTGGGTTCTAACGTCCAAGAAGAAGTTGGTCTGCGTGGCGCTCATACCTCTACAACCAAGTT

TGACCCAGAAGTCTTCCTCGCAGTTGATTGCTCACCAGCAGGTGATGTCTACGGTGGTCAAGGCAAGATTGGAGA
TGGAACCTTGATTCGTTTCTATGATCCAGGTCACTTGCTTCTCCCAGGGATGAAGGATTTCCTTTTGACAACGGCT
GAAGAAGCTGGTATCAAGTACCAATACTACTGTGGTAAAGGCGGAACAGATGCAGGTGCAGCTCATCTGAAAAAT
GGTGGTGTCCCATCAACAACTATCGGTGTCTGCGCTCGTTATATCCATTCTCACCAAACCCTCTATGCAATGGATG
ACTTCCTAGAAGCGCAAGCTTTCTTACAAGCCTTGGTGAAGAAATTGGATCGTTCAACGGTTGATTTGATTAAACA
TTATTAA

MTTLFSKIKEVTELAAVSGHEAPVRAYLREKLTPHVDEVVTDGLGGIFGIKHSEAVDAPRVLVASHMDEVGFMVSEIKP
DGTFRVVEIGGWNPMVVSSQRFKLLTRDGHEIPVISGSVPPHLTRGKGGPTMPAIADIVFDGGFADKAEAESFGIRPGDT
IVPDSSAILTANEKNIISKAWDNRYGVLMVSELAEALSGQKLGNELYLGSNVQEEVGLRGAHTSTTKFDPEVFLAVDCS
PAGDVYGGQGKIGDGTLIRFYDPGHLLLPGMKDFLLTTAEEAGIKYQYYCGKGGTDAGAAHLKNGGVPSTTIGVCARY
IHSHQTLYAMDDFLEAQAFLQALVKKLDRSTVDLIKHYZ

ID31 1182bp

ATGGAATTTTCTATGAAATCAGTCAAAGGACTACTCTTTATCATAGCTAGTTTTATCTTGACTCTTTTGACTTGGAT
GAACACTTCTCCCCAATTCATGATTCCAGGACTAGCTTTAACAAGCCTATCTCTGACTTTTATCCTAGCCACTCGT
CTCCCACTACTAGAAAGCTGGTTTCACAGTTTGGAGAAGGTCTACACCGTCCACAAATTCACAGCCTTTCTCTCAA
TCATCCTACTAATCTTTCATAACTTTAGTATGGGCGGTTTGTGGGGCTCTCGCTTAGCTGCTCAGTTTGGCAATCTT
GCCATCTATATCTTTGCCAGCATCATCCTTGTCGCCTATTTAGGCAAATACATCCAATACGAAGCTTGGCGATGGA
TTCACCGCCTGGTTTACCTAGCCTATATTTTAGGACTCTTTCACATCTACATGATAATGGGCAATCGTCTCCTTACA
TTTAATCTTCTAAGTTTTCTTGTTGGTAGCTATGCCCTTTTAGGCTTACTAGCTGGTTTTTATATCATTTTTCTATAT
CAAAAGATTTCCTTCCCCTATCTAGGGAAAATTACCCATCTCAAACGCTTAAATCACGATACTAGAGAAATTCAA
ATCCATCTTAGCAGACCTTTCAACTATCAATCAGGACAATTTGCCTTTCTAAAGATTTTCCAAGAAGGCTTTGAAA
GTGCTCCGCATCCCTTTTCTATCTCAGGAGGTCATGGTCAAACTCTTTACTTTACTGTTAAAACTTCAGGCGACCA
TACCAAGAATATCTATGATAATCTTCAAGCCGGCAGCAAAGTAACCCTAGACAGAGCTTACGGACACATGATCAT
AGAAGAAGGACGAGAAAATCAGGTTTGGATTGCTGGAGGTATTGGGATCACCCCCTTCATCTCTTACATCCGTGA
ACATCCTATTTTAGATAAACAGGTTCACTTCTACTATAGCTTCCGTGGAGATGAAAATGCAGTCTACCTAGATTTA
CTCCGTAACTATGCTCAGAAAAATCCTAATTTTGAACTCCATCTAATCGACAGTACGAAAGACGGCTATCTTAATT
TTGAACAAAAAGAAGTGCCCGAACATGCAACCGTCTATATGTGTGGTCCTATTTCTATGATGAAGGCACTTGCCA
AACAGATTAAGAAACAAAATCCAAAAACAGAGCATATTTAC

MEFSMKSVKGLLFIIASFILTLLTWMNTSPQFMIPGLALTSLSLTFILATRLPLLESWFHSLEKVYTVHKFTAFLSIILLIFH
NFSMGGLWGSRLAAQFGNLAIYIFASIILVAYLGKYIQYEAWRWIHRLVYLAYILGLFHIYMIMGNRLLTFNLLSFLVGS
YALLGLLAGFYIIFLYQKISFPYLGKITHLKRLNHDTREIQIHLSRPFNYQSGQFAFLKIFQEGFESAPHPFSISGGHGQTLY
FTVKTSGDHTKNIYDNLQAGSKVTLDRAYGHMIIEEGRENQVWIAGGIGITPFISYIREHPILDKQVHFYYSFRGDENAV
YLDLLRNYAQKNPNFELHLIDSTKDGYLNFEQKEVPEHATVYMCGPISMMKALAKQIKKQNPKTEHIY

ID32 900bp

ATGACTTTTAAATCAGGCTTTGTAGCCATTTTAGGACGTCCCAATGTTGGGAAGTCAACCTTTTTAAATCACGTTA
TGGGGCAAAAGATTGCCATCATGAGTGACAAGGCGCAGACAACGCGCAATAAAATCATGGGAATTTACACGACTG
ATAAGGAGCAAATTGTCTTTATCGACACACCAGGGATTCACAAGCCTAAAACAGCTCTCGGAGATTTCATGGTTG
AGTCTGCCTACAGTACCCTTCGCGAAGTGGACACTGTTCTTTTCATGGTGCCTGCTGATGAAGCGCGTGGTAAGGG
GGACGATATGATTATCGAGCGTCTCAAGGCTGCCAAGGTTCCTGTGATTTTGGTGGTGAATAAAATCGATAAGGTC
CATCCAGACCAGCTCTTGTCTCAGATTGATGACTTCCGTAATCAAATGGACTTTAAGGAAATTGTTCCAATCTCAG
CCCTTCAGGGAAATAACGTGTCTCGTCTAGTGGATATTTTGAGTGAAAATCTGGATGAAGGTTTCCAATATTTCCC
GTCTGATCAAATCACAGACCATCCAGAACGTTTCTTGGTTTCAGAAATGGTTCGCGAGAAAGTCTTGCACCTAACT
CGTGAAGAGATTCCGCATTCTGTAGCAGTAGTTGTTGACTCTATGAAACGAGACGAAGAGACAGACAAGGTTCAC
ATCCGTGCAACCATCATGGTCGAGCGCGATAGCCAAAAAGGGATTATCATCGGTAAAGGTGGCGCTATGCTTAAG
AAAATCGGTAGCATGGCCCGTCGTGATATCGAACTCATGCTAGGAGACAAGGTCTTCCTAGAAACCTGGGTCAAG
GTCAAGAAAAACTGGCGCGATAAAAAGCTAGATTTGGCTGACTTTGGCTATAATGAAAGAGAATACTAA

MTFKSGFVAILGRPNVGKSTFLNHVMGQKIAIMSDKAQTTRNKIMGIYTTDKEQIVFIDTPGIHKPKTALGDFMVESAYS
TLREVDTVLFMVPADEARGKGDDMIIERLKAAKVPVILVVNKIDKVHPDQLLSQIDDFRNQMDFKEIVPISALQGNNVS
RLVDILSENLDEGFQYFPSDQITDHPERFLVSEMVREKVLHLTREEIPHSVAVVVDSMKRDEETDKVHIRATIMVERDSQ
KGIIIGKGGAMLKKIGSMARRDIELMLGDKVFLETWVKVKKNWRDKKLDLADFGYNEREYZ

ID33 855bp

CTGCTTCTTGTTTTTACAGAAGGAGGACTTATGCCTGAATTACCTGAGGTTGAAACCGTTTGTCGTGGCTTAGAAA
AATTGATTATAGGAAAGAAGATTTCGAGTATAGAAATTCGCTACCCCAAGATGATTAAGACGGATTTGGAAGAGT
TTCAAAGGGAATTGCCTAGTCAGATTATCGAGTCAATGGGACGTCGTGGAAAATATTTGCTTTTTTATCTGACAGA
CAAGGTCTTGATTTCCCATTTGCGGATGGAGGGCAAGTATTTTTACTATCCAGACCAAGGACCTGAACGCAAGCAT
GCCCATGTTTTCTTTCATTTTGAAGATGGTGGCACGCTTGTTTATGAGGATGTTCGCAAGTTTGGAACCATGGAAC

TCTTGGTGCCTGACCTTTTAGACGTCTACTTTATTTCTAAAAAATTAGGTCCTGAACCAAGCGAACAAGACTTTGA
TTTACAGGTCTTTCAATCTGCCCTTGCCAAGTCCAAAAAGCCTATCAAATCCCATCTCCTAGACCAGACCTTGGTA
GCTGGACTTGGCAATATCTATGTGGATGAGGTTCTCTGGCGAGCTCAGGTTCATCCAGCTAGACCTTCCCAGACTT
TGACAGCAGAAGAAGCGACTGCCATTCATGACCAGACCATTGCTGTTTTGGGCCAGGCTGTTGAAAAAGGTGGCT
CCACCATTCGGACTTATACCAATGCCTTTGGGGAAGATGGAAGCATGCAGGACTTTCATCAGGTCTATGATAAGA
CTGGTCAAGAATGTGTACGCTGTGGTACCATCATTGAGAAAATTCAACTAGGCGGACGTGGAACCCACTTTTGTCC
AAACTGTCAAAGGAGGGACTGA

MLLVFTEGGLMPELPEVETVCRGLEKLIIGKKISSIEIRYPKMIKTDLEEFQRELPSQIIESMGRRGKYLLFYLTDKVLISHL
RMEGKYFYYPDQGPERKHAHVFFHFEDGGTLVYEDVRKFGTMELLVPDLLDVYFISKKLGPEPSEQDFDLQVFQSALA
KSKKPIKSHLLDQTLVAGLGNIYVDEVLWRAQVHPARPSQTLTAEEATAIHDQTIAVLGQAVEKGGSTIRTYTNAFGED
GSMQDFHQVYDKTGQECVRCGTIIEKIQLGGRGTHFCPNCQRRDZ

ID34 633bp

TTGTCCAAACTGTCAAAGGAGGGACTGATGGGAAAAATCATCGGAATCACTGGGGGAATTGCCTCTGGTAAGTCA
ACTGTGACAAATTTTCTAAGACAGCAAGGCTTTCAAGTAGTGGATGCCGACGCAGTCGTCCACCAACTACAGAAA
CCTGGTGGTCGTCTGTTTGAGGCTCTAGTACAGCACTTTGGGCAAGAAATCATTCTTGAAAACGGAGAACTCAATC
GCCCTCTCCTAGCTAGTCTCATCTTTTCAAATCCTGATGAACGAGAATGGTCTAAGCAAATTCAAGGGGAGATTAT
CCGTGAGGAACTGGCTACTTTGAGAGAACAGTTGGCTCAGACAGAAGAGATTTTCTTCATGGATATTCCCCTACTT
TTTGAGCAGGACTACAGCGATTGGTTTGCTGAGACTTGGTTGGTCTATGTGGACCGAGATGCCCAAGTGGAACGC
TTAATGAAAAGGGACCAGTTGTCCAAAGATGAAGCTGAGTCTCGTCTGGCAGCCCAGTGGCCTTTAGAAAAAAAG
AAAGATTTGGCCAGCCAGGTTCTTGATAATAATGGCAATCAGAACCAGCTTCTTAATCAAGTGCATATCCTTCTTG
AGGGAGGTAGGCAAGATGACAGAGATTAA

MSKLSKEGLMGKIIGITGGIASGKSTVTNFLRQQGFQVVDADAVVHQLQKPGGRLFEALVQHFGQEIILENGELNRPLL
ASLIFSNPDEREWSKQIQGEIIREELATLREQLAQTEEIFFMDIPLLFEQDYSDWFAETWLVYVDRDAQVERLMKRDQLS
KDEAESRLAAQWPLEKKKDLASQVLDNNGNQNQLLNQVHILLEGGRQDDRDZ

ID35 1269bp

TTGATAATAATGGCAATCAGAACCAGCTTCTTAATCAAGTGCATATCCTTCTTGAGGGAGGTAGGCAAGATGACA
GAGATTAACTGGAAGGATAATCTGCGCATTGCCTGGTTTGGTAATTTTCTGACAGGAGCCAGTATTTCTTTGGTTG
TACCTTTTATGCCCATCTTCGTGGAAAATCTAGGTGTAGGGAGTCAGCAAGTCGCTTTTTATGCAGGCTTAGCAAT
TTCTGTCTCTGCTATTTCCGCGGCGCTCTTTTCTCCTATTTGGGGTATTCTTGCTGACAAATACGGCCGAAAACCCA
TGATGATTCGGGCAGGTCTTGCTATGACTATCACTATGGGAGGCTTGGCCTTTGTCCCAAATATCTATTGGTTAAT
CTTTCTTCGTTTACTAAACGGTGTATTTGCAGGTTTTGTTCCTAATGCAACGGCACTGATAGCCAGTCAGGTTCCA
AAGGAGAAATCAGGCTCTGCCTTAGGTACTTTGTCTACAGGCGTAGTTGCAGGTACTCTAACTGGTCCCTTTATTG
GTGGCTTTATCGCAGAATTATTTGGCATTCGTACAGTTTTCTTACTGGTTGGTAGTTTTCTATTTTTAGCTGCTATTT
TGACTATTTGCTTTATCAAGGAAGATTTTCAACCAGTAGCCAAGGAAAAGGCTATTCCAACAAAGGAATTATTTAC
CTCGGTTAAATATCCCTATCTTTTGCTCAATCTCTTTTTAACCAGTTTTGTCATCCAATTTTCAGCTCAATCGATTG
GCCCTATTTTGGCTCTTTATGTACGCGACTTAGGGCAGACAGAGAATCTTCTTTTTGTCTCTGGTTTGATTGTGTCC
AGTATGGGCTTTTCCAGCATGATGAGTGCAGGAGTCATGGGCAAGCTAGGTGACAAGGTGGGCAATCATCGTCTC
TTGGTTGTCGCCCAGTTTTATTCAGTCATCATCTATCTCCTCTGTGCCAATGCCTCTAGCCCCCTTCAACTAGGACT
CTATCGTTTCCTCTTTGGATTGGGAACCGGTGCCTTGATTCCCGGGGTTAATGCCCTACTCAGCAAAATGACTCCC
AAAGCCGGCATTTCGAGGGTCTTTGCCTTCAATCAGGTATTCTTTTATCTGGGAGGTGTTGTTGGTCCCATGGCAG
GTTCTGCAGTAGCAGGTCAATTTGGCTACCATGCTGTCTTTTATGCGACAAGCCTTTGTGTTGCCTTTAGTTGTCTC
TTTAACCTGATTCAATTTCGAACATTATTAAAAGTAAAGGAAATCTAG

MIIMAIRTSFLIKCISFLREVGKMTEINWKDNLRIAWFGNFLTGASISLVVPFMPIFVENLGVGSQQVAFYAGLAISVSAIS
AALFSPIWGILADKYGRKPMMIRAGLAMTITMGGLAFVPNIYWLIFLRLLNGVFAGFVPNATALIASQVPKEKSGSALG
TLSTGVVAGTLTGPFIGGFIAELFGIRTVFLLVGSFLFLAAILTICFIKEDFQPVAKEKAIPTKELFTSVKYPYLLLNLFLTS
FVIQFSAQSIGPILALYVRDLGQTENLLFVSGLIVSSMGFSSMMSAGVMGKLGDKVGNHRLLVVAQFYSVIIYLLCANAS
SPLQLGLYRFLFGLGTGALIPGVNALLSKMTPKAGISRVFAFNQVFFYLGGVVGPMAGSAVAGQFGYHAVFYATSLCV
AFSCLFNLIQFRTLLKVKEIZ
ID36 1311bp

ATGGCCCTACCAACTATTGCCATTGTAGGACGTCCCAATGTTGGGAAATCAACCCTATTTAATCGGATCGCTGGTG
AGCGAATCTCCATTGTAGAAGATGTCGAAGGAGTGACACGTGACCGTATTTATGCAACGGGTGAGTGGCTCAATC
GTTCTTTTAGCATGATTGATACAGGAGGAATTGATGATGTCGATGCTCCTTTCATGGAACAAATCAAGCACCAGGC
AGAAATTGCCATGGAAGAAGCAGATGTTATCGTTTTTGTCGTGTCTGGTAAGGAAGGAATTACTGATGCAGACGA
ATACGTAGCTCGTAAGCTTTATAAGACCCACAAACCAGTTATCCTCGCAGTCAACAAGGTGGACAACCCTGAGAT
GAGAAATGATATATATGATTTCTATGCTCTCGGTTTGGGTGAACCATTGCCTATCTCATCTGTCCATGGAATCGGT
ACAGGGGATGTGCTAGATGCGATCGTAGAAAATCTTCCAAATGAATATGAGGAAGAAAATCCAGATGTCATTAAG
TTTAGCTTGATTGGTCGTCCTAACGTTGGAAAATCAAGCTTGATCAATGCTATCTTGGGAGAAGACCGTGTTATTG

CTAGTCCTGTTGCTGGAACAACTCGTGATGCCATTGATACCCACTTTACAGATACAGATGGTCAAGAGTTTACCAT
GATTGATACGGCTGGTATGCGTAAGTCTGGTAAGGTTTATGAAAATACTGAGAAATACTCTGTTATGCGTGCCATG
CGTGCTATTGACCGTTCAGATGTGGTCTTGATGGTCATCAATGCGGAAGAAGGCATTCGTGAGTACGACAAGCGT
ATCGCAGGATTTGCCCATGAAGCTGGTAAAGGGATGATTATCGTGGTCAACAAGTGGGATACGCTTGAAAAAGAT
AACCACACTATGAAAAACTGGGAAGAAGATATCCGTGAGCAGTTCCAATACCTGCCTTACGCACCGATTATCTTT
GTATCAGCTTTAACCAAGCAACGTCTCCACAAACTTCCTGAGATGATTAAGCAAATCAGCGAAAGTCAAAATACA
CGTATTCCATCAGCTGTCTTGAACGATGTCATCATGGATGCCATTGCCATCAACCCAACACCGACAGACAAAGGA
AAACGTCTCAAGATTTTCTATGCGACCCAAGTGGCAACCAAACCACCAACCTTTGTCATCTTTGTCAATGAAGAAG
AACTCATGCACTTTTCTTACCTGCGTTTCTTGGAAAATCAAATCCGCAAGGCCTTTGTTTTTGAGGGAACACCGAT
TCATCTCATCGCAAGAAAACGCAAATAA

MALPTIAIVGRPNVGKSTLFNRIAGERISIVEDVEGVTRDRIYATGEWLNRSFSMIDTGGIDDVDAPFMEQIKHQAEIAM
EEADVIVFVVSGKEGITDADEYVARKLYKTHKPVILAVNKVDNPEMRNDIYDFYALGLGEPLPISSVHGIGTGDVLDAI
VENLPNEYEEENPDVIKFSLIGRPNVGKSSLINAILGEDRVIASPVAGTTRDAIDTHFTDTDGQEFTMIDTAGMRKSGKV
YENTEKYSVMRAMRAIDRSDVVLMVINAEEGIREYDKRIAGFAHEAGKGMIIVVNKWDTLEKDNHTMKNWEEDIREQ
FQYLPYAPIIFVSALTKQRLHKLPEMIKQISESQNTRIPSAVLNDVIMDAIAINPTPTDKGKRLKIFYATQVATKPPTFVIFV
NEEELMHFSYLRFLENQIRKAFVFEGTPIHLIARKRKZ

ID37 714bp

ATGACAGAAACCATTAAATTGATGAAGGCTCATACTTCAGTGCGCAGGTTTAAAGAGCAAGAAATTCCCCAAGTA
GACTTAAATGAGATTTTGACAGCAGCCCAGATGGCATCATCTTGGAAGAATTTCCAATCCTACTCTGTGATTGTGG
TACGAAGTCAAGAGAAGAAAGATGCCTTGTATGAATTGGTACCTCAAGAAGCCATTCGCCAGTCTGCTGTTTTCCT
TCTCTTTGTCGGAGATTTGAACCGAGCAGAAAAGGGAGCCCGACTTCATACCGACACCTTCCAACCCCAAGGTGT
GGAAGGTCTCTTGATTAGTTCGGTCGATGCAGCTCTTGCTGGACAAAACGCCTTGTTGGCAGCTGAAAGCTTGGGC
TATGGTGGTGTGATTATCGGTTTGGTTCGATACAAGTCTGAAGAAGTGGCAGAGCTCTTTAACCTACCTGACTACA
CCTATTCTGTCTTTGGGATGGCACTGGGTGTGCCAAATCAACATCATGATATGAAACCGAGACTGCCACTAGAGA
ATGTTGTCTTTGAGGAAGAATACCAAGAACAGTCAACTGAGGCAATCCAAGCTTATGACCGTGTTCAGGCTGACT
ATGCTGGGGCGCGTGCGACCACAAGCTGGAGTCAGCGCCTAGCAGAACAGTTTGGTCAAGCTGAACCAAGCTCAA
CTAGAAAAAATCTTGAACAGAAGAAATTATTGTAG

MTETIKLMKAHTSVRRFKEQEIPQVDLNEILTAAQMASSWKNFQSYSVIVVRSQEKKDALYELVPQEAIRQSAVFLLFV
GDLNRAEKGARLHTDTFQPQGVEGLLISSVDAALAGQNALLAAESLGYGGVIIGLVRYKSEEVAELFNLPDYTYSVFG
MALGVPNQHHDMKPRLPLENVVFEEEYQEQSTEAIQAYDRVQADYAGARATTSWSQRLAEQFGQAEPSSTRKNLEQK
KLLZ

ID38 729bp

ATGACAGAAATTAGACTAGAGCACGTCAGTTATGCCTATGGTCAGGAGAGGATTTTAGAGGATATCAACCTACAG
GTGACTTCAGGCGAAGTGGTTTCCATCCTAGGCCCAAGTGGTGTTGGAAAGACCACCCTCTTTAATCTAATCGCTG
GGATTTTAGAAGTTCAGTCAGGGAGAATTGTCCTTGATGGTGAAGAAAATCCCAAGGGGCGCGTGAGTTATATGT
TGCAAAAGGATCTGCTCTTGGAGCACAAGACGGTGCTTGGAAATATCATTCTGCCCCTCTTGATTCAAAAGGTGG
ATAAGGCAGAAGCTATTTCCCGAGCGGATAAAATTCTTGCGACCTTCCAGCTGACAGCTGTAAGAGACAAGTATC
CTCATGAACTTAGCGGTGGGATGCGCCAGCGTGTAGCCTTACTCCGGACCTACCTTTTTGGGCACAAGCTCTTTCT
CTTAGATGAGGCCTTTAGCGCCTTGGATGAGATGACAAAGATGGAACTCCACGCTTGGTATCTTGAGATTCACAA
GCAGTTGCAGCTAACAACCCTGATCATCACGCATAGTATTGAGGAGGCCCTCAATCTCAGCGACCGTATCTATATC
TTGAAAAATCGCCCTGGGCAGATTGTTTCAGAAATTAAACTAGATTGGTCTGAAGATGAGGACAAGGAAGTCCAA
AAGATTGCCTACAAACGTCAAATTTTGGCGGAATTAGGCTTAGATAAGTAG

MTEIRLEHVSYAYGQERILEDINLQVTSGEVVSILGPSGVGKTTLFNLIAGILEVQSGRIVLDGEENPKGRVSYMLQKDLL
LEHKTVLGNIILPLLIQKVDKAEAISRADKILATFQLTAVRDKYPHELSGGMRQRVALLRTYLFGHKLFLLDEAFSALDE
MTKMELHAWYLEIHKQLQLTTLIITHSIEEALNLSDRIYILKNRPGQIVSEIKLDWSEDEDKEVQKIAYKRQILAELGLDK
Z

ID39 2433bp

ATGAACTATTCAAAAGCATTGAATGAATGTATCGAAAGTGCCTACATGGTTGCTGGACATTTTGGAGCTCGTTATC
TAGAGTCGTGGCACTTGTTGATTGCCATGTCTAATCACAGTTATAGTGTAGCAGGGGCAACTTTAAATGATTATCC
GTATGAGATGGACCGTTTAGAAGAGGTGGCTTTGGAACTGACTGAAACGGACTATAGCCAGGATGAAACCTTTAC
GGAATTGCCGTTCTCCCGTCGTTTGCAGGTTCTTTTTGATGAAGCAGAGTATGTAGCGTCAGTGGTCCATGCTAAG
GTACTAGGGACAGAGCACGTCCTCTATGCGATTTTGCATGATAGCAATGCCTTGGCGACTCGTATCTTGGAGAGG
GCTGGTTTTTCTTATGAAGACAAGAAAGATCAGGTCAAGATTGCTGCTCTTCGTCGAAATTTAGAAGAACGGGCA
GGCTGGACTCGTGAAGATCTCAAGGCTTTACGCCAACGCCATCGTACAGTAGCTGACAAGCAAAATTCTATGGCC
AATATGATGGGCATGCCGCAGACTCCTAGTGGTGGTCTCGAGGATTATACGCATGATTTGACAGAGCAAGCGCGT
TCTGGCAAGTTAGAACCAGTCATCGGTCGGGACAAGGAAATCTCACGTATGATTCAAATCTTGAGCCGGAAGACT

AAGAACAACCCTGTCTTGGTTGGGGATGCTGGTGTCGGGAAAACAGCTCTGGCGCTTGGTCTTGCCCAGCGTATTG
CTAGTGGTGACGTGCCTGCGGAAATGGCTAAGATGCGCGTGTTAGAACTTGATTTGATGAATGTCGTTGCAGGGA
CACGCTTCCGTGGTGACTTTGAAGAACGCATGAATAATATCATCAAGGATATTGAAGAAGATGGCCAAGTCATCC
TCTTTATCGATGAACTCCACACCATCATGGGTTCTGGTAGCGGGATTGATTCGACTCTGGATGCGGCCAATATCTT
GAAACCAGCCTTGGCGCGTGGAACTTTGAGAACGGTTGGTGCCACTACTCAGGAAGAATATCAAAAACATATCGA
AAAAGATGCGGCACTTTCTCGTCGTTTCGCTAAAGTGACGATTGAAGAACCAAGTGTGGCAGATAGTATGACTAT
TTTACAAGGTTTGAAGGCGACTTATGAGAAACATCACCGTGTACAAATCACAGATGAAGCGGTTGAAACAGCGGT
TAAGATGGCTCATCGTTATTTAACCAGTCGTCACTTGCCAGACTCTGCTATCGATCTCTTGGATGAGGCGGCAGCA
ACAGTGCAAAATAAGGCAAAGCATGTAAAAGCAGACGATTCAGATTTGAGTCCAGCTGACAAGGCCCTGATGGAT
GGCAAGTGGAAACAGGCAGCCCAGCTAATCGCAAAAGAAGAGGAAGTACCTGTCTACAAAGACTTGGTGACAGA
GTCTGATATTTTGACCACCTTGAGTCGCTTGTCAGGAATCCCAGTTCAAAAACTGACTCAAACGGATGCTAAGAAG
TATTTAAATCTTGAAGCAGAACTCCATAAACGGGTTATCGGTCAAGATCAAGCTGTTTCAAGCATTAGCCGTGCCA
TTCGCCGCAACCAGTCAGGGATTCGCAGTCATAAGCGTCCGATTGGTTCCTTTATGTTCCTAGGGCCTACAGGTGT
CGGGAAAACTGAATTAGCCAAGGCTCTGGCAGAAGTTCTTTTTGACGACGAATCAGCCCTTATCCGCTTTGATATG
AGTGAGTATATGGAGAAATTTGCAGCTAGTCGTCTCAACGGAGCTCCTCCAGGCTATGTAGGATATGAAGAAGGT
GGGGAGTTGACAGAGAAGGTTCGCAATAAACCCTATTCCGTTCTCCTCTTTGATGAGGTAGAGAAGGCCCACCCA
GATATCTTTAATGTTCTCTTGCAGGTTCTGGATGACGGTGTCTTGACAGATAGCAAGGGACGCAAGGTCGATTTTT
CAAATACCATTATCATTATGACATCGAATCTAGGTGCGACTGCCCTTCGTGATGATAAGACTGTTGGTTTTGGGGC
TAAGGATATTCGTTTTGACCAGGAAAATATGGAAAAACGCATGTTTGAAGAACTGAAAAAAGCTTATAGACCGGA
ATTCATCAACCGTATTGATGAGAAGGTGGTCTTCCATAGCCTATCTAGTGATCATATGCAGGAAGTGGTGAAGATT
ATGGTCAAGCCTTTAGTGGCAAGTTTGACTGAAAAAGGCATTGACTTGAAATTACAAGCTTCAGCTCTGAAATTGT
TAGCAAATCAAGGATATGACCCAGAGATGGGAGCTCGCCCACTTCGCAGAACCCTGCAAACAGAAGTGGAGGAC
AAGTTGGCAGAACTTCTTCTCAAGGGAGATTTAGTGGCAGGCAGCACACTTAAGATTGGTGTCAAAGCAGGCCAG
TTAAAATTTGATATTGCATAA

MNYSKALNECIESAYMVAGHFGARYLESWHLLIAMSNHSYSVAGATLNDYPYEMDRLEEVALELTETDYSQDETFTE
LPFSRRLQVLFDEAEYVASVVHAKVLGTEHVLYAILHDSNALATRILERAGFSYEDKKDQVKIAALRRNLEERAGWTR
EDLKALRQRHRTVADKQNSMANMMGMPQTPSGGLEDYTHDLTEQARSGKLEPVIGRDKEISRMIQILSRKTKNNPVLV
GDAGVGKTALALGLAQRIASGDVPAEMAKMRVLELDLMNVVAGTRFRGDFEERMNNIIKDIEEDGQVILFIDELHTIM
GSGSGIDSTLDAANILKPALARGTLRTVGATTQEEYQKHIEKDAALSRRFAKVTIEEPSVADSMTILQGLKATYEKHHRV
QITDEAVETAVKMAHRYLTSRHLPDSAIDLLDEAAATVQNKAKHVKADDSDLSPADKALMDGKWKQAAQLIAKEEEV
PVYKDLVTESDILTTLSRLSGIPVQKLTQTDAKKYLNLEAELHKRVIGQDQAVSSISRAIRRNQSGIRSHKRPIGSFMFLGP
TGVGKTELAKALAEVLFDDESALIRFDMSEYMEKFAASRLNGAPPGYVGYEEGGELTEKVRNKPYSVLLFDEVEKAHP
DIFNVLLQVLDDGVLTDSKGRKVDFSNTIIIMTSNLGATALRDDKTVGFGAKDIRFDQENMEKRMFEELKKAYRPEFIN
RIDEKVVFHSLSSDHMQEVVKIMVKPLVASLTEKGIDLKLQASALKLLANQGYDPEMGARPLRRTLQTEVEDKLAELL
LKGDLVAGSTLKIGVKAGQLKFDIAZ

ID40 1008bp

ATGAAGAAAACATGGAAAGTGTTTTTAACGCTTGTAACAGCTCTTGTAGCTGTTGTGCTTGTGGCCTGTGGTCAAG
GAACTGCTTCTAAAGACAACAAAGAGGCAGAACTTAAGAAGGTTGACTTTATCCTAGACTGGACACCAAATACCA
ACCACACAGGGCTTTATGTTGCCAAGGAAAAAGGTTATTTCAAAGAAGCTGGAGTGGATGTTGATTTGAAATTGC
CACCAGAAGAAAGTTCTTCTGACTTGGTTATCAACGGAAAGGCACCATTTGCAGTGTATTTCCAAGACTACATGGC
TAAGAAATTGGAAAAAGGAGCAGGAATCACTGCCGTTGCAGCTATTGTTGAACACAATACATCAGGAATCATCTC
TCGTAAATCTGATAATGTAAGCAGTCCAAAAGACTTGGTTGGTAAGAAATATGGGACATGGAATGACCCAACTGA
ACTTGCTATGTTGAAAACCTTGGTAGAATCTCAAGGTGGAGACTTTGAGAAGGTTGAAAAAGTACCAAATAACGA
CTCAAACTCAATCACACCGATTGCCAATGGCGTCTTTGATACTGCTTGGATTTACTACGGTTGGGATGGTATCCTT
GCTAAATCTCAAGGTGTAGATGCTAACTTCATGTACTTGAAAGACTATGTCAAGGAGTTTGACTACTATTCACCAG
TTATCATCGCAAACAACGACTATCTGAAAGATAACAAAGAAGAAGCTCGCAAAGTCATCCAAGCCATCAAAAAA
GGCTACCAATATGCCATGGAACATCCAGAAGAAGCTGCAGATATTCTCATCAAGAATGCACCTGAACTCAAGGAA
AAACGTGACTTTGTCATCGAATCTCAAAAATACTTGTCAAAAGAATACGCAAGCGACAAGGAAAAATGGGGTCAA
TTTGACGCAGCTCGCTGGAATGCTTTCTACAAATGGGATAAAGAAAATGGTATCCTTAAAGAAGACTTGACAGAC
AAAGGCTTCACCAACGAATTTGTGAAATAA

MKKTWKVFLTLVTALVAVVLVACGQGTASKDNKEAELKKVDFILDWTPNTNHTGLYVAKEKGYFKEAGVDVDLKLP
PEESSSDLVINGKAPFAVYFQDYMAKKLEKGAGITAVAAIVEHNTSGIISRKSDNVSSPKDLVGKKYGTWNDPTELAML
KTLVESQGGDFEKVEKVPNNDSNSITPIANGVFDTAWIYYGWDGILAKSQGVDANFMYLKDYVKEFDYYSPVIIANND
YLKDNKEEARKVIQAIKKGYQYAMEHPEEAADILIKNAPELKEKRDFVIESQKYLSKEYASDKEKWGQFDAARWNAFY
KWDKENGILKEDLTDKGFTNEFVKZ

ID41 762bp

TTGATGAGAAACTTGAGAAGTATACTGAGACGACACATTAGTCTATTGGGCTTTCTCGGAGTATTGTCAATCTGGC
AGTTAGCAGGTTTTCTTAAACTTCTCCCCAAGTTTATCCTGCCGACACCTCTTGAAATTCTCCAGCCCTTTGTTCGT
GACAGAGAATTTCTCTGGCACCATAGCTGGGCGACCTTGAGAGTGGCTTTACTGGGGCTGATTTTGGGAGTTTTGA

TTGCCTGTCTTATGGCTGTGCTCATGGATAGTTTGACTTGGCTCAATGACCTGATTTACCCTATGATGGTGGTCATT
CAGACCATTCCGACCATTGCCATAGCTCCTATCCTGGTCTTGTGGCTAGGTTATGGGATTTTGCCCAAGATTGTCT
TGATTATCTTAACGACAACCTTTCCCATCATCGTTAGTATTTTGGACGGTTTTAGGCATTGCGACAAGGATATGCT
GACCTTGTTTAGTCTGATGCGGGCCAAGCCTTGGCAAATCCTGTGGCATTTTAAAATCCCAGTTAGCCTGCCTTAC
TTTTATGCAGGTCTGAGGGTCAGTGTCTCCTACGCCTTTATCACAACTGTGGTATCTGAGTGGTTGGGAGGTTTTG
AAGGTCTTGGTGTTTATATGATTCAGTCTAAAAAACTGTTTCAGTATGATACCATGTTTGCCATTATTATTCTGGTG
TCGATTATCAGTCTTTTGGGTATGAAGCTGGTCGATATCAGTGAAAAATATGTGATTAAATGGAAACGTTCGTAG

MMRNLRSILRRHISLLGFLGVLSIWQLAGFLKLLPKFILPTPLEILQPFVRDREFLWHHSWATLRVALLGLILGVLIACLM
AVLMDSLTWLNDLIYPMMVVIQTIPTIAIAPILVLWLGYGILPKIVLIILTTTFPIIVSILDGFRHCDKDMLTLFSLMRAKP
WQILWHFKIPVSLPYFYAGLRVSVSYAFITTVVSEWLGGFEGLGVYMIQSKKLFQYDTMFAIILVSIISLLGMKLVDISE
KYVIKWKRSZ

ID42 372bp

TTGATTTTTAATCCTATTTGCTGTATGATAAGGGAAAAGAAAGGGGACAGAGATATGGCTTTTACCAATACCCACA
TGCGATCTGCTAGTTTTGGTATTGTTACCAGCTTGCCTGATGACATCATTGACTCTTTTTGGTATATCATCGACCAT
TTCTTAAAAAATGTCTTTGAATTGGAAGAAGAACTCGAGTTTCAATTGCTTAATAACCAAGGAAAGATTACCTTCC
ACTTTTCAAGTCAACACCTCCCTACAGCCATTGATTTTGACTTTAACCATCCTTTCGACCCTCGTTATCCCCCAAGA
GTACTGGTTTTAGACATGGACGGTAGAGAAACTATCCTCCTCCCAGAAGAAAATGACCTATTTTAA

MIFNPICCMIREKKGDRDMAFTNTHMRSASFGIVTSLPDDIIDSFWYIIDHFLKNVFELEEELEFQLLNNQGKITFHFSSQ
HLPTAIDFDFNHPFDPRYPPRVLVLDMDGRETILLPEENDLFZ

ID43 1569bp

ACAGCGGTGTCATTCTATCTATTTTAAGAAAAGTAATAATCAATTGTTAAAAATAGTAAAAAAATTGGAGGTTCTG
ATGAAATATTTTGTTCCTAATGAGGTATTCAGTATTCGTAAATTAAAGGTGGGGACTTGCTCGGTACTATTGGCAA
TTTCAATTTTGGGAAGCCAAGGTATTTTATCGGATGAAGTTGTTACTAGTTCTTCACCGATGGCTACAAAAGAGTC
TTCTAATGCAATTACTAATGATTTAGATAATTCACCAACTGTTAATCAGAATCGTTCTGCTGAAATGATTGCCTCT
AATTCAACCACTAATGGTTTAGATAATTCGTTAAGTGTTAATAGCATCAGCTCTAATGGTACTATTCGTTCCAATT
CACAATTAGACAACAGAACAGTTGAATCTACAGTAACATCTACTAATGAAAATAAGAGTTATAAGGAAGATGTTA
TAAGTGACAGAATTATCAAAAAAGAATTTGAAGATACTGCTTTAAGTGTAAAAGATTATGGTGCAGTAGGTGATG
GGATTCATGATGATCGACAAGCAATTCAAGATGCAATAGATGCTGCAGCTCAAGGGCTAGGTGGAGGAAATGTAT
ATTTTCCTGAAGGAACTTATTTAGTAAAAGAAATTGTTTTTTTAAAAAGTCATACACACTTAGAATTGAATGAGAA
AGCTACAATTCTAAATGGTATAAATATTAAGAATCACCCTTCCATTGTTTTTATGACAGGTTTATTTACGGATGAT
GGTGCGCAAGTAGAATGGGGCCCAACAGAAGATATTAGTTATTCTGGTGGTACGATTGATATGAACGGTGCTTTG
AATGAAGAAGGAACTAAAGCAAAAAATCTACCACTTATAAATTCTTCAGGTGCATTTGCTATTGGGAATTCAAAT
AACGTAACTATAAAAAATGTAACATTCAAGGATAGTTATCAAGGGCATGCTATTCAAATTGCAGGTTCGAAAAAT
GTATTAGTTGATAATTCTCGTTTTCTTGGGCAAGCCTTACCCAAAACGATGAAGGATGGCAAATCATAAGTAAGG
AGAGCATTCAGATTGAACCATTAACTAGAAAAGGTTTTCCTTATGCCTTGAATGATGATGGGAAAAAATCTGAAA
ATGTGACTATTCAAAATTCCTATTTTGGCAAAAGTGATAAATCTGGGGAATTAGTAACAGCAATTGGCACACACTA
TCAAACATTGTCGACACAGAACCCCTCTAATATTAAAATTCAAAATAATCATTTTGATAACATGATGTATGCAGGT
GTACGTTTTACAGGATTCACTGATGTATTAATCAAAGGAAATCGCTTTGATAAGAAAGTTAAAGGAGAGAGTGTA
CATTATCGAGAAAGCGGAGCAGCTTTAGTAAATGCTTATAGCTATAAAAACACTAAAGACCTATTAGATTTAAAT
AAACAGGTGGTTATCGCCGAAAATATATTTAATATTGCCGATCCTAAAACAAAAGCGATACGAGTTGCAAAAGAT
AGTGCAGAATGTTTAGGAAAAGTATCAGATATTACTGTAACAAAAAATGTAATTAATAATAATTCTAAGGAAACA
GAACAACCAAATATTGAATTATTACGAGTTAGTGATAATTTAGTAGTCTCAGAGAATAGT

QRCHSIYFKKSNNQLLKIVKKLEVLMKYFVPNEVFSIRKLKVGTCSVLLAISILGSQGILSDEVVTSSSPMATKESSNAITN
DLDNSPTVNQNRSAEMIASNSTTNGLDNSLSVNSISSNGTIRSNSQLDNRTVESTVTSTNENKSYKEDVISDRIIKKEFEDT
ALSVKDYGAVGDGIHDDRQAIQDAIDAAAQGLGGGNVYFPEGTYLVKEIVFLKSHTHLELNEKATILNGINIKNHPSIVF
MTGLFTDDGAQVEWGPTEDISYSGGTIDMNGALNEEGTKAKNLPLINSSGAFAIGNSNNVTIKNVTFKDSYQGHAIQIA
GSKNVLVDNSRFLGQALPKTMKDGQIISKESIQIEPLTRKGFPYALNDDGKKSENVTIQNSYFGKSDKSGELVTAIGTHY
QTLSTQNPSNIKIQNNHFDNMMYAGVRFTGFTDVLIKGNRFDKKVKGESVHYRESGAALVNAYSYKNTKDLLDLNKQ
VVIAENIFNIADPKTKAIRVAKDSAECLGKVSDITVTKNVINNNSKETEQPNIELLRVSDNLVVSENS

ID44 324bp

GTGATGAAAGAAACTCAGCTATTAAAAGGTGTTCTTGAAGGTTGTGTCTTGGATATGATTGGTCAAAAAGAGCGG
TATGGTTATGAGTTGGTTCAGACTTTGCGAGAGGCTGGATTTGATACTATCGTTCCAGGAACTATTTATCCTTTGTT
GCAAAAGTTAGAAAAAAATCAATGGATAAGAGGCGACATGCGCCCGTCGCCAGATGGTCCAGATCGGAAGTATTT
TTCATTAATGAAAGAAGGAGAAGAGCGTGTCTCAGTCTTTTGGCAACAATGGGACGATTTGAGTCAAAAAGTAGA
AGGGATTAAGAATGGGGGTTAA

MMKETQLLKGVLEGCVLDMIGQKERYGYELVQTLREAGFDTIVPGTIYPLLQKLEKNQWIRGDMRPSPDGPDRKYFSL
MKEGEERVSVFWQQWDDLSQKVEGIKNGGZ

ID45 816bp

ATGAAGAAAATGAAGTATTACGAAGAAACAAGCGCTTTGCTACATGAGTTTTCTGAGGAGAATCAAAAGTATTTT
GAGGAGTTGTGGGAAAGTTTTAATCTTGCTGGATTTCTCTATGATGAAGACTATCTCAGAGAGCAGATCTATTTGA
TGATGCTAGATTTCTCAGAAGCAGAACGAGATGGCATGAGTGCAGAGGATTATCTAGGTAAGAATCCTAAAAAAA
TAATGAAAGAGATTCTCAAGGGAGCACCTCGCAGTTCTATCAAAGAGTCCCTTTTGACGCCAATTCTTGTCCTGGC
GGTATTACGTTATTATCAACTACTAAGTGATTTTTCTAAAGGTCCTCTCTTAACAGTCAATTTGCTCACATTTTTAG
GGCAACTTCTTATTTTTCTGATTGGATTTGGACTTGTGGCCACAATTTTACGAAGAAGTTTAGTCCAAGATTCTCCT
AAAATGAAAATTGGCACTTACATTGTTGTTGGGACTATAGTTCTTCTAGTTGTTTTAGGATATGTAGGAATGGCAA
GCTTCATACAAGAAGGAGCCTTTTATATTCCGGCTCCCTGGGATAGTTTGTCTGTCTTTACGATTTCGCTAGTTATC
GGTATTTGGAATTGGAAAGAAGCGGTCTTTCGTCCATTTGTCAGTATGATTATTGCCCATCTTGTGGTGGGTTCTCT
GCTCCGTTATTATGAGTGGATGGGAATTTCAAATGTTTTCCTTACAAAAGTTATTCCTTTAGCTGTCCTCTTTATTG
GAATCTTTGTCTTGTTCCGTGGGTTTAAGAAGATAAAATGGAGTGAAGTATAG

MKKMKYYEETSALLHEFSEENQKYFEELWESFNLAGFLYDEDYLREQIYLMMLDFSEAERDGMSAEDYLGKNPKKIM
KEILKGAPRSSIKESLLTPILVLAVLRYYQLLSDFSKGPLLTVNLLTFLGQLLIFLIGFGLVATILRRSLVQDSPKMKIGTYI
VVGTIVLLVVLGYVGMASFIQEGAFYIPAPWDSLSVFTISLVIGIWNWKEAVFRPFVSMIIAHLVVGSLLRYYEWMGISN
VFLTKVIPLAVLFIGIFVLFRGFKKIKWSEVZ

ID46 348bp

CTGTTTTTTTATTTATACTCAATGAAAATCAAAGAGCAAACTAGGAAGCTAGCCGCAGGTTGCTCAAAACACTGTT
TTGAGGTTGTAGACGAAACTGACGAAGTCAGCTCAAAACATGTTTTTGAGGTTGTAGATGAAACTGACGAAGTCA
GCTCAAAACACTGTTTTGAGGTTGTAGATGAAACTGACGAAGTCAGCTCAAAACACTGTTTTGAGGTTGTAGATG
AAACTGACGAAGTCAGCTCAAAACATGTTTTTGAGGTTGTAGATGAAACTGACGAAGTCAGTAACCATACATACG
GTAGGGCGACGCTGACGTGGTTTGAAGAGATTTTCGAAGAGTATTAA

MFFYLYSMKIKEQTRKLAAGCSKHCFEVVDETDEVSSKHVFEVVDETDEVSSKHCFEVVDETDEVSSKHCFEVVDETD
EVSSKHVFEVVDETDEVSNHTYGRATLTWFEEIFEEYZ

ID47 1260bp

ATGCAGAATCTGAAATTTGCCTTTTCATCTATCATGGCTCACAAGATGCGTTCTTTGCTTACTATGATTGGGATTAT
TATCGGTGTTTCATCAGTTGTTGTGATTATGGCTTTGGGTGATTCCCTATCTCGTCAAGTCAATAAAGATATGACTA
AATCTCAGAAAAATATTAGCGTCTTTTTCTCTCCTAAAAAAAGTAAAGACGGGTCTTTTACTCAGAAACAATCAGC
TTTTACGGTTTCTGGAAAGGAAGAGGAAGTTCCTGTTGAACCGCCAAAACCGCAAGAATCCTGGGTCCAAGAGGC
AGCTAAACTGAAGGGAGTGGATAGTTACTATGTAACCAATTCAACGAATGCCATCTTGACCTATCAAGATAAAAA
GGTTGAGAATGCTAATTTGACAGGTGGAAACAGAACTTACATGGACGCTGTTAAGAATGAAATTATTGCAGGTCG
TAGTCTGAGAGAGCAAGATTTCAAAGAGTTTGCAAGTGTCATTTTGCTAGATGAGGAATTGTCCATTAGTTTATTT
GAATCTCCTCAAGAGGCTATTAACAAGGTTGTAGAAGTCAATGGATTTAGTTACCGGGTCATTGGGGTTTATACTA
GTCCGGAGGCTAAAAGATCAAAAATATATGGGTTTGGTGGCTTGCCTATTACTACCAATATCTCCCTTGCTGCGAA
TTTTAATGTAGATGAAATAGCTAATATTGTCTTTCGAGTGAATGATACCAGTTTAACCCCAACTCTGGGTCCAGAA
CTGGCACGAAAAATGACAGAGCTTGCAGGCTTACAACAGGGAGAATACCAGGTGGCAGATGAGTCCGTTGTATTT
GCAGAAATTCAACAATCGTTTAGTTTTATGACGACGATTATTAGTTCCATCGCAGGGATTTCTCTCTTTGTTGGAG
GAACTGGTGTCATGAACATCATGCTGGTTTCGGTGACAGAGCGCACTCGTGAGATTGGTCTTCGTAAGGCTTTGGG
TGCAACACGTGCCAATATTTTAATTCAGTTTTTGATTGAATCCATGATTTTGACCTTGTTAGGTGGCTTAATTGGCT
TGACAATTGCAAGTGGTTTAACTGCCTTAGCAGGTTTGTTACTGCAAGGTTTAATAGAAGGTATAGAAGTTGGAGT
ATCAATCCCAGTCGCCCTATTTAGTCTTGCAGTTTCGGCTAGTGTTGGTATGATTTTTGGAGTCTTGCCAGCCAAC
AAGGCATCGAAACTTGATCCAATTGAAGCCCTTCGTTATGAATGA

MQNLKFAFSSIMAHKMRSLLTMIGIIIGVSSVVVIMALGDSLSRQVNKDMTKSQKNISVFFSPKKSKDGSFTQKQSAFTVS
GKEEEVPVEPPKPQESWVQEAAKLKGVDSYYVTNSTNAILTYQDKKVENANLTGGNRTYMDAVKNEIIAGRSLREQDF
KEFASVILLDEELSISLFESPQEAINKVVEVNGFSYRVIGVYTSPEAKRSKIYGFGGLPITTNISLAANFNVDEIANIVFRVN
DTSLTPTLGPELARKMTELAGLQQGEYQVADESVVFAEIQQSFSFMTTIISSIAGISLFVGGTGVMNIMLVSVTERTREIG
LRKALGATRANILIQFLIESMILTLLGGLIGLTIASGLTALAGLLLQGLIEGIEVGVSIPVALFSLAVSASVGMIFGVLPANK
ASKLDPIEALRYEZ

ID48 705bp

CTGATGAAGCAACTAATTAGTCTAAAAAATATCTTCAGAAGTTACCGTAATGGTGACCAAGAACTGCAGGTTCTC
AAAAATATCAATCTAGAAGTGAATGAGGGTGAATTTGTAGCCATCATGGGACCATCTGGGTCTGGTAAGTCCACT
CTGATGAATACGATTGGCATGTTGGATACACCAACCAGTGGAGAATATTATCTTGAAGGTCAAGAAGTGGCTGGG

CTTGGTGAAAAACAACTAGCTAAGGTCCGTAACCAACAAATCGGTTTTGTCTTTCAGCAGTTCTTTCTTCTATCGA
AGCTCAATGCTCTGCAAAATGTAGAATTGCCCTTGATTTACGCAGGAGTTTCGTCTTCAAAACGTCGCAAGTTGGC
TGAGGAATATTTAGACAAGGTTGAATTGACAGAACGTAGTCACCATTTACCTTCAGAATTATCTGGTGGTCAAAA
GCAACGTGTAGCCATTGCGCGTGCCTTGGTAAACAATCCTTCTATTATCCTAGCGGATGAACCGACAGGAGCCTTG
GATACCAAAACAGGTAACCAAATTATGCAATTATTGGTTGATTTGAATAAAGAAGGAAAAACCATTATCATGGTA
ACGCATGAGCCTGAGATTGCTGCCTATGCCAAACGTCAGATTGTCATTCGGGATGGGGTCATTTCGTCTGACAGTG
CTCAGTTAGGAAAGGAGGAAAACTAA

MMKQLISLKNIFRSYRNGDQELQVLKNINLEVNEGEFVAIMGPSGSGKSTLMNTIGMLDTPTSGEYYLEGQEVAGLGEK
QLAKVRNQQIGFVFQQFFLLSKLNALQNVELPLIYAGVSSSKRRKLAEEYLDKVELTERSHHLPSELSGGQKQRVAIARA
LVNNPSIILADEPTGALDTKTGNQIMQLLVDLNKEGKTIIMVTHEPEIAAYAKRQIVIRDGVISSDSAQLGKEENZ

ID49 1200bp

ATGAAGAAAAAGAATGGTAAAGCTAAAAAGTGGCAACTGTATGCAGCAATCGGTGCTGCGAGTGTAGTTGTATTG
GGTGCTGGGGGGATTTTACTCTTTAGACAACCTTCTCAGACTGCTCTAAAAGATGAGCCTACTCATCTTGTTGTTG
CCAAGGAAGGAAGCGTGGCCTCCTCTGTTTTATTGTCAGGGACAGTAACAGCAAAAAATGAACAATATGTTTATT
TTGATGCTAGTAAGGGTGATTTAGATGAAATCCTTGTTTCTGTGGGCGATAAGGTCAGCGAAGGGCAGGCTTTAGT
CAAGTACAGTAGTTCAGAAGCGCAGGCGGCCTATGATTCAGCTAGTCGAGCAGTAGCTAGGGCAGATCGTCATAT
CAATGAACTCAATCAAGCACGAAATGAAGCCGCTTCAGCTCCGGCTCCACAGTTACCAGCGCCAGTAGGAGGAGA
AGATGCAACGGTGCAAAGCCCAACTCCAGTGGCTGGAAATTCTGTTGCTTCTATTGACGCTCAATTGGGTGATGCC
CGTGATGCGCGTGCAGATGCTGCGGCGCAATTAAGCAAGGCTCAAAGTCAATTGGATGCAACAACTGTTCTCAGT
ACCCTAGAGGGAACTGTGGTCGAAGTCAATAGCAATGTTTCTAAATCTCCAACAGGGGCGAGTCAAGTTATGGTT
CATATTGTCAGCAATGAAAATTTACAAGTCAAGGGAGAATTGTCTGAGTACAATCTAGCCAACCTTTCTGTAGGTC
AAGAAGTAAGCTTTACTTCTAAAGTGTATCCTGATAAAAAATGGACTGGGAAATTAAGCTATATTTCTGACTATCC
TAAAAACAATGGTGAAGCAGCTAGTCCAGCAGCCGGGAATAATACAGGTTCTAAATACCCTTATACTATTGATGT
GACAGGCGAGGTTGGTGATTTGAAACAAGGTTTTTCTGTCAACATTGAGGTTAAAAGCAAAACTAAGGCTATTCTT
GTTCCTGTTAGCAGTCTAGTAATGGATGATAGTAAAAATTATGTCTGGATTGTGGATGAACAACAAAAGGCTAAA
AAAGTTGAGGTTTCATTGGGAAATGCTGACGCAGAAAATCAAGAAATCACTTCTGGTTTAACGAACGGTGCTAAG
GTCATCAGTAATCCAACATCTTCCTTGGAAGAAGGAAAAGAGGTGAAGGCTGATGAAGCAACTAATTAG

MKKKNGKAKKWQLYAAIGAASVVVLGAGGILLFRQPSQTALKDEPTHLVVAKEGSVASSVLLSGTVTAKNEQYVYFD
ASKGDLDEILVSVGDKVSEGQALVKYSSSEAQAAYDSASRAVARADRHINELNQARNEAASAPAPQLPAPVGGEDATV
QSPTPVAGNSVASIDAQLGDARDARADAAAQLSKAQSQLDATTVLSTLEGTVVEVNSNVSKSPTGASQVMVHIVSNEN
LQVKGELSEYNLANLSVGQEVSFTSKVYPDKKWTGKLSYISDYPKNNGEAASPAAGNNTGSKYPYTIDVTGEVGDLKQ
GFSVNIEVKSKTKAILVPVSSLVMDDSKNYVWIVDEQQKAKKVEVSLGNADAENQEITSGLTNGAKVISNPTSSLEEGKE
VKADEATNZ

ID50 759bp

ATGTCACGTAAACCATTTATCGCTGGTAACTGGAAAATGAACAAAAATCCAGAAGAAGCTAAAGCATTCGTTGAA
GCAGTTGCATCAAAACTTCCTTCATCAGATCTTGTTGAAGCAGGTATCGCTGCTCCAGCTCTTGATTTGACAACTG
TTCTTGCTGTTGCAAAAGGCTCAAACCTTAAAGTTGCTGCTCAAAACTGCTACTTTGAAAATGCAGGTGCTTTCAC
TGGTGAAACTAGCCCACAAGTTTTGAAAGAAATCGGTACTGACTACGTTGTTATCGGTCACTCAGAACGCCGTGA
CTACTTCCATGAAACTGATGAAGATATCAACAAAAAAGCAAAAGCAATCTTTGCGAACGGTATGCTTCCAATCAT
CTGTTGTGGTGAATCACTTGAAACTTACGAAGCTGGTAAAGCTGCTGAATTCGTAGGTGCTCAAGTATCTGCTGCA
TTGGCTGGATTGACTGCTGAACAAGTTGCTGCCTCAGTTATCGCTTATGAGCCAATCTGGGCTATCGGTACTGGTA
AATCAGCTTCACAAGACGATGCACAAAAAATGTGTAAAGTTGTTCGTGACGTTGTAGCTGCTGACTTTGGTCAAG
AAGTCGCAGACAAAGTTCGTGTTCAATACGGTGGTTCTGTTAAACCTGAAAATGTTGCTTCATACATGGCTTGCCC
AGACGTTGACGGTGCCCTTGTAGGTGGTGCGTCACTTGAAGCTGAAAGCTTCTTGGCTTTGCTTGACTTTGTAAAA
TAA

MSRKPFIAGNWKMNKNPEEAKAFVEAVASKLPSSDLVEAGIAAPALDLTTVLAVAKGSNLKVAAQNCYFENAGAFTG
ETSPQVLKEIGTDYVVIGHSERRDYFHETDEDINKKAKAIFANGMLPIICCGESLETYEAGKAAEFVGAQVSAALAGLTA
EQVAASVIAYEPIWAIGTGKSASQDDAQKMCKVVRDVVAADFGQEVADKVRVQYGGSVKPENVASYMACPDVDGAL
VGGASLEAESFLALLDFVKZ

ID51 1473bp

TTGAAAACAAAAAATTGGATTAGCAAGTATCTGTTTACTAGGCTTGGCAACTAGTCATGTCGCTGCAAATGAAACTG
AAGTAGCAAAAACTTCGCAGGATACAACGACAGCTTCAAGTAGTTCAGAGCAAAATCAGTCTTCTAATAAAACGC
AAACGAGCGCAGAAGTACAGACTAATGCTGCTGCCCACTGGGATGGGGATTATTATGTAAAGGATGATGGTTCTA
AAGCTCAAAGTGAATGGATTTTTGACAACTACTATAAGGCTTGGTTTTATATTAATTCAGATGGTCGTTACTCGCA
GAATGAATGGCATGGAAATTACTACCTGAAATCAGGTGGATATATGGCCCAAAACGAGTGGATCTATGACAGTAA
TTACAAGAGTTGGTTTTATCTCAAGTCAGATGGGGCTTATGCTCATCAAGAATGGCAATTGATTGGAAATAAGTGG

TACTACTTCAAGAAGTGGGGTTACATGGCTAAAAGCCAATGGCAAGGAAGTTATTTCTTGAATGGTCAAGGAGCT
ATGATGCAAAATGAATGGCTCTATGATCCAGCCTATTCTGCTTATTTTTATCTAAAATCCGATGGAACTTATGCTA
ACCAAGAGTGGCAAAAAGTGGGCGGCAAATGGTACTATTTCAAGAAGTGGGGCTATATGGCTCGGAATGAGTGGC
AAGGCAACTACTATTTGACTGGAAGTGGTGCCATGGCGACTGACGAAGTGATTATGGATGGTACTCGCTATATCTT
TGCGGCCTCTGGTGAGCTCAAAGAAAAAAAAGATTTGAATGTCGGCTGGGTTCACAGAGATGGTAAGCGCTATTT
CTTTAATAATAGAGAAGAACAAGTGGGAACCGAACATGCTAAGAAAGTCATTGATATTAGTGAGCACAATGGTCG
TATCAATGATTGGAAAAAGGTTATTGATGAGAACGAAGTGGATGGTGTCATTGTTCGTCTAGGTTATAGCGGTAA
AGAAGACAAGGAATTGGCGCATAACATTAAGGAGTTAAACCGTCTGGGAATTCCTTATGGTGTCTATCTCTATAC
CTATGCTGAAAATGAGACCGTGCTGAGAGTGACGCTAAACAGACCATTGAACTTATAAAGAAATACAATATGAAC
CTGTCTTACCCTATCTATTATGATGTTGAGAATTGGGAATATGTAAATAAGAGCAAGAGAGCTCCAAGTGATACA
GGCACTTGGGTTAAAATCATCAACAAGTACATGGACACGATGAAGCAGGCGGGTTATCAAAATGTGTATGTCTAT
AGCTATCGTAGTTTATTACAGACGCGTTTAAAACACCCAGATATTTTAAAACATGTAAACTGGGTAGCGGCCTATA
CGAATGCTTTAGAATGGGAAAACCCTCATTATTCAGGAAAAAAAGGTTGGCAATATACCTCTTCTGAATACATGA
AAGGAATCCAAGGGCGCGTAGATGTCAGCGTTTGGTATTAA

MKTKIGLASICLLGLATSHVAANETEVAKTSQDTTTASSSSEQNQSSNKTQTSAEVQTNAAAHWDGDYYVKDDGSKAQ
SEWIFDNYYKAWFYINSDGRYSQNEWHGNYYLKSGGYMAQNEWIYDSNYKSWFYLKSDGAYAHQEWQLIGNKWYY
FKKWGYMAKSQWQGSYFLNGQGAMMQNEWLYDPAYSAYFYLKSDGTYANQEWQKVGGKWYYFKKWGYMARNE
WQGNYYLTGSGAMATDEVIMDGTRYIFAASGELKEKKDLNVGWVHRDGKRYFFNNREEQVGTEHAKKVIDISEHNG
RINDWKKVIDENEVDGVIVRLGYSGKEDKELAHNIKELNRLGIPYGVYLYTYAENETDAESDAKQTIELIKKYNMNLSY
PIYYDVENWEYVNKSKRAPSDTGTWVKIINKYMDTMKQAGYQNVYVYSYRSLLQTRLKHPDILKHVNWVAAYTNAL
EWENPHYSGKKGWQYTSSEYMKGIQGRVDVSVWYZ

ID52 774bp

ATGAAAAAATTTGCCAACCTTTATCTGGGACTGGTCTTTCTGGTCCTCTACCTGCCTATCTTTTACTTGATTGGCTA
TGCCTTTAATGCTGGTGATGATATGAATAGCTTTACAGGTTTTAGCTGGACTCACTTTGAAACCATGTTTGGAGAT
GGGAGACTCATGCTGATTTTGGCTCAGACATTTTTCTTGGCCTTCCTATCAGCCTTGATAGCGACCATTATCGGGA
CTTTTGGTGCCATTTACATCTACCAGTCTCGTAAGAAATACCAAGAAGCCTTTCTATCACTCAATAATATCCTCAT
GGTTGCGCCTGACGTTATGATTGGTGCTAGCTTCTTGATTCTCTTTACCCAACTCAAGTTTTCACTTGGCTTTTTGA
CCGTTCTATCTAGTCACGTGGCCTTCTCCATTCCTATCGTGGTCTTGATGGTCTTGCCTCGACTCAAGGAAATGAA
TGGCGACATGATTCATGCGGCCTATGACTTGGGAGCTAGTCAATTTCAGATGTTCAAGGAAATCATGCTTCCTTAC
CTGACTCCGTCTATCATTACTGGTTATTTCATGGCCTTCACCTATTCGTTAGATGACTTTGCCGTGACCTTCTTTGT
AACAGGAAATGGCTTTTCAACCCTATCAGTCGAGATTTACTCTCGTGCTCGCAAGGGGATTTCCTTAGAAATCAAT
GCCCTGTCTGCTCTAGTCTTTCTCTTTAGTATTATCCTAGTTGTAGGTTATTACTTTATCTCTCGTGAGAAGGAGGA
GCAAGCATGA

MKKFANLYLGLVFLVLYLPIFYLIGYAFNAGDDMNSFTGFSWTHFETMFGDGRLMLILAQTFFLAFLSALIATIIGTFGA
IYIYQSRKKYQEAFLSLNNILMVAPDVMIGASFLILFTQLKFSLGFLTVLSSHVAFSIPIVVLMVLPRLKEMNGDMIHAAY
DLGASQFQMFKEIMLPYLTPSIITGYFMAFTYSLDDFAVTFFVTGNGFSTLSVEIYSRARKGISLEINALSALVFLFSIILVV
GYYFISREKEEQAZ

ID59 1071bp

ATGAAAAAAATCTATTCATTTTTAGCAGGAATTGCAGCGATTATCCTTGTCTTGTGGGGAATTGCGACTCATTTAG
ATAGTAAAATCAATAGTCGAGATAGTCAAAAATTGGTTATCTATAACTGGGGAGACTATATCGATCCTGAACTCTT
GACTCAGTTTACAGAAGAAACAGGAATTCAAGTTCAGTACGAGACTTTTGACTCCAACGAAGCCATGTACACTAA
GATAAAGCAGGGTGGAACGACCTACGATATTGCCATTCCAAGTGAATACATGATTAACAAGATGAAGGACGAAG
ACCTCTTGGTTCCGCTTGATTATTCAAAAATTGAAGGAATCGAAAATATCGGACCAGAGTTTCTCAACCAGTCCTT
TGACCCAGGTAATAAATTCTCCATCCCTTACTTCTGGGGAACCTTAGGAATTGTCTACAACGAAACCATGGTAGAT
GAAGCGCCTGAGCATTGGGATGACCTTTGGAAGCCGGAGTATAAGAATTCTATCATGCTCTTTGATGGGGCGCGT
GAGGTGCTGGGACTAGGACTCAATTCCCTCGGCTACAGCCTCAACTCCAAGGATCTGCAGCAGTTGGAAGAGACA
GTGGATAAGCTCTACAAACTGACTCCAAATATCAAGGCTATCGTTGCGGACGAGATGAAGGGCTATATGATTCAG
AATAATGTTGCAATCGGCGTGACCTTCTCTGGTGAAGCCAGCCAAATGTTAGAAAAAAATGAAAATCTACGTTAT
GTGGTACCGACAGAGGCCAGCAATCTTTGGTTTGACAATATGGTCATTCCCAAAACAGTTAAAAACCAAAACTCA
GCCTATGCCTTTATCAACTTTATGTTGAAACCTGAAAATGCTCTCCAAAATGCGGAGTATGTCGGCTATTCAACAC
CAAACCTACCAGCGAAGGAATTGCTCCCAGAGGAAACAAAGGAAGATAAGGCCTTCTATCCCGATGTTGAAACCA
TGAAACACCTAGAAGTTTATGAGAAATTTGACCATAAATGGACAGGGAAATATAGCGACCTCTTCCTACAGTTTA
AAATGTATCGGAAGTAG

MKKIYSFLAGIAAIILVLWGIATHLDSKINSRDSQKLVIYNWGDYIDPELLTQFTEETGIQVQYETFDSNEAMYTKIKQGG
TTYDIAIPSEYMINKMKDEDLLVPLDYSKIEGIENIGPEFLNQSFDPGNKFSIPYFWGTLGIVYNETMVDEAPEHWDDLW
KPEYKNSIMLFDGAREVLGLGLNSLGYSLNSKDLQQLEETVDKLYKLTPNIKAIVADEMKGYMIQNNVAIGVTFSGEAS
QMLEKNENLRYVVPTEASNLWFDNMVIPKTVKNQNSAYAFINFMLKPENALQNAEYVGYSTPNLPAKELLPEETKED
KAFYPDVETMKHLEVYEKFDHKWTGKYSDLFLQFKMYRKZ

ID61 1851bp

ATGAATAAAAAACTAACAGATTATGTGATTGATCTGGTGGAAATTTTAAATAAACAACAAAAGCAGGTTTTCTGG
GGAATATTTGATATTTTCAGTATGGTGGTTTCCATCATTGTATCTTATATTTTATTTTATGGGCTGATTAATCCAGC
ACCTGTTGACTACATTATCTATACGAGTTTGGCCTTCCTGTTCTATCAATTGATGATTGGTTTTTGGGGGTTGAACG
CGAGCATTAGTCGTTACAGCAAGATTACGGATTTCATGAAAATCTTTTTTGGTGTGACTGCTAGCAGTGTCTTGTC
ATATAGTATCTGTTATGCCTTCTTGCCACTCTTCTCCATCCGTTTCATCATTCTCTTTATCTTGTTGAGTACCTTCTT
GATTTTATTGCCACGGATTACTTGGCAGTTAATCTACTCCAGACGCAAAAAAGGTAGTGGTGATGGAGAACACCG
TCGGACCTTCTTGATTGGTGCCGGTGATGGTGGGGCTCTTTTTATGGATAGTTACCAACATCCAACCAGTGAATTA
GAACTGGTCGGTATTTTGGATAAGGATTCTAAGAAAAAGGGTCAAAAACTTGGTGGTATTCCTGTTTTGGGCTCTT
ATGACAATCTGCCTGAATTAGCCAAACGCCATCAAATCGAGCGTGTCATCGTTGCGATTCCGTCGCTGGATCCGTC
AGAATATGAGCGTATCTTGCAGATGTGTAATAAGCTGGGTGTCAAATGTTACAAGATGCCTAAGGTTGAAACTGT
TGTTCAGGGCCTTCACCAAGCAGGTACTGGCTTCCAAAAAATTGATATTACGGACCTTTTGGGTCGTCAGGAAATC
CGTCTTGACGAATCGCGTCTGGGTGCAGAACTGACAGGTAAGACCATCTTAGTCACAGGAGCTGGAGGTTCAATC
GGTTCTGAAATCTGTCGTCAAGTTAGTCGCTTCAATCCTGAACGCATTGTCTTGCTCGGTCATGGGGAAAACTCAA
TCTACCTTGTTTATCATGAATTGATTCGTAAGTTCCAAGGGATTGATTATGTACCTGTGATTGCGGACATTCAAGA
CTATGATCGTTTGTTGCAAGTCTTTGAGCAGTACAAACCTGCTATTGTTTATCATGCGGCAGCCCACAAGCATGTT
CCTATGATGGAGCGCAATCCAAAAGAAGCCTTCAAAAACAATATCCGTGGAACTTACAATGTTGCTAAGGCTGTT
GATGAAGCTAAAGTGTCTAAGATGGTTATGATTTCGACAGATAAGGCAGTCAATCCACCAAATGTTATGGGAGCA
ACCAAGCGCGTGGCGGAGTTGATTGTCACTGGCTTTAACCAACGTAGCCAATCAACCTACTGTGCAGTTCGTTTTG
GGAATGTTCTTGGTAGCCGTGGTAGTGTCATTCCAGTCTTTGAACGTCAGATTGCTGAAGGTGGCCCTGTAACGGT
GACAGACTTCCGTATGACCCGTTACTTTATGACCATTCCAGAAGCTAGCCGTCTGGTTATCCATGCTGGTGCTTAT
GCCAAAGATGGGGAAGTCTTTATCCTTGATATGGGCAAACCAGTCAAGATTTATGACTTGGCCAAGAAGATGGTG
CTTCTAAGTGGCCACACTGAAAGTGAAATTCCAATCGTTGAAGTTGGAATCCGCCCAGGTGAAAAACTCTACGAA
GAACTCTTGGTATCAACCGAACTCGTTGATAATCAAGTTATGGATAAGATTTTCGTTGGTAAGGTTAATGTCATGC
CTTTAGAATCCATCAATCAAAAGATTGGAGAGTTCCGCACTCTCAGTGGAGATGAGTTGAAGCAAGCTATTATCG
CCTTTGCTAATCAAACAACCCACATTGAATAA

MNKKLTDYVIDLVEILNKQQKQVFWGIFDIFSMVVSIIVSYILFYGLINPAPVDYIIYTSLAFLFYQLMIGFWGLNASISRY
SKITDFMKIFFGVTASSVLSYSICYAFLPLFSIRFIILFILLSTFLILLPRITWQLIYSRRKKGSGDGEHRRTFLIGAGDGGALF
MDSYQHPTSELELVGILDKDSKKKGQKLGGIPVLGSYDNLPELAKRHQIERVIVAIPSLDPSEYERILQMCNKLGVKCYK
MPKVETVVQGLHQAGTGFQKIDITDLLGRQEIRLDESRLGAELTGKTILVTGAGGSIGSEICRQVSRFNPERIVLLGHGEN
SIYLVYHELIRKFQGIDYVPVIADIQDYDRLLQVFEQYKPAIVYHAAAHKHVPMMERNPKEAFKNNIRGTYNVAKAVD
EAKVSKMVMISTDKAVNPPNVMGATKRVAELIVTGFNQRSQSTYCAVRFGNVLGSRGSVIPVFERQIAEGGPVTVTDFR
MTRYFMTIPEASRLVIHAGAYAKDGEVFILDMGKPVKIYDLAKKMVLLSGHTESEIPIVEVGIRPGEKLYEELLVSTELV
DNQVMDKIFVGKVNVMPLESINQKIGEFRTLSGDELKQAIIAFANQTTHIEZ

ID101 1338bp

ATGATTGAACTTTATGATAGTTACAGTCAAGAAAGTCGAGATTTACATGAAAGTCTAGTCGCTACTGGTCTTTCTC
AACTTGGAGTGGTCATCGATGCAGATGGTTTTCTGCCTGATGGTCTGCTTTCTCCTTTTACCTATTATCTAGGTTAC
GAGGATGGAAAACCTCTCTATTTTAATCAAGTTCCCGTTTCAGATTTTTGGGAAATTTTAGGAGATAATCAGTCTG
CTTGTATTGAAGATGTGACGCAGGAGAGGGCTGTCATTCATTATGCTGATGGAATGCAGGCTCGCTTGGTTAAACA
GGTAGACTGGAAAGACCTAGAAGGTCGAGTACGTCAGGTTGACCACTACAATCGCTTCGGAGCTTGTTTTGCTAC
AACGACTTATAGCGCAGATAGCGAGCCGATTATGACAGTTTACCAAGATGTCAATGGTCAACAAGTTTTACTGGA
AAACCATGTGACGGGTGATATCTTATTGACTTTGCCAGGTCAGTCCATGCGTTACTTTGCAAATAAAGTTGAATTT
ATCACCTTCTTTTTGCAAGATTTGGAAATAGATACCAGTCAGCTTATCTTTAATACTCTAGCGACTCCTTTCTTGGT
TTCCTTCCATCATCCAGATAAATCTGGCTCGGATGTCTTGGTATGGCAGGAACCTCTCTATGATGCCATTCCAGGT
AATATGCAGTTGATTTTGGAAAGTGATAATGTGCGTACTAAGAAGATCATCATTCCAAATAAGGCGACTTATGAG
CGCGCTTTAGAGTTAACTGACGAGAAATACCATGATCAGTTTGTGCACTTGGGTTATCATTACCAGTTCAAACGTG
ATAATTTCCTAAGACGAGATGCCTTAATCTTGACCAATTCAGATCAGATTGAGCAAGTAGAAGCAATCGCAGGAG
CCTTGCCTGATGTCACTTTCCGTATTGCAGCGGTGACAGAGATGTCTTCTAAGCTCTTAGACATGCTTTGCTATCCT
AATGTGGCCCTTTACCAGAACGCTAGTCCACAGAAGATTCAGGAGCTGTATCAACTGTCGGATATTTACTTGGATA
TAAACCACAGTAATGAGTTGCTACAGGCAGTGCGTCAGGCCTTTGAGCACAATCTCTTGATTCTTGGCTTTAATCA
GACGGTGCACAATAGACTTTATATCGCTCCAGACCATCTATTTGAAAGTAGTGAAGTTGCTGCTTTGGTTGAGACC
ATTAAATTGGCCCTTTCAGATGTTGATCAAATGCGTCAGGCACTTGGCAAACAAGGCCAACATGCAAATTATGTTG
ACTTGGTGAGATATCAGGAAACCATGCAAACTGTTTTAGGAGGCTAA

MIELYDSYSQESRDLHESLVATGLSQLGVVIDADGFLPDGLLSPFTYYLGYEDGKPLYFNQVPVSDFWEILGDNQSACIE
DVTQERAVIHYADGMQARLVKQVDWKDLEGRVRQVDHYNRFGACFATTTYSADSEPIMTVYQDVNGQQVLLENHV
TGDILLTLPGQSMRYFANKVEFITFFLQDLEIDTSQLIFNTLATPFLVSFHHPDKSGSDVLVWQEPLYDAIPGNMQLILES
DNVRTKKIIIPNKATYERALELTDEKYHDQFVHLGYHYQFKRDNFLRRDALILTNSDQIEQVEAIAGALPDVTFRIAAVT
EMSSKLLDMLCYPNVALYQNASPQKIQELYQLSDIYLDINHSNELLQAVRQAFEHNLLILGFNQTVHNRLYIAPDHLFE
SSEVAALVETIKLALSDVDQMRQALGKQGQHANYVDLVRYQETMQTVLGGZ

ID102 1512bp

ATGACAATTTACAATATAAATTTAGGAATTGGTTGGGCTAGTAGCGGTGTTGAATACGCTCAAGCCTATCGTGCTG
GTGTTTTTCGGAAATTAAATCTGTCCTCTAAGTTTATCTTTACAGATATGATTTTAGCCGATAATATTCAGCACTTA
ACAGCCAATATTGGTTTTGATGATAATCAGGTTATCTGGCTTTATAATCATTTCACAGATATCAAAATTGCACCTA
CTAGCGTGACAGTGGATGATGTCTTGGCTTACTTTGGTGGTGAAGAAAGTCACAGAGAAAAAAATGGCAAGGTTT
TACGTGTATTCTTTTTTGACCAAGATAAGTTTGTAACCTGTTATTTGGTTGATGAGAACAAGGACTTGGTTCAACA
TGCCGAGTATGTTTTTAAGGGAAACCTGATTCGGAAGGATTACTTTTCTTATACGCGTTATTGTAGCGAGTATTTT
GCTCCCAAGGACAATGTTGCAGTCTTATACCAACGAACTTTTTATAATGAAGACGGGACTCCAGTCTATGATATCT
TGATGAATCAAGGGAAGGAAGAAGTTTATCATTTCAAGGATAAGATTTTCTATGGAAAGCAAGCTTTTGTGCGTG
CCTTTATGAAATCTTTGAATTTGAATAAGTCTGATTTGGTCATTCTCGATAGGGAGACAGGTATTGGACAGGTTGT
GTTTGAGGAAGCACAGACAGCACATCTAGCGGTAGTTGTTCATGCGGAGCATTATAGTGAAAATGCTACAAATGA
GGACTATATCCTTTGGAATAACTATTATGACTATCAGTTTACCAATGCAGATAAGGTTGACTTCTTTATCGTGTCT
ACTGATAGACAAAATGAAGTTCTACAAGAGCAATTTGCCAAATATACTCAGCATCAGCCAAAGATTGTTACCATT
CCTGTAGGCAGTATTGATTCCTTGACAGATTCAAGTCAAGGGCGCAAACCATTTTCATTGATTACGGCTTCACGTC
TTGCCAAAGAAAAGCACATTGATTGGCTTGTGAAAGCTGTGATTGAAGCTCATAAGGAGTTACCGGAACTAACCT
TTGATATCTATGGTAGTGGTGGAGAAGATTCTCTGCTTAGAGAAATTATTGCAAATCATCAGGCAGAGGACTATAT
CCAACTCAAGGGGCATGCGGAACTTTCGCAGATTTATAGCCAGTATGAGGTCTACTTAACGGCTTCTACCAGCGA
AGGATTTGGTCTGACCTTGATGGAAGCTATTGGTTCAGGTCTACCTCTAATTGGTTTTGATGTGCCTTATGGTAATC
AGACCTTTATAGAGGATGGGCAAAATGGTTATTTGATTCCAAGTTCATCTGACCATGTAGAAGACCAAATCAAGC
AAGCTTATGCCGCTAAGATTTGTCAATTGTATCAAGAAAATCGTTTGGAAGCTATGCGTGCCTATTCTTACCAAAT
TGCAGAAGGCTTCTTGACCAAAGAAATTTTAGAAAAGTGGAAGAAAACAGTAGAGGAGGTGCTCCATGATTGA

MTIYNINLGIGWASSGVEYAQAYRAGVFRKLNLSSKFIFTDMILADNIQHLTANIGFDDNQVIWLYNHFTDIKIAPTSVT
VDDVLAYFGGEESHREKNGKVLRVFFFDQDKFVTCYLVDENKDLVQHAEYVFKGNLIRKDYFSYTRYCSEYFAPKDN
VAVLYQRTFYNEDGTPVYDILMNQGKEEVYHFKDKIFYGKQAFVRAFMKSLNLNKSDLVILDRETGIGQVVFEEAQTA
HLAVVVHAEHYSENATNEDYILWNNYYDYQFTNADKVDFFIVSTDRQNEVLQEQFAKYTQHQPKIVTIPVGSIDSLTDS
SQGRKPFSLITASRLAKEKHIDWLVKAVIEAHKELPELTFDIYGSGGEDSLLREIIANHQAEDYIQLKGHAELSQIYSQYE
VYLTASTSEGFGLTLMEAIGSGLPLIGFDVPYGNQTFIEDGQNGYLIPSSSDHVEDQIKQAYAAKICQLYQENRLEAMRA
YSYQIAEGFLTKEILEKWKKTVEEVLHDZ

ID103 2292bp

ATGTCCTCTCTTTCGGATCAAGAATTAGTAGCTAAAACAGTAGAGTTTCGTCAGCGTCTTTCCGAGGGAGAAAGTC
TAGACGATATTTTGGTTGAAGCTTTTGCTGTGGTGCGTGAAGCAGATAAGCGGATTTTAGGGATGTTTCCTTATGA
TGTTCAAGTCATGGGAGCTATTGTCATGCACTATGGAAATGTTGCTGAGATGAATACGGGGGAAGGTAAGACCTT
GACAGCTACCATGCCTGTCTATTTGAACGCTTTTTCAGGAGAAGGAGTGATGGTTGTGACTCCTAATGAGTATTTA
TCAAAGCGTGATGCCGAGGAAATGGGTCAAGTTTATCGTTTTCTAGGATTGACCATTGGTGTACCATTTACGGAAG
ATCCAAAGAAGGAGATGAAAGCTGAAGAAAAGAAGCTTATCTATGCTTCGGATATCATCTACACAACCAATAGTA
ATTTAGGTTTTGATTATCTAAATGATAACCTAGCCTCGAATGAAGAAGGTAAGTTTTTACGACCGTTTAACTATGT
GATTATTGATGAAATTGATGATATCTTGCTTGATAGTGCACAAACTCCTCTGATTATTGCGGGTTCTCCTCGTGTTC
AGTCTAATTACTATGCGATCATTGATACACTTGTAACAACCTTGGTCGAAGGAGAGGATTATATCTTTAAAGAGGA
GAAAGAGGAGGTTTGGCTCACTACTAAGGGGGCCAAGTCTGCTGAGAATTTCCTAGGGATTGATAATTTATACAA
GGAAGAGCATGCGTCTTTTGCTCGTCATTTGGTTTATGCGATTCGAGCTCATAAGCTCTTTACTAAAGATAAGGAC
TATATCATTCGTGGAAATGAGATGGTACTGGTTGATAAGGGAACAGGGCGTCTAATGGAAATGACTAAACTTCAA
GGAGGTCTCCATCAGGCTATTGAAGCCAAGGAACATGTCAAATTATCTCCTGAGACGCGGGCTATGGCCTCGATC
ACCTATCAGAGTCTTTTTAAGATGTTTAATAAGATATCTGGTATGACAGGGACAGGTAAGGTCGCGGAAAAAGAG
TTTATTGAAACTTACAATATGTCTGTAGTACGCATTCCAACCAATCGTCCGAGACAACGGATTGACTATCCAGATA
ATCTATATATCACTTTACCTGAAAAAGTGTATGCATCCTTGGAGTACATCAAGCAATACCATGCTAAGGGAAATCC
TTTACTCGTTTTTGTAGGCTCAGTTGAAATGTCTCAACTCTATTCGTCTCTCTTGTTTCGTGAAGGGATTGCCCATA
ATGTCCTAAATGCTAATAATGCGGCGCGTGAGGCTCAGATTATCTCCGAGTCAGGTCAGATGGGGGCTGTGACAG
TGGCTACCTCTATGGCAGGACGTGGTACGGATATCAAGCTTGGTAAAGGAGTCGCAGAGCTTGGGGGCTTGATTG
TTATTGGGACTGAGCGGATGGAAAGTCAGCGGATCGACCTACAAATTCGTGGCCGTTCTGGTCGTCAGGGAGATC
CTGGTATGAGTAAATTTTTTGTATCCTTAGAGGATGATGTTATCAAGAAATTTGGTCCATCTTGGGTGCATAAAAA
GTACAAAGACTATCAGGTTCAAGATATGACTCAACCGGAAGTATTGAAAGGTCGTAAATACCGGAAACTAGTCGA
AAAGGCTCAGCATGCCAGTGATAGTGCTGGACGTTCAGCACGTCGTCAGACTCTGGAGTATGCTGAAAGTATGAA
TATACAACGGGATATAGTCTATAAAGAGAGAAATCGTCTAATAGATGGTTCTCGTGACTTAGAGGATGTTGTTGTG
GATATCATTGAGAGATATACAGAAGAGGTAGCGGCTGATCACTATGCTAGTCGTGAATTATTGTTTCACTTTATTG
TGACCAATATTAGTTTTCATGTTAAAGAGGTTCCAGATTATATAGATGTAACTGACAAAACTGCAGTTCGTAGCTT
TATGAAGCAGGTGATTGATAAAGAACTTTCTGAAAAGAAAGAATTACTTAATCAACATGACTTATATGAACAGTT

TTTACGACTTTCACTGCTTAAAGCCATTGATGACAACTGGGTAGAGCAGGTAGACTATCTACAACAGCTATCCATG
GCTATCGGTGGTCAATCTGCTAGTCAGAAAAATCCAATCGTAGAGTACTATCAAGAAGCCTACGCGGGCTTTGAA
GCTATGAAAGAACAGATTCATGCGGATATGGTGCGTAATCTCCTGATGGGGCTGGTTGAGGTCACTCCAAAAGGT
GAAATCGTGACTCATTTTCCATAA

MSSLSDQELVAKTVEFRQRLSEGESLDDILVEAFAVVREADKRILGMFPYDVQVMGAIVMHYGNVAEMNTGEGKTLT
ATMPVYLNAFSGEGVMVVTPNEYLSKRDAEEMGQVYRFLGLTIGVPFTEDPKKEMKAEEKKLIYASDIIYTTNSNLGF
DYLNDNLASNEEGKFLRPFNYVIIDEIDDILLDSAQTPLIIAGSPRVQSNYYAIIDTLVTTLVEGEDYIFKEEKEEVWLTTK
GAKSAENFLGIDNLYKEEHASFARHLVYAIRAHKLFTKDKDYIIRGNEMVLVDKGTGRLMEMTKLQGGLHQAIEAKEH
VKLSPETRAMASITYQSLFKMFNKISGMTGTGKVAEKEFIETYNMSVVRIPTNRPRQRIDYPDNLYITLPEKVYASLEYIK
QYHAKGNPLLVFVGSVEMSQLYSSLLFREGIAHNVLNANNAAREAQIISESGQMGAVTVATSMAGRGTDIKLGKGVAE
LGGLIVIGTERMESQRIDLQIRGRSGRQGDPGMSKFFVSLEDDVIKKFGPSWVHKKYKDYQVQDMTQPEVLKGRKYRK
LVEKAQHASDSAGRSARRQTLEYAESMNIQRDIVYKERNRLIDGSRDLEDVVVDIIERYTEEVAADHYASRELLFHFIVT
NISFHVKEVPDYIDVTDKTAVRSFMKQVIDKELSEKKELLNQHDLYEQFLRLSLLKAIDDNWVEQVDYLQQLSMAIGG
QSASQKNPIVEYYQEAYAGFEAMKEQIHADMVRNLLMGLVEVTPKGEIVTHFPZ

ID104 879bp

ATGAAACAAGAATGGTTTGAAAGTAATGATTTTGTAAAAACAACAAGCAAGAACAAGCCTGAAGAGCAAGCTCA
AGAGGTTGCAGACAAGGCTGAAGAAAGGATACCCGATCTCGATACACCAATTGAAAAAAATACTCAGTTAGAGG
AGGAAGTCTCTCAAGCTGAAGTCGAATTGGAAAGCCAGCAAGAAGAGAAAATTGAAGCTCCTGAAGACAGTGAA
GCGAGAACAGAAATAGAAGAAAAGAAGGCATCTAATTCTACTGAAGAAGAGCCAGACCTTTCTAAAGAAACAGA
AAAAGTCACTATAGCTGAAGAGAGCCAAGAAGCTCTTCCTCAGCAAAAAGCAACCACGAAAGAGCCACTTCTTAT
CAGTAAATCTTTAGAAAGTCCTTATATCCCCGACCAAGCTCCAAAATCTAGGGATAAATGGAAAGAGCAAGTGCT
TGATTTTTGGTCTTGGCTAGTGGAAGCGATCAAATCTCCTACAAGTAAGTTGGAAACAAGTATCACACACAGTTAC
ACAGCCTTTCTCTTGCTCATTCTGTTTCTGCATCTTCCTTTTTCTTTAGTATCTATCACATCAAACATGCTTACTAT
GGACATATAGCAAGCATTAACAGTCGCTTCCCTGAGCAGCTAGCTCCTTTAACTCTTTTTTCTATCATCTCTATCCT
AGTAGCGACAACACTCTTCTTCTTTTCATTCCTCTTGGGTAGTTTCGTTGTGAGACGATTTATCCACCAGGAAAAG
GACTGGACGCTAGACAAGGTTCTCCAACAATATAGTCAACTCTTGGCAATTCCAATCTCCTCACTGCTATTGCTAG
TTTCTTTGCTTTCTTTGATAGCCTACGATTTACAGCCCTCTTGTGTGTGA

MKQEWFESNDFVKTTSKNKPEEQAQEVADKAEERIPDLDTPIEKNTQLEEEVSQAEVELESQQEEKIEAPEDSEARTEIE
EKKASNSTEEEPDLSKETEKVTIAEESQEALPQQKATTKEPLLISKSLESPYIPDQAPKSRDKWKEQVLDFWSWLVEAIKS
PTSKLETSITHSYTAFLLLILFSASSFFFSIYHIKHAYYGHIASINSRFPEQLAPLTLFSIISILVATTLFFFSFLLGSFVVRRFIH
QEKDWTLDKVLQQYSQLLAIPISSLLLLVSLLSLIAYDLQPSCVZ

ID106 327bp

ATGTACTTTCCAACATCCTCTGCCTTGATTGAATTTCTCATCTTGGCTGTACTGGAGCAGGGTGATTCTTATGGTTA
TGAGATTAGCCAAACCATTAAGCTGATCGCTAATATCAAAGAATCCACACTCTATCCCATTCTCAAAAAATTGGA
AGGCAATAGCTTTCTGACAACCTATTCTAGAGAGTTCCAAGGTCGCATGCGCAAATACTACTCCTTGACAAACGG
TGGTATAGAGCAGCTCTTGACCCTAAAAGATGAATGGGCACTCTATACAGACACCATCAATGGCATCATAGAAGG
GAGTATCCGCCATGACAAGAACTGA

MYFPTSSALIEFLILAVLEQGDSYGYEISQTIKLIANIKESTLYPILKKLEGNSFLTTYSREFQGRMRKYYSLTNGGIEQLLT
LKDEWALYTDTINGIIEGSIRHDKNZ

ID108 954bp

ATGGATTTTGAAAAAATTGAACAAGCTTATATCTATTTACTAGAGAATGTCCAAGTCATCCAAAGTGATTTGGCGA
CCAACTTTTATGACGCCTTGGTGGAGCAAAATAGCATCTATCTGGATGGTGAAACTGAGCTAAACCAGGTCAAAG
ACAACAATCAGGCCCTTAAGCGTTTAGCACTACGCAAAGAAGAATGGCTCAAGACCTACCAGTTTCTCTTGATGA
AGGCTGGGCAAACAGAACCCTTGCAGGCCAATCACCAGTTTACACCGGATGCTATTGCTTTGCTTTTGGTGTTTAT
TGTGGAAGAGTTGTTTAAAGAGGAGGAAATTACTATCCTCGAAATGGGTTCTGGGATGGGAATTCTAGGCGCTAT
TTTCTTGACCTCGCTTACTAAAAAGGTGGATTACTTGGGAATGGAAGTGGATGATTTGCTGATTGATCTGGCAGCT
AGCATGGCAGATGTAATTGGTTTGCAGGCTGGCTTTGTCCAAGGAGATGCCGTTCGCCCACAAATGCTCAAAGAA
AGCGATGTGGTCATCAGTGACTTGCCTGTCGGCTATTATCCTGATGATGCCGTTGCGTCGCGCCATCAAGTTGCTT
CTAGCCAAGAACATACTTACGCCCATCACTTGCTCATGGAACAAGGGCTTAAGTACCTCAAGTCAGACGGATACG
CTATTTTTCTAGCTCCGAGTGATTTGTTGACCAGTCCTCAAAGTGATTTGTTAAAAGAATGGCTGAAAGAAGAGGC
GAGTCTGGTTGCTATGATTAGTCTGCCTGAAAATCTCTTTGCTAATGCCAAACAATCTAAGACTATTTTTATCTTAC
AGAAGAAAAATGAAATAGCAGTAGAGCCTTTTGTTTATCCACTTGCTAGCTTGCAAGATGCAAGTGTTTTAATGAA
ATTTAAAGAAAATTTTCAAAAATGGACTCAAGGTACTGAAATATAA

MDFEKIEQAYIYLLENVQVIQSDLATNFYDALVEQNSIYLDGETELNQVKDNNQALKRLALRKEEWLKTYQFLLMKA
GQTEPLQANHQFTPDAIALLLVFIVEELFKEEEITILEMGSGMGILGAIFLTSLTKKVDYLGMEVDDLLIDLAASMADVI

GLQAGFVQGDAVRPQMLKESDVVISDLPVGYYPDDAVASRHQVASSQEHTYAHHLLMEQGLKYLKSDGYAIFLAPSD
LLTSPQSDLLKEWLKEEASLVAMISLPENLFANAKQSKTIFILQKKNEIAVEPFVYPLASLQDASVLMKFKENFQKWTQG
TEIZ

ID110 1902bp

ATGATTATTTTACAAGCTAATAAAATTGAACGTTCTTTTGCAGGAGAGGTTCTTTTCGATAATATCAACCTGCAGG
TTGATGAACGAGATCGGATTGCTCTTGTTGGGAAAAATGGTGCAGGTAAGTCTACTCTTTTGAAGATTTTAGTTGG
AGAAGAGGAGCCAACTAGCGGAGAAATCAATAAGAAAAAAGATATTTCTCTGTCTTACCTAGCCCAAGATAGCCG
TTTTGAGTCTGAAAATACCATCTACGATGAAATGCTTCATGTCTTTAATGATTTGCGTCGGACGGAGAGACAACTG
CGTCAGATGGAGCTGGAGATGGGTGAAAAGTCTGGTGAGGATTTGGATAAACTGATGTCAGATTATGACCGCTTA
TCTGAGAATTTTCGCCAAGCAGGTGGCTTTACCTATGAAGCTGATATTCGAGCGATTTTGAATGGATTCAAGTTTG
ACGAGTCTATGTGGCAGATGAAAATTGCTGAGCTTTCTGGTGGTCAAAATACTCGTTTGGCACTTGCCAAAATGCT
CCTTGAAAAGCCCAATCTCTTGGTCTTGGACGAGCCAACTAACCACTTGGATATTGAAACCATCGCCTGGCTAGA
GAATTACTTGGTAAACTATAGCGGTGCCCTCATTATCGTCAGCCACGACCGTTATTTCTTGGACAAGGTTGCGACA
ATTACGCTAGATTTGACCAAGCATTCCTTGGATCGCTATGTGGGGAATTACTCTCGTTTTGTCGAATTGAAGGAGC
AAAAGCTAGTTACTGAGGCAAAAAACTATGAAAAGCAACAGAAGGAAATCGCTGCTCTGGAAGACTTTGTCAATC
GCAATCTAGTTCGTGCTTCAACGACTAAACGTGCTCAATCTCGCCGTAAACAACTAGAAAAAATGGAGCGTTTGG
ACAAGCCTGAAGCTGGCAAGAAAGCAGCCAACATGACCTTCCAGTCTGAAAAAACGTCGGGCAATGTTGTTTTGA
CTGTTGAAAATGCAGCTGTTGGCTATGACGGGGAAGTCTTGTCACAACCTATCAACCTAGATCTTCGTAAGATGAA
TGCTGTCGCTATCGTTGGTCCAAATGGTATCGGCAAGTCAACCTTTATCAAGTCTATTGTGGACCAGATTCCTTTT
ATCAAGGGAGAAAAGCGCTTTGGCGCTAATGTTGAGGTTGGTTACTATGACCAAACCCAAAGCAAGCTGACACCA
AGTAATACGGTGCTGGATGAACTCTGGAATGATTTCAAACTGACACCAGAAGTTGAAATCCGCAACCGTCTTGGA
GCCTTCCTTTTCTCAGGAGATGATGTTAAAAAATCAGTCGGCATGCTATCTGGTGGCGAAAAAGCTCGTTTGCTTT
TAGCTAAATTGTCTATGGAAAACAATAACTTTTTGATTCTGGATGAGCCGACCAACCACTTGGATATTGATAGTAA
GGAAGTGCTAGAAAATGCCTTGATTGACTTTGATGGAACCTTGCTGTTTGTCAGTCATGATCGTTACTTTATCAAT
CGTGTGGCAACTCATGTTTTGGAATTGTCTGAGAATGGTTCAACTCTCTACCTTGGAGATTACGACTACTATGTTG
AGAAGAAAGCAACAGCAGAAATGAGTCAGACTGAGGAAGCTTCAACTAGCAATCAAGCAAAGGAAGCAAGTCCA
GTCAATGACTATCAGGCCCAGAAAGAAAGTCAAAAAGAAGTTCGCAAACTCATGCGACAAATCGAAAGTCTAGA
AGCTGAAATTGAAGAGCTAGAAAGTCAAAGCCAAGCCATTTCTGAACAAATGTTGGAAACAAACGATGCCGACA
AACTCATGGAATTACAGGCTGAGCTGGACAAAATCAGCCATCGTCAGGAAGAAGCTATGCTTGAGTGGGAAGAAT
TATCAGAGCAGGTGTAA

MIILQANKIERSFAGEVLFDNINLQVDERDRIALVGKNGAGKSTLLKILVGEEEPTSGEINKKKDISLSYLAQDSRFESENT
IYDEMLHVFNDLRRTERQLRQMELEMGEKSGEDLDKLMSDYDRLSENFRQAGGFTYEADIRAILNGFKFDESMWQMK
IAELSGGQNTRLALAKMLLEKPNLLVLDEPTNHLDIETIAWLENYLVNYSGALIIVSHDRYFLDKVATITLDLTKHSLDR
YVGNYSRFVELKEQKLVTEAKNYEKQQKEIAALEDFVNRNLVRASTTKRAQSRRKQLEKMERLDKPEAGKKAANMTF
QSEKTSGNVVLTVENAAVGYDGEVLSQPINLDLRKMNAVAIVGPNGIGKSTFIKSIVDQIPFIKGEKRFGANVEVGYYDQ
TQSKLTPSNTVLDELWNDFKLTPEVEIRNRLGAFLFSGDDVKKSVGMLSGGEKARLLLAKLSMENNNFLILDEPTNHL
DIDSKEVLENALIDFDGTLLFVSHDRYFINRVATHVLELSENGSTLYLGDYDYYVEKKATAEMSQTEEASTSNQAKEAS
PVNDYQAQKESQKEVRKLMRQIESLEAEIEELESQSQAISEQMLETNDADKLMELQAELDKISHRQEEAMLEWEELSEQ
VZ

ID111 1179bp

ATGAATCGCTATGCAGTGCAGTTGATTAGCCGTGGGGCTATCAATAAAATGGGAAATATGCTCTATGATTATGGA
AATAGTGTCTGGTTGGCTTCTATGGGGACTATAGGACAGACAGTTTTAGGAATGTATCAGATTTCTGAGCTCGTCA
CATCTATTCTCGTCAATCCCTTTGGCGGAGTTATTTCAGACCGTTTTTCTCGTCGTAAGATTTTAATGACGGCAGAT
CTTGTTTGTGGGATTCTTTGTCTGGCTATTTCTTTCATAAGGAATGATAGCTGGATGATTGGCGCTTTGATTGTTGC
TAACATTGTGCAGGCTATTGCTTTTGCCTTTTCTCGCACAGCCAATAAAGCTATCATAACTGAAGTGGTGGAGAAA
GATGAGATTGTGATCTATAATTCTCGCTTAGAGCTGGTTTTGCAGGTTGTAGGTGTTAGCTCTCCTGTTCTTTCCTT
CCTTGTTTTACAGTTTGCAAGTCTCCATATGACGCTACTGCTAGACTCGCTGACTTTTTTCATTGCTTTTGTTCTAG
TGGCTTTCCTTCCAAAAGAGGAAGCAAAAGTTCAAGAGAAAAAGGCTTTTACTGGGAGAGATATTTTTGTAGATA
TCAAGGATGGGTTACACTATATCTGGCATCAGCAAGAAATTTTCTTCCTTTTGCTGGTAGCTTCCAGCGTTAATTT
CTTTTTTGCAGCTTTTGAATTTCTACTTCCCTTTTCGAATCAGCTTTACGGGTCAGAAGGAGCCTATGCAAGTATTT
TAACTATGGGGGCTATTGGTTCCATCATTGGGGCTCTTCTAGCTAGTAAAATTAAAGCTAATATTTATAATCTTTT
GATTTTACTGGCTTTGACAGGTGTCGGAGTTTTTATGATGGGATTACCACTTCCAACTTTTCTTTCCTTTTCTGGAA
ATTTAGTTTGTGAATTGTTTATGACGATTTTTAATATTCACTTTTTACTCAAGTACAAACCAAGGTTGAGAGCGAA
TTTCTTGGAAGAGTACTGAGTACAATTTTTACCTTAGCTATTCTATTTATGCCTATTGCAAAAGGATTTATGACAGT
CTTGCCAAGTGTCCATCTTTATTCTTTCTTGATTATTGGACTTGGAGTTGTAGCCTTATATTTCTTAGCTCTCGGAT
ATGTTCGAACTCATTTTGAAAAATTGATATAA

MNRYAVQLISRGAINKMGNMLYDYGNSVWLASMGTIGQTVLGMYQISELVTSILVNPFGGVISDRFSRRKILMTADLV
CGILCLAISFIRNDSWMIGALIVANIVQAIAFAFSRTANKAIITEVVEKDEIVIYNSRLELVLQVVGVSSPVLSFLVLQFASL
HMTLLLDSLTFFIAFVLVAFLPKEEAKVQEKKAFTGRDIFVDIKDGLHYIWHQQEIFFLLLVASSVNFFFAAFEFLLPFSN

QLYGSEGAYASILTMGAIGSIIGALLASKIKANIYNLLILLALTGVGVFMMGLPLPTFLSFSGNLVCELFMTIFNIHFFTQV
QTKVESEFLGRVLSTIFTLAILFMPIAKGFMTVLPSVHLYSFLIIGLGVVALYFLALGYVRTHFEKLIZ

ID113 2466bp

ATGCAAAATCAATTAAATGAATTAAAACGAAAAATGCTGGAATTTTTCCAGCAAAAACAAAAAAATAAAAAATCA
GCTAGACCTGGCAAGAAAGGTTCAAGTACCAAAAAATCTAAAACCTTAGATAAGTCAGCCATTTTCCCAGCTATT
TTACTGAGTATAAAAGCCTTATTTAACTTACTCTTTGTACTCGGTTTTCTAGGAGGAATGTTGGGAGCTGGGATTG
CTTTGGGATACGGAGTGGCCTTATTTGACAAGGTTCGGGTGCCTCAGACAGAAGAATTGGTGAATCAGGTCAAGG
ACATCTCTTCTATTTCAGAGATTACCTATTCGGACGGGACGGTGATTGCTTCCATAGAGAGTGATTTGTTGCGCAC
TTCTATCTCATCTGAGCAAATTTCGGAAAATCTGAAGAAGGCTATCATTGCGACAGAAGATGAACACTTTAAAGA
ACATAAGGGTGTAGTACCCAAGGCGGTGATTCGTGCGACCTTGGGGAAATTTGTAGGTTTGGGTTCCTCTAGTGGG
GGTTCAACCTTGACCCAGCAACTAATTAAACAGCAGGTGGTTGGGGATGCGCCGACCTTGGCTCGTAAGGCGGCA
GAGATTGTGGATGCTCTTGCCTTGGAACGCGCCATGAATAAAGATGAGATTTTAACGACCTATCTCAATGTGGCTC
CCTTTGGCCGAAATAATAAGGGACAGAATATTGCAGGGGCTCGGCAAGCAGCTGAGGGAATTTTCGGTGTAGATG
CCAGTCAGTTGACTGTTCCTCAAGCAGCATTTTTAGCAGGACTTCCACAGAGTCCCATTACTTACTCTCCTTATGA
AAATACTGGGGAGTTGAAGAGTGATGAAGACCTAGAAATTGGCTTAAGACGGGCTAAGGCAGTTCTTTACAGTAT
GTATCGTACAGGTGCATTAAGCAAAGACGAGTATTCTCAGTACAAGGATTATGACCTTAAACAGGACTTTTTACC
ATCGGGCACGGTTACAGGAATTTCACGAGACTATTTATACTTTACAACTTTGGCAGAAGCTCAAGAACGTATGTAT
GACTATCTAGCTCAGAGAGACAATGTCTCCGCTAAGGAGTTGAAAAATGAGGCAACTCAGAAGTTTTATCGAGAT
TTGGCAGCCAAGGAAATTGAAAATGGTGGTTATAAGATTACTACTACCATAGATCAGAAAATTCATTCTGCCATG
CAAAGTGCGGTTGCTGATTATGGCTATCTTTTAGACGATGGAACAGGTCGTGTAGAAGTAGGGAATGTCTTGATG
GATAACCAAACAGGTGCTATTCTAGGCTTTGTAGGTGGTCGTAATTATCAAGAAAATCAAAATAATCATGCCTTTG
ATACCAAACGTTCGCCAGCTTCTACTACCAAGCCCTTGCTGGCCTACGGTATTGCTATTGACCAGGGCTTGATGGG
AAGTGAAACGATTCTATCTAACTATCCAACAAACTTTGCTAATGGCAATCCGATTATGTATGCTAATAGCAAGGG
AACAGGAATGATGACCTTGGGAGAAGCTCTGAACTATTCATGGAATATCCCTGCTTACTGGACCTATCGTATGCTC
CGTGAAAAGGGTGTTGATGTCAAGGGTTATATGGAAAAGATGGGTTACGAGATTCCTGAGTACGGTATTGAGAGC
TTGCCAATGGGTGGTGGTATTGAAGTCACAGTTGCCCAGCATACCAATGGCTATCAGACCTTAGCTAATAATGGA
GTTTATCATCAGAAGCATGTGATTTCAAAGATTGAAGCAGCAGATGGTAGAGTGGTGTATGAGTATCAGGATAAA
CCGGTTCAAGTCTATTCAAAAGCTACTGCGACGATTATGCAGGGATTGCTACGAGAAGTTCTATCCTCTCGTGTGA
CAACAACCTTCAAGTCTAACCTGACTTCTTTAAATCCTACTCTGGCTAATGCAGATTGGATTGGGAAGACTGGTAC
AACCAACCAAGACGAAAATATGTGGCTCATGCTTTCGACACCTAGATTAACCCTAGGTGGCTGGATTGGGCATGA
TGATAATCATTCATTGTCACGTAGAGCAGGTTATTCTAATAACTCTAATTACATGGCTCATCTGGTAAATGCGATT
CAGCAAGCTTCCCCAAGCATTTGGGGGAACGAGCGCTTTGCTTTAGATCCTAGTGTAGTGAAATCGGAAGTCTTG
AAATCAACAGGTCAAAAACCAGAGAAGGTTTCTGTTGAAGGAAAAGAAGTAGAGGTCACAGGTTCGACTGTTACC
AGCTATTGGGCTAATAAGTCAGGAGCGCCAGCGACAAGTTATCGCTTTGCTATTGGCGGAAGTGATGCGGATTAT
CAGAATGCTTGGTCTAGTATTGTGGGGAGTCTACCAACTCCATCCAGCTCCAGCAGTTCAAGTAGTAGTTCTAGCG
ATAGCAGTAACTCAAGTACTACACGACCTTCTTCTTCAAGGGCGAGACGATAA

MQNQLNELKRKMLEFFQQKQNKKSARPGKKGSSTKKSKTLDKSAIFPAILLSIKALFNLLFVLGFLGGMLGAGIALGY
GVALFDKVRVPQTEELVNQVKDISSISEITYSDGTVIASIESDLLRTSISSEQISENLKKAIIATEDEHFKEHKGVVPKAVIR
ATLGKFVGLGSSSGGSTLTQQLIKQQVVGDAPTLARKAAEIVDALALERAMNKDEILTTYLNVAPFGRNNKGQNIAGA
RQAAEGIFGVDASQLTVPQAAFLAGLPQSPITYSPYENTGELKSDEDLEIGLRRAKAVLYSMYRTGALSKDEYSQYKDY
DLKQDFLPSGTVTGISRDYLYFTTLAEAQERMYDYLAQRDNVSAKELKNEATQKFYRDLAAKEIENGGYKITTTIDQKI
HSAMQSAVADYGYLLDDGTGRVEVGNVLMDNQTGAILGFVGGRNYQENQNNHAFDTKRSPASTTKPLLAYGIAIDQG
LMGSETILSNYPTNFANGNPIMYANSKGTGMMTLGEALNYSWNIPAYWTYRMLREKGVDVKGYMEKMGYEIPEYGIE
SLPMGGGIEVTVAQHTNGYQTLANNGVYHQKHVISKIEAADGRVVYEYQDKPVQVYSKATATIMQGLLREVLSSRVTT
TFKSNLTSLNPTLANADWIGKTGTTNQDENMWLMLSTPRLTLGGWIGHDDNHSLSRRAGYSNNSNYMAHLVNAIQQA
SPSIWGNERFALDPSVVKSEVLKSTGQKPEKVSVEGKEVEVTGSTVTSYWANKSGAPATSYRFAIGGSDADYQNAWSSI
VGSLPTPSSSSSSSSSSDSSNSSTTRPSSSRARRZ

ID114 1974bp

ATGAAAAAATTTTATGTAAGTCCAATTTTTCCTATTCTAGTAGGATTGATTGCGTTTGGAGTCTTATCCACTTTCAT
TATTTTTGTTAATAATAATCTGTTGACGGTTTTAATTTTGTTTCTTTTTGTAGGAGGCTATGTTTTTTTATTTAAGAA
ACTGAGAGTGCATTATACAAGGAGTGATGTAGAACAGATACAGTATGTAAACCACCAAGCGGAAGAAAGTTTGAC
AGCTCTATTGGAACAGATGCCTGTAGGTGTTATGAAATTGAATTTATCTTCTGGAGAGGTTGAGTGGTTTAATCCC
TATGCTGAATTGATTTTGACCAAGGAAGATGGTGATTTTGATTTAGAAGCTGTTCAAACGATTATCAAGGCTTCAG
TAGGAAATCCGTCTACTTATGCCAAGCTTGGTGAGAAGCGTTATGCTGTTCATATGGATGCTTCTTCCGGTGTTTT
GTATTTTGTAGATGTATCCAGGGAACAAGCCATAACAGATGAATTGGTAACAAGTAGACCAGTGATTGGGATTGT
CTCTGTGGATAATTATGATGATTTGGAGGATGAAACTTCTGAGTCAGATATTAGTCAAATCAATAGTTTTGTAGCT
AATTTTATATCAGAGTTTTCAGAAAAACACATGATGTTTTCTCGTCGGGTAAGTATGGATCGATTTTATCTATTTAC
TGACTACACGGTGCTTGAGGGCTTGATGAATGATAAATTTTCTGTTATTGATGCTTTCAGAGAAGAGTCGAAACAG
AGACAGTTGCCCTTGACCTTAAGTATGGGATTTTCTTATGGCGATGGAAATCATGATGAGATAGGGAAAGTTGCTT
TGCTCAATTTGAACTTGGCTGAAGTACGTGGTGGCGACCAGGTGGTTGTTAAGGAAAACGACGAAACGAAAAATC

CAGTTTATTTTGGTGGTGGGTCTGCTGCTTCAATCAAGCGTACACGGACTCGTACGCGCGCTATGATGACAGCTAT
TTCAGATAAGATTCGGAGTGTAGATCAGGTTTTTGTAGTCGGTCACAAAAATTTAGACATGGATGCTTTGGGCTCT
GCTGTAGGTATGCAGTTGTTCGCCAGCAATGTGATTGAAAATAGCTATGCTCTTTATGATGAAGAACAAATGTCTC
CAGATATTGAACGAGCTGTTTCATTCATAGAAAAAGAAGGAGTTACGAAGTTGTTGTCTGTTAAGGATGCAATGG
GGATGGTGACCAATCGTTCTTTGTTGATTCTTGTAGACCATTCAAAGACAGCCTTAACATTATCAAAAGAATTTTA
TGATTTATTTACCCAAACCATTGTTATTGACCACCATAGAAGGGATCAGGATTTTCCAGATAATGCGGTTATTACT
TATATCGAAAGTGGTGCAAGTAGTGCCAGTGAGTTGGTAACGGAATTGATTCAGTTCCAGAATTCTAAGAAAAAT
CGTTTGAGTCGTATGCAAGCAAGTGTCTTGATGGCTGGTATGATGTTGGATACTAAAAATTTCACCTCGCGAGTAA
CTAGTCGGACATTTGATGTTGCTAGCTATCTCAGAACGCGCGGAAGTGATAGTATTGCTATCCAGGAAATCGCTGC
GACAGATTTTGAAGAATATCGTGAGGTCAATGAACTTATTTTACAGGGGCGTAAATTAGGTTCAGATGTACTAATA
GCAGAGGCTAAGGACATGAAATGCTATGATACAGTTGTTATTAGTAAGGCAGCAGATGCCATGTTAGCCATGTCA
GGTATTGAAGCGAGTTTTGTTCTTGCGAAGAATACACAAGGATTTATCTCTATCTCAGCTCGAAGTCGTAGTAAAC
TGAATGTACAACGGATTATGGAAGAGTTAGGCGGTGGAGGCCACTTTAATTTGGCAGCAGCTCAAATTAAAGATG
TAACCTTGTCAGAAGCAGGTGAAAAACTGACAGAAATTGTATTAAATGAAATGAAGGAAAAGGAGAAAGAAGAA
TGA

MKKFYVSPIFPILVGLIAFGVLSTFIIFVNNNLLTVLILFLFVGGYVFLFKKLRVHYTRSDVEQIQYVNHQAEESLTALLE
QMFVGVMKLNLSSGEVEWFNPYAELILTKEDGDFDLEAVQTIIKASVGNPSTYAKLGEKRYAVHMDASSGVLYFVDVS
REQAITDELVTSRPVIGIVSVDNYDDLEDETSESDISQINSFVANFISEFSEKHMMFSRRVSMDRFYLFTDYTVLEGLMN
DKFSVIDAFREESKQRQLPLTLSMGFSYGDGNHDEIGKVALLNLNLAEVRGGDQVVVKENDETKNPVYFGGGSAASIK
RTRTRTRAMMTAISDKIRSVDQVFVVGHKNLDMDALGSAVGMQLFASNVIENSYALYDEEQMSPDIERAVSFIEKEGV
TKLLSVKDAMGMVTNRSLLILVDHSKTALTLSKEFYDLFTQTIVIDHHRRDQDFPDNAVITYIESGASSASELVTELIQFQ
NSKKNRLSRMQASVLMAGMMLDTKNFTSRVTSRTFDVASYLRTRGSDSIAIQEIAATDFEEYREVNELILQGRKLGSDV
LIAEAKDMKCYDTVVISKAADAMLAMSGIEASFVLAKNTQGFISISARSRSKLNVQRIMEELGGGGHFNLAAAQIKDVT
LSEAGEKLTEIVLNEMKEKEKEEZ

ID115 663bp

ATGAAGTGCTTGTTATGTGGGCAGACTATGAAGACTGTTTTAACTTTTAGTAGTCTCTTACTTCTGAGGAATGATG
ACTCTTGTCTTTGTTCAGACTGTGATTCTACTTTTGAAAGAATTGGGGAAGAGAACTGTCCAAATTGTATGAAAAC
AGAGTTGTCAACAAAGTGTCAAGATTGTCAACTTTGGTGTAAAGAGGGAGTTGAAGTCAGTCATAGAGCGATTTT
TACTTACAATCAAGCTATGAAGGATTTTTTCAGTCGGTATAAGTTTGATGGAGACTTCCTGTTAAGAAAAGTTTTC
GCTTCATTTTTAAGTGAGGAGTTGAAAAAGTACAAAGAGTATCAATTTGTTGTAATTCCCCTAAGTCCTGATAGAT
ATGCTAATAGAGGATTTAATCAGGTTGAGGGCTTGGTAGAGGCAGCAGGCTTTGAGTATCTGGATTTATTAGAGA
AAAGAGAAGAGAGAGCCAGTTCTTCTAAAAATCGTTCAGAGCGCTTGGGGACAGAACTTCCTTTCTTTATTAAAA
GTGGAGTCACTATTCCTAAAAAAATCCTACTTATAGATGATATCTATACTACAGGAGCAACTATAAATCGTGTTAA
GAAACTGTTGGAAGAAGCTGGTGCTAAGGATGTAAAAACATTTTCCCTTGTAAGATGA

MKCLLCGQTMKTVLTFSSLLLLRNDDSCLCSDCDSTFERIGEENCPNCMKTELSTKCQDCQLWCKEGVEVSHRAIFTY
NQAMKDFFSRYKFDGDFLLRKVFASFLSEELKKYKEYQFVVIPLSPDRYANRGFNQVEGLVEAAGFEYLDLLEKREER
ASSSKNRSERLGTELPFFIKSGVTIPKKILLIDDIYTTGATINRVKKLLEEAGAKDVKTFSLVRZ

ID116 1299bp

ATGAAAGTAAATTTAGATTATCTCGGTCGTTTATTTACTGAGAATGAATTAACAGAAGAAGAACGTCAGTTGGCG
GAGAAACTTCCAGCAATGAGAAAGGAGAAGGGGAAACTTTTCTGTCAACGCTGTAATAGTACTATTCTAGAAGAA
TGGTATTTGCCCATCGGTGCTTACTATTGTCGAGAGTGCTTGCTGATGAAGCGAGTCAGAAGTGATCAAACTTTAT
ACTATTTTCCGCAGGAGGATTTTCCAAAGCAAGATGTTCTCAAATGGCGCGGCCAATTAACTCCTTTTCAAGAGAA
GGTGTCAGAGGGATTGCTTCAAGTAGTAGACAAGCAAAAGCCAACCTTAGTTCATGCGGTAACAGGAGCTGGAAA
GACAGAAATGATTTATCAAGTAGTGGCTAAAGTGATCAATGCGGGTGGTGCAGTGTGTTTGGCTAGTCCTCGCAT
AGATGTTTGTTTGGAGCTGTACAAGCGCCTGCAACAGGATTTTTCTTGCGGGATAGCTTTGCTACATGGAGAATCG
GAACCTTATTTTCGAACACCACTAGTTGTTGCAACAACCCATCAGTTATTGAAGTTTTATCAAGCTTTTGATTTGCT
GATAGTGGATGAAGTAGATGCTTTTCCTTATGTTGATAATCCCATGCTTTACCACGCTGTCAAGAATAGTGTAAAG
GAGAATGGATTGAGAATCTTTTTAACAGCGACTTCGACCAATGAGTTAGATAAAAAGGTCCGTTTAGGAGAACTA
AAAAGACTGAATTTACCGAGACGGTTTCATGGAAATCCGTTGATTATTCCAAAACCAATTTGGTTATCGGATTTTA
ATCGCTACTTAGACAAGAATCGTTTGTCACCAAAGTTAAAGTCCTATATTGAGAAGCAGAGAAAGACAGCTTATC
CGTTACTCATTTTTGCTTCAGAAATTAAGAAAGGGGAGCAGTTAGCAGAAATCTTACAGGAGCAATTTCCAAATG
AGAAAATTGGCTTTGTATCTTCTGTAACAGAGGATCGATTAGAGCAAGTACAAGCTTTTCGAGATGGAGAACTGA
CAATACTTATCAGTACGACAATCTTGGAGCGCGGAGTTACCTTCCCTTGTGTGGATGTTTTCGTAGTAGAGGCCAA
TCATCGTTTGTTTACCAAGTCTAGTTTGATTCAGATTGGTGGACGAGTTGGACGAAGCATGGATAGACCGACAGGA
GATTTGCTTTTCTTCCATGATGGGTTAAATGCTTCAATCAAGAAGGCGATTAAGGAAATTCAGATGATGAATAAGG
AGGCTGGTCTATGA

MKVNLDYLGRLFTENELTEEERQLAEKLPAMRKEKGKLFCQRCNSTILEEWYLPIGAYYCRECLLMKRVRSDQTLYYF
PQEDFPKQDVLKWRGQLTPFQEKVSEGLLQVVDKQKPTLVHAVTGAGKTEMIYQVVAKVINAGGAVCLASPRIDVCL

ELYKRLQQDFSCGIALLHGESEPYFRTPLVVATTHQLLKFYQAFDLLIVDEVDAFPYVDNPMLYHAVKNSVKENGLRIF
LTATSTNELDKKVRLGELKRLNLPRRFHGNPLIIPKPIWLSDFNRYLDKNRLSPKLKSYIEKQRKTAYPLLIFASEIKKGE
QLAEILQEQFPNEKIGFVSSVTEDRLEQVQAFRDGELTILISTTILERGVTFPCVDVFVVEANHRLFTKSSLIQIGGRVGRS
MDRPTGDLLFFHDGLNASIKKAIKEIQMMNKEAGLZ

ID117 870bp

ATGCAAATTCAAAAAAGTTTTAAGGGGCAGTCTCCCTATGGCAAGCTGTATCTAGTGGCAACGCCGATTGGCAAT
CTAGATGATATGACTTTTCGTGCTATCCAGACCTTGAAAGAAGTGGACTGGATTGCTGCTGAGGATACGCGCAAT
ACAGGGCTTTTGCTCAAGCATTTTGACATTTCCACCAAGCAGATCAGTTTTCATGAGCACAATGCCAAGGAAAAA
ATTCCTGATTTGATTGGTTTCTTGAAAGCAGGGCAAAGTATTGCTCAGGTCTCTGATGCCGGTTTGCCTAGCATTT
CAGACCCTGGTCATGATTTAGTTAAGGCAGCTATTGAGGAAGAAATTGCAGTTGTGACAGTTCCAGGTGCCTCTGC
AGGAATTTCTGCCTTGATTGCCAGTGGTTTAGCGCCACAGCCACATATCTTTTACGGTTTTTTACCGAGAAAATCA
GGTCAGCAGAAGCAATTTTTTGGCTTGAAAAAAGATTATCCTGAAACACAGATTTTTTATGAATCACCTCATCGTG
TAGCAGACACGTTGGAAAATATGTTAGAAGTCTACGGTGACCGCTCCGTTGTCTTGGTCAGGGAATTGACCAAAA
TCTATGAAGAATACCAACGAGGTACTATCTCTGAGTTATTAGAAAGCATTGCTGAAACGCCACTCAAGGGCGAAT
GTCTTCTCATTGTTGAGGGTGCCAGTCAGGGTGTGGAGGAAAAGGACGAGGAAGACTTGTTCGTAGAAATTCAAA
CCCGCATCCAGCAAGGTGTGAAGAAAAACCAAGCTATCAAGGAAGTCGCTAAGATTTACCAGTGGAATAAAAGTC
AGCTCTACGCTGCCTACCACGACTGGGAAGAAAAACAATAA

MQIQKSFKGQSPYGKLYLVATPIGNLDDMTFRAIQTLKEVDWIAAEDTRNTGLLLKHFDISTKQISFHEHNAKEKIPDLI
GFLKAGQSIAQVSDAGLPSISDPGHDLVKAAIEEEIAVVTVPGASAGISALIASGLAPQPHIFYGFLPRKSGQQKQFFGLKK
DYPETQIFYESPHRVADTLENMLEVYGDRSVVLVRELTKIYEEYQRGTISELLESIAETPLKGECLLIVEGASQGVEEKDE
EDLFVEIQTRIQQGVKKNQAIKEVAKIYQWNKSQLYAAYHDWEEKQZ

ID118 345bp

ATGATAAAGAAAGGAAAGGGCTGTTTTATGGACAAAAAAGAATTATTTGACGCGCTGGATGATTTTTCCCAACAA
TTATTGGTAACCTTAGCCGATGTGGAAGCCATCAAGAAAAATCTCAAGAGCCTGGTAGAGGAAAATACAGCTCTT
CGCTTGGAAAATAGTAAGTTGCGAGAACGCTTGGGTGAGGTGGAAGCAGATGCTCCTGTCAAGGCCAAGCATGTT
CGCGAAAGTGTCCGTCGTATTTACCGTGATGGATTTCACGTATGTAATGATTTTTATGGACAACGTCGAGAGCAGG
ACGAAGAATGTATGTTTTGTGACGAGTTGTTATACAGGGAGTAA

MIKKGKGCFMDKKELFDALDDFSQQLLVTLADVEAIKKNLKSLVEENTALRLENSKLRERLGEVEADAPVKAKHVRES
VRRIYRDGFHVCNDFYGQRREQDEECMFCDELLYREZ

ID119 639bp

ATGTCAAAAGGATTTTTAGTCTCTCTTGAGGGACCAGAGGGAGCAGGCAAGACCAGTGTTTTAGAGGCTCTGCTA
CCAATTTTAGAGGAAAAAGGAGTAGAGGTGTTGACGACCCGTGAACCTGGCGGAGTCTTGATTGGGGAGAAGATT
CGGGAAGTGATTTTGGATCCAAGTCATACTCAGATGGATGCTAAAACAGAGCTACTTCTCTATATTGCCAGTCGCA
GACAGCATTTGGTGGAAAAAGTTCTTCCAGCCCTTGAAGCTGGCAAGTTGGTCATCATGGATCGTTTTATCGATAG
TTCTGTTGCCTATCAGGGATTTGGTCGTGGCTTAGATATTGAAGCCATTGACTGGCTCAATCAGTTTGCGACAGAT
GGCCTCAAACCCGATTTGACACTCTATTTTGACATCGAGGTGGAAGAAGGGCTGGCTCGTATTGCTGCTAATAGTG
ACCGCGAGGTTAATCGTTTGGATTTGGAAGGGTTGGACTTGCATAAAAAAGTTCGTCAAGGCTACCTTTCTCTTCT
GGATAAAGAGGGAAATCGCATTGTCAAGATTGATGCTAGTCTCCCTTTGGAGCAAGTTGTGGAAACTACCAAGGC
TGTCTTGTTTGACGGAATGGGCTTGGCCAAATGA

MSKGFLVSLEGPEGAGKTSVLEALLPILEEKGVEVLTTREPGGVLIGEKIREVILDPSHTQMDAKTELLLYIASRRQHLVE
KVLPALEAGKLVIMDRFIDSSVAYQGFGRGLDIEAIDWLNQFATDGLKPDLTLYFDIEVEEGLARIAANSDREVNRLDL
EGLDLHKKVRQGYLSLLDKEGNRIVKIDASLPLEQVVETTKAVLFDGMGLAKZ

ID120 408bp

ATGGTAGAACAAAGAAAATCAATTACCATGAAAGATGTTGCTTTAGAAGCAGGAGTTAGTGTTGGAACTGTTTCA
CGTGTAATTAATAAAGAAAAAGGCATTAAAGAAGTAACTTTGAAAAAAGTGGAACAAGCGATTAAAACTTTGAAT
TACATTCCAGATTACTACGCTAGAGGAATGAAAAAAAATCGAACAGAAACGATTGCAATCATTGTACCAAGTATC
TGGCATCCCTTCTTTTCAGAATTTGCTATGCATGTGGAAAATGAAGTCTATAAGAGAAATAACAAATTACTCTTAT
GTTCTATCAATGGTACAAATAGAGAGCAAGACTATCTGGAGATGTTGCGTCATAATAAAGTTGATGGAGTGGTTG
CCATTACCTATAGGCCAATTGAACATTACTTGACGTCAGGAATTCCCTTTGTTAGTATTGACCGCACATACTCAGA
GATTGCCATTCCTTGTGTTTCA

MVEQRKSITMKDVALEAGVSVGTVSRVINKEKGIKEVTLKKVEQAIKTLNYIPDYYARGMKKNRTETIAIIVPSIWHPFF
SEFAMHVENEVYKRNNKLLLCSINGTNREQDYLEMLRHNKVDGVVAITYRPIEHYLTSGIPFVSIDRTYSEIAIPCVS

ID121 285bp

ATGAATATATTTAGAACAAAGAATGTTAGTTTAGATAAAACAGAGATGCATAGGCATTTGAAGTTATGGGATTTG
ATTTTGCTGGGTATCGGAGCCATGGTAGGGACAGGCGTCTTTACAATCACAGGTACTGCAGCTGCAACACTTGCTG
GCCCAGCCCTAGTGATTTCAATCGTTATTTCTGCCTTGTGTGTGGGATTATCAGCCCTCTTTTTTGCAGAATTTGCC
TCGCGAGTACCCGCTACAGGAGGTGCCTATAGTTACCTCTATGCTATCTTAGGAGAATTCCCTGCCTGGTTGGCTG
GTTGGTTAACCATGATGGAGTTCATGACAGCCATATCAGGCGTAGCTTCGGGTTGGGCAGCTTATTTTAA

MNIFRTKNVSLDKTEMHRHLKLWDLILLGIGAMVGTGVFTITGTAAATLAGPALVISIVISALCVGLSALFFAEFASRVP
ATGGAYSYLYAILGEFPAWLAGWLTMMEFMTAISGVASGWAAYF

ID124 1311bp

ATGAAATCAAGAGTAAAGGAAACGAGTATGGATAAAATTGTGGTTCAAGGTGGCGATAATCGTCTGGTAGGAAGC
GTGACGATCGAGGGAGCAAAAAATGCAGTCTTACCCTTGTTGGCAGCGACTATTCTAGCAAGTGAAGGAAAGACC
GTCTTGCAGAATGTTCCGATTTTGTCGGATGTCTTTATTATGAATCAGGTAGTTGGTGGTTTGAATGCCAAGGTTG
ACTTTGATGAGGAAGCTCATCTTGTCAAGGTGGATGCTACTGGCGACATCACTGAGGAAGCCCCTTACAAGTATG
TCAGCAAGATGCGCGCCTCCATCGTTGTATTAGGGCCAATCCTTGCCCGTGTGGGTCATGCCAAGGTATCCATGCC
AGGTGGTTGTACGATTGGTAGCCGTCCTATTGATCTTCATTTGAAAGGTCTGGAAGCTATGGGGGTTAAGATTAGT
CAGACAGCTGGTTACATCGAAGCCAAGGCAGAACGCTTGCATGGTGCTCATATCTATATGGACTTTCCAAGTGTTG
GTGCAACGCAGAACTTGATGATGGCAGCGACTCTGGCTGATGGGGTGACAGTGATTGAGAATGCTGCGCGTGAGC
CTGAGATTGTTGACTTAGCCATTCTCCTTAATGAAATGGGAGCCAAGGTCAAAGGTGCTGGTACAGAGACTATAA
CCATTACTGGTGTTGAGAAACTTCATGGTACGACTCACAATGTAGTCCAAGACCGTATCGAAGCAGGAACCTTTAT
GGTAGCTGCTGCCATGACTGGTGGTGATGTCTTGATTCGAGACGCTGTCTGGGAGCACAACCGTCCCTTGATTGCC
AAGTTACTTGAAATGGGTGTTGAAGTAATTGAAGAAGACGAAGGAATTCGTGTTCGTTCTCAACTAGAAAATCTA
AAAGCTGTTCATGTGAAAACCTTGCCCCACCCAGGATTTCCAACAGATATGCAGGCTCAATTTACAGCCTTGATGA
CAGTTGCAAAAGGCGAATCAACCATGGTGGAGACAGTTTTCGAAAATCGTTTCCAAACCTAGAAGAGATGCGCCG
CATGGGCTTGCATTCTGAGATTATCCGTGATACAGCTCGTATTGTTGGTGGACAGCCTTTGCAGGGAGCAGAAGTT
CTTTCAACTGACCTTCGTGCCAGTGCGGCCTTGATTTTGACAGGTTTGGTAGCACAGGGAGAAACTGTGGTCGGTA
AATTGGTTCACTTGGATAGAGGTTACTACGGTTTCCATGAGAAGTTGGCGCAGCTAGGTGCTAAGATTCAGCGGAT
TGAGGCAAGTGATGAAGATGAATAA

MKSRVKETSMDKIVVQGGDNRLVGSVTIEGAKNAVLPLLAATILASEGKTVLQNVPILSDVFIMNQVVGGLNAKVDFD
EEAHLVKVDATGDITEEAPYKYVSKMRASIVVLGPILARVGHAKVSMPGGCTIGSRPIDLHLKGLEAMGVKISQTAGYIE
AKAERLHGAHIYMDFPSVGATQNLMMAATLADGVTVIENAAREPEIVDLAILLNEMGAKVKGAGTETITITGVEKLHG
TTHNVVQDRIEAGTFMVAAAMTGGDVLIRDAVWEHNRPLIAKLLEMGVEVIEEDEGIRVRSQLENLKAVHVKTLPHP
GFPTDMQAQFTALMTVAKGESTMVETVFENRFQHLEEMRRMGLHSEIIRDTARIVGGQPLQGAEVLSTDLRASAALIL
TGLVAQGETVVGKLVHLDRGYYGFHEKLAQLGAKIQRIEASDEDEZ

ID125 1101bp

ATGTTATTAGCGTCAACAGTAGCCTTGTCATTTGCCCCAGTATTGGCAACTCAAGCAGAAGAAGTTCTTTGGACTG
CACGTAGTGTTGAGCAAATCCAAAACGATTTGACTAAAACGGACAACAAAACAAGTTATACCGTACAGTATGGTG
ATACTTTGAGCACCATTGCAGAAGCCTTGGGTGTAGATGTCACAGTGCTTGCGAATCTGAACAAAATCACTAATAT
GGACTTGATTTTCCCAGAAACTGTTTTGACAACGACTGTCAATGAAGCAGAAGAAGTAACAGAAGTTGAAATCCA
AACACCTCAAGCAGACTCTAGTGAAGAAGTGACAACTGCGACAGCAGATTTGACCACTAATCAAGTGACCGTTGA
TGATCAAACTGTTCAGGTTGCAGACCTTTCTCAACCAATTGCAGAAGTTACAAAGACAGTGATTGCTTCTGAAGAA
GTGGCACCATCTACGGGCACTTCTGTCCCAGAGGAGCAAACGACCGAAACAACTCGCCCAGTTGCAGAAGAAGCT
CCTCAGGAAACGACTCCAGCTGAGAAGCAGGAAACACAAACAAGCCCTCAAGCTGCATCAGCAGTGGAAGCAAC
TACAACAAGTTCAGAAGCAAAAGAAGTAGCATCATCAAATGGAGCTACAGCAGCAGTTTCTACTTATCAACCAGA
AGAAACGAAAGTAATTTCAACAACTTACGAGGCTCCAGCTGCGCCCGATTATGCTGGACTTGCAGTAGCAAAATC
TGAAAATGCAGGTCTTCAACCACAAACAGCTGCCTTTAAWGAAGAAATTGCTAACTTGTTTGGCATTACATCCTTT
AGTGGTTATCGTCCAGGAGACAGTGGAGATCACGGAAAAGGTTTGGCTATCGACTTTATGGTACCAGAACGTTCA
GAATTAGGGGATAAGATTGCGGAATATGCTATTCAAAATATGGCCAGCCGTGGCATTAGTTACATCATCTGGAAA
CAACGTTTCTATGCTCCATTCGATAGCAAATATGGGCCAGCTAACACTTGGAACCCAATGCCAGACCGTGGTAGT
GTGACAGAAAATCACTATGATCACGTTCACGTTTCAATGAATGGATAA

MLLASTVALSFAPVLATQAEEVLWTARSVEQIQNDLTKTDNKTSYTVQYGDTLSTIAEALGVDVTVLANLNKITNMDL
IFPETVLTTTVNEAEEVTEVEIQTPQADSSEEVTTATADLTTNQVTVDDQTVQVADLSQPIAEVTKTVIASEEVAPSTGTS
VPEEQTTETTRPVAEEAPQETTPAEKQETQTSPQAASAVEATTTSSEAKEVASSNGATAAVSTYQPEETKVISTTYEAPA
APDYAGLAVAKSENAGLQPQTAAFKKKLLTCLALHPLVVIVQETVEITEKVWLSTLWYQNVQNZGIRLRNMLFKIWPA
VALVTSSGNNVSMLHSIANMGQLTLGTQCQTVVVZQKITMITFTFQZMD

ID126 1281bp

TTGTTTAAGAAAAATAAAGACATTCTTAATATTGCATTGCCAGCTATGGGTGAAAACTTTTTGCAGATGCTAATGG
GAATGGTGGACAGTTATTTGGTTGCTCATTTAGGATTGATAGCTATTTCAGGGGTTTCAGTAGCTGGTAATATTAT
CACCATTTATCAGGCGATTTTCATCGCTCTGGGAGCTGCTATTTCCAGTGTTATTTCAAAAAGCATAGGGCAGAAA
GACCAGTCGAAGTTGGCCTATCATGTGACTGAGGCGTTGAAGATTACCTTACTATTAAGTTTCCTTTTAGGATTTT
TGTCCATCTTCGCTGGGAAAGAGATGATAGGACTTTTGGGGACGGAGAGGGATGTAGCTGAGAGTGGTGGACTGT
ATCTATCTTTGGTAGGCGGATCGATTGTTCTCTTAGGTTTAATGACTAGTCTAGGAGCCTTGATTCGTGCAACGCA
TAATCCACGTCTGCCTCTCTATGTTAGTTTTTTATCCAATGCCTTGAATATTCTTTTTTCAAGTCTAGCTATTTTTGT
TCTGGATATGGGGATAGCTGGTGTTGCTTGGGGGACAATTGTGTCTCGTTTGGTTGGTCTTGTGATTTTGTGGTCAC
AATTAAAACTGCCTTATGGGAAGCCAACTTTTGGTTTAGATAAGGAACTGTTGACCTTGGCTTTACCAGCAGCTGG
AGAGCGACTTATGATGAGGGCTGGAGATGTAGTGATCATTGCCTTGGTCGTTTCTTTTGGGACGGAGGCAGTTGCT
GGGAATGCAATCGGAGAAGTCTTGACCCAGTTTAACTATATGCCTGCCTTTGGCGTCGCTACGGCAACGGTCATG
CTGTTGGCCCGAGCAGTTGGAGAGGATGATTGGAAAAGAGTTGCTAGTTTGAGTAAACAAACCTTTTGGCTTTCTC
TGTTCCTCATGTTGCCCCTGTCCTTTAGTATATATGTCTTGGGTGTACCATTAACTCATCTCTATACGACTGATTCT
CTAGCGGTGGAGGCTAGTGTTCTAGTGACACTGTTTTCACTACTTGGGACCCCTATGACGACAGGAACAGTCATCT
ATACGGCAGTCTGGCAGGGATTAGGAAATGCACGCCTCCCTTTTTATGCGACAAGTATAGGAATGTGGTGTATCC
GCATTGGGACAGGATATCTGATGGGGATTGTGCTTGGTTGGGGCTTGCCTGGTATTTGGGCAGGGTCTCTCTTGGA
TAATGGTTTTCGCTGGTTATTTCTACGCTATCGTTACCAGCGCTATATGAGCTTGAAAGGATAG

LFKKNKDILNIALPAMGENFLQMLMGMVDSYLVAHLGLIAISGVSVAGNIITIYQAIFIALGAAISSVISKSIGQKDQSKLA
YHVTEALKITLLLSFLLGFLSIFAGKEMIGLLGTERDVAESGGLYLSLVGGSIVLLGLMTSLGALIRATHNPRLPLYVSFL
SNALNILFSSLAIFVLDMGIAGVAWGTIVSRLVGLVILWSQLKLPYGKPTFGLDKELLTLALPAAGERLMMRAGDVVIIA
LVVSFGTEAVAGNAIGEVLTQFNYMPAFGVATATVMLLARAVGEDDWKRVASLSKQTFWLSLFLMLPLSFSIYVLGVP
LTHLYTTDSLAVEASVLVTLFSLLGTPMTTGTVIYTAVWQGLGNARLPFYATSIGMWCIRIGTGYLMGIVLGWGLPGIW
AGSLLDNGFRWLFLRYRYQRYMSLKGZ

ID127 894bp

GTGGGAAGAATTATCAGAGCAGGTGTAAAGATGGAACATCTTGGAAAAGTATTTCGTGAATTTCGAACAAGTGGA
AATTATTCTTTAAAGGAAGCAGCAGGCGAATCCTGCTCTACCTCTCAGTTATCTCGCTTTGAGCTTGGGGAGTCTG
ACCTGGCAGTCTCCCGTTTCTTTGAGATTTTGGATAACATTCATGTAACAATCGAAAATTTCATGGATAAGGCAAG
GAATTTTCATAATCATGAACATGTGTCTATGATGGCACAGATTATCCCACTTTACTATTCAAACGATATTGCAGGT
TTTCAAAAGCTTCAAAGAGAACAACTTGAAAAGTCTAAGAGTTCGACGACTCCCCTTTATTTTGAGCTGAACTGGA
TTTTGCTACAAGGTCTGATTTGTCAAAGAGATGCGAGTTATGATATGAAGCAGGATGATTTGGGTAAGGTAGCAG
ATTATCTCTTCAAAACAGAAGAATGGACCATGTATGAGTTGATTCTTTTCGGTAACCTCTATAGTTTCTACGATGT
AGACTATGTCACTCGGATTGGTAGAGAAGTTATGGAGAGGGAGGAATTTTACCAAGAGATTAGTCGCCATAAGAG
ATTAGTGTTGATTTTGGCCCTCAATTGTTACCAGCATTGTTTAGAGCATTCTTCTTTTTATAATGCCAACTATTTTG
AGGCTTATACAGAGAAGATTATTGACAAAGGTATTAAGCTTTATGAGCGTAATGTTTTCCATTATTTAAAAGGTTT
TGCCTTATATCAAAAAGGACAGTGTAAAGAAGGCTGTAAGCAGATGCAAGAGGCCATGCATATTTTTGATGTGTT
AGGTCTTCCAGAGCAAGTAGCCTATTATCAGGAACACTACGAAAAATTTGTCAAAAGTTAA

VGRIIRAGVKMEHLGKVFREFRTSGNYSLKEAAGESCSTSQLSRFELGESDLAVSRFFEILDNIHVTIENFMDKARNFHN
HEHVSMMAQIIPLYYSNDIAGFQKLQREQLEKSKSSTTPLYFELNWILLQGLICQRDASYDMKQDDLGKVADYLFKTEE
WTMYELILFGNLYSFYDVDYVTRIGREVMEREEFYQEISRHKRLVLILALNCYQHCLEHSSFYNANYFEAYTEKIIDKGI
KLYERNVFHYLKGFALYQKGQCKEGCKQMQEAMHIFDVLGLPEQVAYYQEHYEKFVKSZ

TABLE 3

ID1 1068bp

ATGTCTAACATTCAAAACATGTCCCTGGAGGACATCATGGGAGAGCGCTTTGGTCGCTACTCCAAGTACATTATTC
AAGACCGGGCTTTGCCAGATATTCGTGATGGGTTGAAGCCGGTTCAGCGCCGTATTCTTTATTCTATGAATAAGGA
TAGCAATACTTTTGACAAGAGCTACCGTAAGTCGGCCAAGTCAGTCGGGAACATCATGGGGAATTTCCACCCACA
CGGGGATTCTTCTATCTATGATGCCATGGTTCGTATGTCACAGAACTGGAAAAATCGTGAGATTCTAGTTGAAATG
CACGGTAATAACGGTTCTATGGACGGAGATCCTCCTGCGGCTATGCGTTATACTGAGGCACGTTTGTCTGAAATTG
CAGGCTACCTTCTTCAGGATATCGAGAAAAAGACAGTTCCTTTTGCATGGAACTTTGACGATACGGAGAAAGAAC
CAACGGTCTTGCCAGCAGCCTTTCCAAACCTCTTGGTCAATGGTTCGACTGGGATTTCGGCTGGTTATGCCACAGA
CATTCCTCCCCATAATTTAGCTGAGGTCATAGATGCTGCAGTTTACATGATTGACCACCCAACTGCAAAGATTGAT
AAACTCATGGAATTCTTGCCTGGACCAGACTTCCCTACAGGGGCTATTATTCAGGGTCGTGATGAAATCAAGAAA
GCTTATGAGACTGGGAAAGGGCGCGTGGTTGTTCGTTCCAAGACTGAAATTGAAAAGCTAAAAGGTGGTAAGGAA
CAAATCGTTATTATTGAGATTCCTTATGAAATCAATAAGGCCAATCTAGTCAAGAAAATCGATGATGTTCGTGTTA
ATAACAAGGTAGCTGGGATTGCTGAGGTTCGTGATGAGTCTGACCGTGATGGTCTTCGTATCGCTATCGAACTTAA
GAAAGACGCTAATACTGAGCTTGTTCTCAACTACTTATTTAAGTACACCGACCTACAAATCAACTACAACTTTAAT
ATGGTGGCGATTGACAATTTCACACCTCGTCAGGTTGGATTGTTCCAATCCTGTCTAGCTATATCGCTCACCGTCG
AGAAGTGA

MSNIQNMSLEDIMGERFGRYSKYIIQDRALPDIRDGLKPVQRRILYSMNKDSNTFDKSYRKSAKSVGNIMGNFHPHGDS
SIYDAMVRMSQNWKNREILVEMHGNNGSMDGDPPAAMRYTEARLSEIAGYLLQDIEKKTVPFAWNFDDTEKEPTVLP
AAFPNLLVNGSTGISAGYATDIPPHNLAEVIDAAVYMIDHPTAKIDKLMEFLPGPDFPTGAIIQGRDEIKKAYETGKGRV
VVRSKTEIEKLKGGKEQIVIIEIPYEINKANLVKKIDDVRVNNKVAGIAEVRDESDRDGLRIAIELKKDANTELVLNYLFK
YTDLQINYNFNMVAIDNFTPRQVGLFQSCLAISLTVEKZ

ID12 684bp

ATGCCGACATTAGAAATAGCACAAAAAAAACTGGAGTTCATTAAGAAGGCAGAAGAATATTACAATGCCTTGTGT
ACAAATATACAGTTGAGCGGAGATAAACTAAAAGTAATTTCCGTTACTTCTGTTAACCCTGGGGAAGGAAAAACA
ACTACTTCCATAAATATAGCATGGTCGTTTGCGCGTGCAGGCTATAAAACTCTTTTGATCGATGGCGATACTCGAA
ATTCAGTTATGTTAGGAGTTTTTAAATCTCGTGAAAAAATTACAGGGCTAACAGAATTTTTATCTGGGACAGCTGA
TTTATCTCACGGTTTATGTGATACAAATATTGAAAATTTATTTGTAGTTCAATCGGGATCTGTATCACCAAACCCT
ACAGCCTTGTTACAAAGTAAAAATTTTAATGATATGATTGAAACATTGCGTAAATATTTTGATTATATCATTATTG
ATACACCGCCTATTGGAATTGTTATTGATGCGGCAATTATCACTCAAAAGTGTGATGCGTCCATCTTGGTAACAGC
AACAGGTGAGGCGAATAAACGTGATATCCAAAAAGCGAAACAACAATTAAAACAAACAGGGAAACTGTTCCTAG
GAGTTGTTTTAAATAAATTGGATATCTCGGTTAATAAGTATGGAGTTTACGGTTCCTATGGAAATTATGGTAAAAA
ATAA

MPTLEIAQKKLEFIKKAEEYYNALCTNIQLSGDKLKVISVTSVNPGEGKTTTSINIAWSFARAGYKTLLIDGDTRNSVML
GVFKSREKITGLTEFLSGTADLSHGLCDTNIENLFVVQSGSVSPNPTALLQSKNFNDMIETLRKYFDYIIIDTPPIGIVIDAA
IITQKCDASILVTATGEANKRDIQKAKQQLKQTGKLFLGVVLNKLDISVNKYGVYGSYGNYGKKZ

ID13 1182bp

ATGGAGGCAAATATGAAACATCTAAAAACATTTTACAAAAAATGGTTTCAATTATTAGTCGTTATCGTCATTAGCT
TTTTTAGTGGAGCCTTGGGTAGTTTTTCAATAACTCAACTAACTCAAAAAAGTAGTGTAAACAACTCTAACAACAA
TAGTACTATTACACAAACTGCCTATAAGAACGAAAATTCAACAACACAGGCTGTTAACAAAGTAAAAGATGCTGT
TGTTTCTGTTATTACTTATTCGGCAAACAGACAAAATAGCGTATTTGGCAATGATGATACTGACACAGATTCTCAG
CGAATCTCTAGTGAAGGATCTGGAGTTATTTATAAAAAGAATGATAAAGAAGCTTACATCGTCACCAACAATCAC
GTTATTAATGGCGCCAGCAAAGTAGATATTCGATTGTCAGATGGGACTAAAGTACCTGGAGAAATTGTCGGAGCT
GACACTTTCTCTGATATTGCTGTCGTCAAAATCTCTTCAGAAAAAGTGACAACAGTAGCTGAGTTTGGTGATTCTA
GTAAGTTAACTGTAGGAGAAACTGCTATTGCCATCGGTAGCCCGTTAGGTTCTGAATATGCAAATACTGTCACTCA
AGGTATCGTATCCAGTCTCAATAGAAATGTATCCTTAAAATCGGAAGATGGACAAGCTATTTCTACAAAAGCCAT
CCAAACTGATACTGCTATTAACCCAGGTAACTCTGGCGGCCCACTGATCAATATTCAAGGGCAGGTTATCGGAAT
TACCTCAAGTAAAATTGCTACAAATGGAGGAACATCTGTAGAAGGTCTTGGTTTCGCAATTCCTGCAAATGATGCT
ATCAATATTATTGAACAGTTAGAAAAAAACGGAAAAGTGACGCGTCCAGCTTTGGGAATCCAGATGGTTAATTTA
TCTAATGTGAGTACAAGCGACATCAGAAGACTCAATATTCCAAGTAATGTTACATCTGGTGTAATTGTTCGTTCGG
TACAAAGTAATATGCCTGCCAATGGTCACCTTGAAAAATACGATGTAATTACAAAAGTAGATGACAAAGAGATTG
CTTCATCAACAGACTTACAAAGTGCTCTTTACAACCATTCTATCGGAGACACCATTAAGATAACCTACTATCGTAA
CGGGAAAGAAGAAACTACCTCTATCAAACTTAACAAGAGTTCAGGTGATTTAGAATCTTAA

MEANMKHLKTFYKKWFQLLVVIVISFFSGALGSFSITQLTQKSSVNNSNNNSTITQTAYKNENSTTQAVNKVKDAVVSV
ITYSANRQNSVFGNDDTDTDSQRISSEGSGVIYKKNDKEAYIVTNNHVINGASKVDIRLSDGTKVPGEIVGADTFSDIAV
VKISSEKVTTVAEFGDSSKLTVGETAIAIGSPLGSEYANTVTQGIVSSLNRNVSLKSEDGQAISTKAIQTDTAINPGNSGGP

LINIQGQVIGITSSKIATNGGTSVEGLGFAIPANDAINIIEQLEKNGKVTRPALGIQMVNLSNVSTSDIRRLNIPSNVTSGVIV
RSVQSNMPANGHLEKYDVITKVDDKEIASSTDLQSALYNHSIGDTIKITYYRNGKEETTSIKLNKSSGDLESZ

ID15 939bp

ATGGCAGAAATTTATCTAGCAGGTGGTTGTTTTTGGGGCCTAGAGGAATATTTTTCACGCATTTCTGGAGTGCTAG
AAACCAGTGTTGGCTACGCTAATGGTCAAGTCGAAACGACCAATTACCAGTTGCTCAAGGAAACAGACCATGCAG
AAACGGTCCAAGTGATTTACGATGAGAAGGAAGTGTCACTCAGAGAGATTTTACTTTATTATTTCCGAGTTATCGA
TCCTCTATCTATCAATCAACAAGGGAATGACCGTGGTCGCCAATATCGAACTGGGATTTATTATCAGGATGAAGC
AGATTTGCCAGCTATCTACACAGTGGTGCAGGAGCAGGAACGCATGCTGGGTCGAAAGATTGCAGTAGAAGTGGA
GCAATTACGCCACTACATTCTGGCTGAAGACTACCACCAAGACTATCTCAGGAAGAATCCTTCAGGTTACTGTCAT
ATCGATGTGACCGATGCTGATAAGCCATTGATTGATGCAGCAAACTATGAAAAGCCTAGTCAAGAGGTGTTGAAG
GCCAGTCTATCTGAAGAGTCTTATCGTGTCACACAAGAAGCTGCTACAGAGGCTCCATTTACCAATGCCTATGACC
AAACCTTTGAAGAGGGGATTTATGTAGATATTACGACAGGTGAGCCACTCTTTTTTGCCAAGGATAAGTTTGCTTC
AGGTTGTGGTTGGCCAAGTTTTAGCCGTCCGATTTCCAAAGAGTTGATTCATTATTACAAGGATCTGAGCCATGGA
ATGGAGCGAATTGAAGTTCGTTCTCGTTCAGGCAGTGCTCACTTGGGTCATGTTTTCACAGATGGACCGCGGGAGT
TAGGCGGCCTCCGTTACTGTATCAATTCTGCTTCTTTACGCTTTGTGGCCAAGGATGAGATGGAAAAAGCAGGATA
TGGCTATCTATTGCCTTACTTAAACAAATAA

MAEIYLAGGCFWGLEEYFSRISGVLETSVGYANGQVETTNYQLLKETDHAETVQVIYDEKEVSLREILLYYFRVIDPLSI
NQQGNDRGRQYRTGIYYQDEADLPAIYTVVQEQERMLGRKIAVEVEQLRHYILAEDYHQDYLRKNPSGYCHIDVTDA
DKPLIDAANYEKPSQEVLKASLSEESYRVTQEAATEAPFTNAYDQTFEEGIYVDITTGEPLFFAKDKFASGCGWPSFSRPI
SKELIHYYKDLSHGMERIEVRSRSGSAHLGHVFTDGPRELGGLRYCINSASLRFVAKDEMEKAGYGYLLPYLNKZ

ID17 870bp

ATGAAGATTATTGTACCTGCAACCAGTGCCAATATCGGGCCAGGTTTTGACTCGGTCGGTGTAGCTGTAACCAAGT
ATCTTCAAATTGAGGTCTGCGAAGAACGAGATGAGTGGCTGATTGAACACCAGATTGGCAAATGGATTCCACATG
ACGAGCGTAATCTCTTGCTCAAAATCGCTTTGCAAATTGTACCAGACTTGCAACCAAGACGCTTGAAAATGACCA
GTGATGTCCCTTTGGCGCGCGGTTTGGGTTCTTCCAGCTCGGTTATCGTTGCTGGGATTGAACTAGCCAACCAACT
GGGTCAACTCAACTTATCAGACCATGAAAAATTGCAGTTAGCGACCAAGATTGAAGGGCATCCTGACAATGTGGC
TCCAGCCATTTATGGTAATCTCGTTATTGCAAGTTCTGTTGAAGGGCAAGTCTCTGCTATCGTAGCAGACTTTCCA
GAGTGTGATTTTCTAGCTTACATTCCAAACTATGAATTACGTACTCGCGACAGCCGTAGTGTCTTGCCTAAAAAAT
TGTCTTATAAGGAAGCTGTTGCTGCAAGTTCTATCGCCAATGTAGCGGTTGCTGCCTTGTTGGCAGGAGACATGGT
GACCGCTGGGCAAGCAATCGAGGGAGACCTCTTCCATGAGCGCTATCGTCAGGACTTGGTAAGAGAATTTGCGAT
GATTAAGCAAGTGACCAAAGAAAATGGGGCCTATGCAACCTACCTTTCTGGTGCTGGGCCGACAGTTATGGTTCT
GGCTTCTCATGACAAGATGCCAACAATTAAGGCAGAATTGGAAAAGCAACCTTTCAAAGGAAAACTGCATGACTT
GAGAGTTGATACCCAAGGTGTCCGTGTAGAAGCAAAATAA

MKIIVPATSANIGPGFDSVGVAVTKYLQIEVCEERDEWLIEHQIGKWIPHDERNLLLKIALQIVPDLQPRRLKMTSDVPLA
RGLGSSSSVIVAGIELANQLGQLNLSDHEKLQLATKIEGHPDNVAPAIYGNLVIASSVEGQVSAIVADFPECDFLAYIPNY
ELRTRDSRSVLPKKLSYKEAVAASSIANVAVAALLAGDMVTAGQAIEGDLFHERYRQDLVREFAMIKQVTKENGAYAT
YLSGAGPTVMVLASHDKMPTIKAELEKQPFKGKLHDLRVDTQGVRVEAKZ

ID20 564bp

ATGAAATATCACGATTACATCTGGGATTTAGGTGGAACTTTACTGGATAATTATGAAACTTCAACAGCTGCATTTG
TTGAAACATTGGCACTGTATGGTATCACACAAGACCATGACAGTGTCTATCAAGCTTTAAAGGTTTCTACTCCTTT
TGCGATTGAGACATTCGCTCCCAATTTAGAGAATTTTTTAGAAAAGTACAAGGAAAATGAAGCCAGAGAGCTTGA
ACACCCGATTTTATTTGAAGGAGTTTCTGACCTATTGGAAGACATTTCAAATCAAGGTGGCCGTCATTTTTTGGTC
TCTCATCGAAATGATCAGGTTTTGGAAATTTTAGAAAAAACCTCTATAGCAGCTTATTTTACAGAAGTGGTGACTT
CTAGCTCAGGCTTTAAGAGAAAGCCAAATCCCGAATCCATGCTTTATTTAAGAGAAAAGTATCAGATTAGCTCTG
GTCTTGTCATTGGTGATCGGCCGATTGATATCGAAGCAGGTCAAGCTGCAGGACTTGATACCCACTTGTTTACCAG
TATCGTGAATTTAAGACAAGTATTAGACATATAA

MKYHDYIWDLGGTLLDNYETSTAAFVETLALYGITQDHDSVYQALKVSTPFAIETFAPNLENFLEKYKENEARELEHPI
LFEGVSDLLEDISNQGGRHFLVSHRNDQVLEILEKTSIAAYFTEVVTSSSGFKRKPNPESMLYLREKYQISSGLVIGDRPID
IEAGQAAGLDTHLFTSIVNLRQVLDIZ

ID21 1875bp

ATGACAGAAGAAATCAAAAATCTGCAGGCACAGGATTATGATGCCAGTCAAATTCAAGTTTTAGAGGGCTTAGAG
GCTGTTCGTATGCGTCCAGGGATGTACATTGGATCAACCTCAAAAGAAGGTCTTCACCATCTAGTCTGGGAAATTG
TTGATAACTCAATTGACGAGGCCTTGGCAGGATTTGCCAGCCATATTCAAGTTTTTATTGAGCCAGATGATTCGAT
TACTGTTGTGGATGATGGGCGTGGTATCCCAGTCGATATTCAGGAAAAAACAGGCCGTCCTGCTGTTGAGACCGT

CTTTACAGTCCTTCACGCTGGAGGAAAGTTCGGCGGTGGTGGATACAAGGTTTCAGGTGGTCTTCACGGGGTGGG
GTCGTCAGTAGTTAATGCCCTTTCCACTCAATTAGACGTTCATGTTCACAAAAATGGTAAGATTCATTACCAAGAA
TACCGTCGTGGTCATGTTGTCGCAGATCTTGAAATAGTTGGAGATACGGATAAAACAGGAACAACTGTTCACTTC
ACACCGGACCCAAAAATCTTCACTGAAACAACAATCTTTGATTTTGATAAATTAAATAAACGGATTCAAGAGTTG
GCCTTTCTAAATCGCGGTCTTCAAATTTCAATTACAGATAAGCGCCAAGGTTTGGAACAAACCAAGCATTATCATT
ATGAAGGTGGGATTGCTAGTTACGTTGAATATATCAACGAGAACAAGGATGTAATCTTTGATACACCAATCTATA
CAGACGGTGAGATGGATGATATCACAGTTGAGGTAGCCATGCAGTACACAACTGGTTACCATGAAAATGTCATGA
GTTTCGCCAATAATATTCATACCCATGAAGGTGGAACACATGAACAAGGTTTCCGTACAGCCTTGACACGTGTTAT
CAACGATTATGCTCGTAAAAATAAGTTACTGAAAGACAATGAAGATAATTTAACAGGGGAAGATGTTCGCGAAGG
CTTAACTGCAGTTATCTCAGTTAAACACCCAAATCCACAGTTTGAAGGACAAACCAAGACCAAATTGGGAAATAG
CGAAGTGGTCAAGATTACCAATCGCCTCTTCAGTGAAGCTTTCTCCGATTTCCTCATGGAAAATCCACAGATTGCC
AAACGTATCGTAGAAAAAGGAATTTTGGCTGCCAAGGCTCGTGTGGCTGCCAAGCGTGCGCGTGAAGTCACACGT
AAAAAATCTGGTTTGGAAATTTCCAACCTTCCAGGGAAACTAGCAGACTGTTCTTCTAATAACCCTGCTGAAACAG
AACTCTTCATCGTCGAAGGAGACTCAGCTGGTGGATCAGCCAAATCTGGTCGTAACCGTGAGTTTCAGGCTATCCT
TCCAATTCGCGGTAAGATTTTGAACGTTGAAAAAGCAAGTATGGATAAGATTCTAGCCAACGAAGAAATTCGTAG
TCTTTTCACAGCCATGGGAACAGGATTTGGCGCAGAATTTGATGTTTCGAAAGCCCGTTACCAAAAACTCGTTTTG
ATGACCGATGCCGATGTCGATGGAGCCCACATTCGTACCCTTCTTTTAACCTTGATTTATCGTTATATGAAACCAA
TCCTAGAAGCTGGTTATGTTTATATTGCCCAACCACCAATCTATGGTGTCAAGGTTGGAAGCGAGATTAAAGAATA
TATCCAGCCGGGTGCAGATCAAGAAATCAAACTCCAAGAAGCTTTAGCCCGTTATAGTGAAGGTCGTACCAAACC
GACTATTCAGCGTTATAAGGGGCTAGGTGAAATGGACGATCATCAGCTGTGGGAAACAACCATGGATCCCGAACA
TCGCTTGATGGCTAGAGTTTCTGTAGATGATGTGCAGAAGCAGATAAAATCTTTGATATGTTGA

MTEEIKNLQAQDYDASQIQVLEGLEAVRMRPGMYIGSTSKEGLHHLVWEIVDNSIDEALAGFASHIQVFIEPDDSITVVD
DGRGIPVDIQEKTGRPAVETVFTVLHAGGKFGGGGYKVSGGLHGVGSSVVNALSTQLDVHVHKNGKIHYQEYRRGHV
VADLEIVGDTDKTGTTVHFTPDPKIFTETTIFDFDKLNKRIQELAFLNRGLQISITDKRQGLEQTKHYHYEGGIASYVEYI
NENKDVIFDTPIYTDGEMDDITVEVAMQYTTGYHENVMSFANNIHTHEGGTHEQGFRTALTRVINDYARKNKLLKDN
EDNLTGEDVREGLTAVISVKHPNPQFEGQTKTKLGNSEVVKITNRLFSEAFSDFLMENPQIAKRIVEKGILAAKARVAAK
RAREVTRKKSGLEISNLPGKLADCSSNNPAETELFIVEGDSAGGSAKSGRNREFQAILPIRGKILNVEKASMDKILANEEI
RSLFTAMGTGFGAEFDVSKARYQKLVLMTDADVDGAHIRTLLLTLIYRYMKPILEAGYVYIAQPPIYGVKVGSEIKEYI
QPGADQEIKLQEALARYSEGRTKPTIQRYKGLGEMDDHQLWETTMDPEHRLMARVSVDDVQKQIKSLICZ

ID54 1446bp

ATGAGTAGACGTTTTAAAAAATCACGTTCACAGAAAGTGAAGCGAAGTGTTAATATAGTTTTGCTGACTATTTATT
TATTGTTAGTTTGTTTTTTATTGTTCTTAATCTTTAAGTACAATATCCTTGCTTTTAGATATCTTAATCTAGTGGTAA
CTGCGTTAGTCCTACTAGTTGCCTTGGTAGGGCTACTCTTGATTATCTATAAAAAAGCTGAAAAGTTTACTATTTTT
CTGTTGGTGTTCTCTATCCTTGTCAGCTCTGTGTCGCTCTTTGCAGTACAGCAGTTTGTTGGACTGACCAATCGTTT
AAATGCGACTTCTAATTACTCAGAATATTCAATCAGTGTCGCTGTTTTAGCAGATAGTGAGATCGAAAATGTTACG
CAACTGACGAGTGTGACAGCACCGACTGGGACTAATAATGAAAATATTCAGAAATTACTAGCTGATATCAAGTCA
AGTCAGAATACCGATTTGACGGTCAACCAGAGTTCGTCTTACTTGGCAGCTTACAAGAGTTTGATTGCAGGGGAG
ACTAAGGCCATTGTCCTAAATAGTGTCTTTGAAAACATCATCGAGTCAGAGTATCCAGACTACGCATCGAAGATA
AAAAAGATTTATACTAAGGGATTCACTAAAAAAGTAGAAGCTCCTAAGACGTCTAAGAGTCAGTCTTTCAATATC
TATGTTAGTGGAATTGACACCTATGGTCCTATTAGTTCGGTGTCGCGATCAGATGTCAACATCCTGATGACTGTCA
ATCGAGATACCAAGAAAATCCTCTTGACCACAACGCCACGTGATGCCTATGTACCAATCGCAGATGGTGGAAATA
ATCAAAAAGATAAATTGACTCATGCGGGCATTTATGGAGTTGATTCGTCCATTCACACCTTAGAAAATCTCTATGG
AGTGGATATCAATTACTATGTGCGATTGAACTTCACTTCGTTTTTGAAATTGATTGATTTGTTGGGTGGAATTGATG
TTTATAATGATCAAGAATTTACTGCCCATACGAATGGAAAGTATTACCCTGCAGGCAATGTTCATCTTGATTCAGA
ACAGGCTCTCGGTTTTGTTCGTGAGCGCTACTCCCTAGCAGATGGCGATCGTGACCGCGGGCGCCATCAACAAAA
GGTGATTGTGGCTATCCTTCAAAAATTAACGTCAACCGAAGTGCTGAAAAATTATAGTACGATCATTAATAGCTTG
CAAGATTCTATCCAAACAAATATGCCACTTGAGACCATGATAAATTTGGTCAATGCTCAGTTAGAAAGTGGAGGG
AATTATAAAGTAAATTCTCAAGATTTAAAAGGGACAGGTCGGATGGATCTTCCTTCTTATGCAATGCCAGACAGTA
ACCTCTATGTGATGGAAATAGATGATAGTAGTTTAGCTGTAGTTAAAGCAGCTATACAGGATGTGATGGAGGGTA
GATGA

MSRRFKKSRSQKVKRSVNIVLLTIYLLLVCFLLFLIFKYNILAFRYLNLVVTALVLLVALVGLLLIIYKKAEKFTIFLLVFS
ILVSSVSLFAVQQFVGLTNRLNATSNYSEYSISVAVLADSEIENVTQLTSVTAPTGTNNENIQKLLADIKSSQNTDLTVNQ
SSSYLAAYKSLIAGETKAIVLNSVFENIIESEYPDYASKIKKIYTKGFTKKVEAPKTSKSQSFNIYVSGIDTYGPISSVSRSDV
NILMTVNRDTKKILLTTTPRDAYVPIADGGNNQKDKLTHAGIYGVDSSIHTLENLYGVDINYYVRLNFTSFLKLIDLLGG
IDVYNDQEFTAHTNGKYYPAGNVHLDSEQALGFVRERYSLADGDRDRGRHQQKVIVAILQKLTSTEVLKNYSTIINSLQ
DSIQTNMPLETMINLVNAQLESGGNYKVNSQDLKGTGRMDLPSYAMPDSNLYVMEIDDSSLAVVKAAIQDVMEGRZ

ID55 732bp

ATGATAGACATCCATTCGCATATCGTTTTTGATGTAGATGACGGTCCCAAGTCAAGAGAGGAAAGCAAGGCTCTC
TTGGCAGAATCCTACAGACAGGGGGTGCGAACCATTGTTTCTACCTCTCACCGTCGCAAGGGCATGTTTGAAACTC
CGGAAGAGAAGATAGCAGAAAACTTTCTTCAGGTTCGGGAAATAGCTAAGGAAGTGGCGAGTGACTTGGTCATTG
CTTACGGGGCTGAAATTTATTACACACCAGATGTTCTGGATAAGCTGGAAAAAAAGCGGATTCCGACCCTCAATG
ATAGTCGTTATGCCTTGATAGAGTTTAGTATGAACACTCCTTATCGCGATATTCATAGCGCCTTGAGCAAGATCTT
GATGTTGGGAATTACTCCAGTCATTGCCCACATTGAGCGCTATGATGCTCTTGAAAATAATGAAAAACGCGTTCGA
GAACTGATCGATATGGGCTGTTACACGCAAGTAAATAGTTCACATGTCCTCAAACCCAAACTTTTTGGCGAACGTT
ATAAATTCATGAAAAAAAGAGCTCAGTATTTTTTAGAGCAGGATTTGGTTCATGTCATTGCAAGTGATATGCACAA
TCTAGACGGTAGACCTCCTCATATGGCAGAAGCATATGACCTTGTTACCCAAAAATACGGAGAAGCGAAGGCTCA
GGAACTTTTTATAGACAATCCTCGAAAAATTGTAATGGATCAACTAATTTAG

MIDIHSHIVFDVDDGPKSREESKALLAESYRQGVRTIVSTSHRRKGMFETPEEKIAENFLQVREIAKEVASDLVIAYGAEI
YYTPDVLDKLEKKRIPTLNDSRYALIEFSMNTPYRDIHSALSKILMLGITPVIAHIERYDALENNEKRVRELIDMGCYTQV
NSSHVLKPKLFGERYKFMKKRAQYFLEQDLVHVIASDMHNLDGRPPHMAEAYDLVTQKYGEAKAQELFIDNPRKIVM
DQLIZ

ID58 3990bp

TTGATTTATATAATCGCTATCAATATAACAATGCAATCAGGAGGTTTTGCAATGAAACATGAAAAACAACAGCGT
TTTTCTATTCGTAAATACGCTGTAGGAGCAGCTTCTGTTCTAATTGGATTTGCCTTCCAAGCACAGACTGTTGCAG
CCGATGGAGTTACTCCTACTACTACAGAAAACCAACCGACCATCCATACGGTTTCTGATTCCCCTCAATCATCCGA
AAATCGGACTGAGGAAACACCTAAAGCAGTGCTTCAACCAGAAGCTCCAAAAACTGTAGAAACAGAAACTCCAG
CTACTGATAAGGTAGCTAGTCTTCCAAAAACAGAAGAAAAACCACAAGAGGAAGTTAGTTCAACTCCTAGTGATA
AAGCAGAAGTGGTAACTCCAACTTCTGCTGAAAAAGAAACTGCTAATAAAAAGGCAGAAGAAGCTAGCCCTAAA
AAGGAAGAAGCGAAAGAGGTTGATTCTAAAGAGTCAAATACAGACAAGACTGACAAGGATAAACCAGCTAAAAA
AGATGAAGCGAAAGCAGAGGCTGACAAACCGGCAACAGAGGCAGGAAAGGAACGTGCTGCAACTGTAAATGAAA
AACTAGCGAAAAAGAAAATTGTTTCTATTGATGCTGGACGTAAATATTTCTCACCAGAACAGCTCAAGGAAATCA
TCGATAAAGCGAAACATTATGGCTACACTGATTTACACCTATTAGTCGGAAATGATGGACTCCGTTTCATGTTGGA
CGATATGAGCATCACAGCTAACGGCAAGACCTATGCCAGTGACGATGTCAAACGCGCCATTGAAAAAGGTACAAA
TGATTATTACAACGATCCAAACGGCAATCACTTAACAGAAAGTCAAATGACAGATCTGATTAACTATGCCAAAGA
TAAAGGTATCGGTCTCATTCCGACAGTAAATAGTCCTGGACACATGGATGCGATTCTCAATGCCATGAAAGAATT
GGGAATCCAAAACCCTAACTTTAGCTATTTTGGGAAGAAATCAGCCCGTACTGTCGATCTTGACAACGAACAAGC
TGTCGCTTTTACAAAAGCCCTTATCGACAAGTATGCTGCTTATTTCGCGAAAAAGACTGAAATCTTCAACATCGGA
CTTGATGAATATGCCAATGATGCGACAGATGCTAAAGGTTGGAGTGTGCTTCAAGCTGATAAATACTATCCAAAC
GAAGGCTACCCTGTAAAAGGCTATGAAAAATTTATTGCCTACGCCAATGACCTCGCTCGTATTGTAAAATCGCAC
GGTCTCAAACCAATGGCTTTTAACGACGGTATCTACTACAATAGCGACACAAGCTTTGGTAGTTTTGACAAAGAC
ATCATCGTTTCTATGTGGACTGGTGGTTGGGGAGGCTACGATGTCGCTTCTTCTAAACTACTAGCTGAAAAAGGTC
ACCAAATCCTTAATACCAATGATGCTTGGTACTACGTTCTTGGACGAAACGCTGATGGCCAAGGCTGGTACAATCT
CGATCAGGGGCTCAATGGTATTAAAAACACACCAATCACTTCTGTACCAAAAACAGAAGGAGCTGATATCCCAAT
CATCGGTGGTATGGTAGCTGCTTGGGCTGACACTCCATCTGCACGTTATTCACCATCACGCCTCTTCAAACTCATG
CGTCATTTTGCAAATGCCAACGCTGAATACTTCGCAGCTGATTATGAATCTGCAGAGCAAGCACTTAACGAGGTA
CCAAAAGACCTGAACCGTTATACTGCAGAAAGCGTCACGGCCGTAAAAGAAGCTGAAAAAGCTATTCGCTCTCTC
GATAGCAACCTTAGCCGTGCCCAACAAGATACGATTGATCAAGCCATTGCTAAACTTCAAGAAACTGTCAACAAC
TTGACCCTCACGCCTGAAGCTCAAAAAGAAGAAGAAGCTAAACGTGAGGTTGAAAAACTTGCCAAAAACAAGGT
AATCTCAATCGATGCTGGACGCAAATACTTTACTCTGAACCAGCTCAAACGCATCGTAGACAAGGCCAGTGAGCT
CGGATATTCTGATGTCCATCTCCTTCTAGGAAATGACGGACTTCGCTTTCTACTCGATGATATGACCATTACTGCC
AACGGAAAAACCTATGCTAGTGATGACGTTAAAAAAGCTATTATCGAAGGAACTAAAGCTTACTACGACGATCCA
AACGGTACTGCACTAACACAGGCAGAAGTAACAGAGCTAATTGAATACGCTAAATCTAAGGACATCGGTCTCATC
CCAGCTATTAACAGTCCAGGTCACATGGATGCTATGCTGGTTGCCATGGAAAAATTAGGTATTAAAAATCCTCAA
GCCCACTTTGATAAAGTTTCAAAAACAACTATGGACTTGAAAAACGAAGAAGCGATGAACTTTGTAAAAGCCCTC
ATCGGTAAATACATGGACTTCTTTGCAGGTAAAACAAAGATTTTCAACTTTGGTACTGACGAATACGCCAACGAT
GCGACTAGTGCCCAAGGCTGGTACTACCTCAAGTGGTATCAACTCTATGGCAAATTTGCCGAATATGCCAACACC
CTCGCAGCTATGGCCAAAGAAAGAGGGCTTCAACCAATGGCCTTCAACGATGGCTTCTACTATGAAGACAAGGAC
GATGTTCAGTTTGACAAAGATGTCTTGATTTCTTACTGGTCTAAAGGCTGGTGGGGATATAACCTCGCATCACCTC
AATACCTAGCAAGCAAAGGCTATAAATTCTTGAATACCAACGGTGACTGGTACTACATTCTTGGTCAAAAACCAG
AAGATGGTGGTGGTTTCCTCAAGAAAGCTATTGAGAATACTGGAAAAACACCATTCAATCAACTAGCTTCTACCA
AATATCCTGAAGTAGATCTTCCAACAGTCGGAAGTATGCTTTCAATCTGGGCAGATAGACCAAGCGCTGAATACA
AGGAAGAGGAAATCTTTGAACTCATGACTGCCTTTGCAGACCACAACAAAGACTACTTTCGTGCTAATTATAATG
CTCTCCGCGAAGAATTAGCTAAAATTCCTACAAACTTAGAAGGATATAGTAAAGAAAGTCTTGAGGCCCTTGACG
CAGCTAAAACAGCTCTAAATTACAACCTCAACCGTAATAAACAAGCTGAGCTTGACACGCTTGTAGCCAACCTAA
AAGCCGCTCTTCAAGGCCTCAAACCAGCTGTAACTCATTCAGGAAGCCTAGATGAAAATGAAGTGGCTGCCAATG
TTGAAACCAGACCAGAACTCATCACAAGAACTGAAGAAATTCCATTTGAAGTTATCAAGAAAGAAAATCCTAACC
TCCCAGCCGGTCAGGAAAATATTATCACAGCAGGAGTCAAAGGTGAACGAACTCATTACATCTCTGTACTCACTG
AAAATGGAAAAACAACAGAAACAGTCCTTGATAGCCAGGTAACCAAAGAAGTTATAAACCAAGTGGTTGAAGTT
GGCGCTCCTGTAACTCACAAGGGTGATGAAAGTGGTCTTGCACCAACTACTGAGGTAAAACCTAGACTGGATATC

CAAGAAGAAGAAATTCCATTTACCACAGTGACTTGTGAAAATCCACTCTTACTCAAAGGAAAAACACAAGTCATT
ACTAAGGGCGTCAATGGACATCGTAGCAACTTCTACTCTGTGAGCACTTCTGCCGATGGTAAGGAAGTGAAAACA
CTTGTAAATAGTGTCGTAGCACAGGAAGCCGTTACTCAAATAGTCGAAGTCGGAACTATGGTAACACATGTAGGC
GATGAAAACGGACAAGCCGCTATTGCTGAAGAAAAACCAAAACTAGAAATCCCAAGCCAACCAGCTCCATCAAC
TGCTCCTGCTGAGGAAAGCAAAGTTCTTCCTCAAGATCCAGCTCCTGTGGTAACAGAGAAAAAACTTCCTGAAAC
AGGAACTCACGATTCTGCAGGACTAGTAGTCGCAGGACTCATGTCCACACTAGCAGCCTATGGACTCACTAAAAG
AAAAGAAGACTAA

MIYIIAINITMQSGGFAMKHEKQQRFSIRKYAVGAASVLIGFAFQAQTVAADGVTPTTTENQPTIHTVSDSPQSSENRTEE
TPKAVLQPEAPKTVETETPATDKVASLPKTEEKPQEEVSSTPSDKAEVVTPTSAEKETANKKAEEASPKKEEAKEVDSKE
SNTDKTDKDKPAKKDEAKAEADKPATEAGKERAATVNEKLAKKKIVSIDAGRKYFSPEQLKEIIDKAKHYGYTDLHLL
VGNDGLRFMLDDMSITANGKTYASDDVKRAIEKGTNDYYNDPNGNHLTESQMTDLINYAKDKGIGLIPTVNSPGHMD
AILNAMKELGIQNPNFSYFGKKSARTVDLDNEQAVAFTKALIDKYAAYFAKKTEIFNIGLDEYANDATDAKGWSVLQA
DKYYPNEGYPVKGYEKFIAYANDLARIVKSHGLKPMAFNDGIYYNSDTSFGSFDKDIIVSMWTGGWGGYDVASSKLLA
EKGHQILNTNDAWYYVLGRNADGQGWYNLDQGLNGIKNTPITSVPKTEGADIPIIGGMVAAWADTPSARYSPSRLFKL
MRHFANANAEYFAADYESAEQALNEVPKDLNRYTAESVTAVKEAEKAIRSLDSNLSRAQQDTIDQAIAKLQETVNNLT
LTPEAQKEEEAKREVEKLAKNKVISIDAGRKYFTLNQLKRIVDKASELGYSDVHLLLGNDGLRFLLDDMTITANGKTYA
SDDVKKAIIEGTKAYYDDPNGTALTQAEVTELIEYAKSKDIGLIPAINSPGHMDAMLVAMEKLGIKNPQAHFDKVSKTT
MDLKNEEAMNFVKALIGKYMDFFAGKTKIFNFGTDEYANDATSAQGWYYLKWYQLYGKFAEYANTLAAMAKERGL
QPMAFNDGFYYEDKDDVQFDKDVLISYWSKGWWGYNLASPQYLASKGYKFLNTNGDWYYILGQKPEDGGGFLKKAI
ENTGKTPFNQLASTKYPEVDLPTVGSMLSIWADRPSAEYKEEEIFELMTAFADHNKDYFRANYNALREELAKIPTNLEG
YSKESLEALDAAKTALNYNLNRNKQAELDTLVANLKAALQGLKPAVTHSGSLDENEVAANVETRPELITRTEEIPFEVI
KKENPNLPAGQENIITAGVKGERTHYISVLTENGKTTETVLDSQVTKEVINQVVEVGAPVTHKGDESGLAPTTEVKPRL
DIQEEEIPFTTVTCENPLLLKGKTQVITKGVNGHRSNFYSVSTSADGKEVKTLVNSVVAQEAVTQIVEVGTMVTHVGDE
NGQAAIAEEKPKLEIPSQPAPSTAPAEESKVLPQDPAPVVTEKKLPETGTHDSAGLVVAGLMSTLAAYGLTKRKEDZ

ID122 825bp

ATGAACAAAAAAACAAGACAGACACTAATCGGACTGCTAGTGTTATTGCTTTTGTCTACAGGGAGCTATTATATC
AAGCAGATGCCGTCGGCACCTAATAGTCCCAAAACCAATCTTAGTCAGAAAAAACAAGCGTCTGAAGCTCCTAGT
CAAGCATTGGCAGAGAGTGTCTTAACAGACGCAGTCAAGAGTCAAATAAAGGGGAGTCTGGAGTGGAATGGCTC
AGGTGCTTTTATCGTCAATGGTAATAAAACAAATCTAGATGCCAAGGTTTCAAGTAAGCCCTACGCTGACAATAA
AACAAAGACAGTGGGCAAGGAAACTGTTCCAACCGTAGCTAATGCCCTCTTGTCTAAGGCCACTCGTCAGTACAA
GAATCGTAAAGAAACTGGGAATGGTTCAACTTCTTGGACTCCTCCAGGTTGGCATCAGGTCAAGAATCTAAAGGG
CTCTTATACCCATGCAGTCGATAGAGGTCATTTGTTAGGCTATGCCTTAATCGGTGGTTTGGATGGTTTTGATGCCT
CAACAAGCAATCCTAAAAACATTGCTGTTCAGACAGCCTGGGCAAATCAGGCACAAGCCGAGTATTCGACTGGTC
AAAACTACTATGAAAGCAAGGTGCGTAAAGCCTTGGACCAAAACAAGCGTGTCCGTTACCGTGTAACCCTTTACT
ACGCTTCAAACGAGGATTTAGTTCCCTCAGCTTCACAGATTGAAGCCAAGTCTTCGGATGGAGAATTGGAATTCA
ATGTTCTAGTTCCCAATGTTCAAAAGGGACTTCAACTGGATTACCGAACTGGAGAAGTAACTGTAACTCAGTAA

MNKKTRQTLIGLLVLLLLSTGSYYIKQMPSAPNSPKTNLSQKKQASEAPSQALAESVLTDAVKSQIKGSLEWNGSGAFIV
NGNKTNLDAKVSSKPYADNKTKTVGKETVPTVANALLSKATRQYKNRKETGNGSTSWTPPGWHQVKNLKGSYTHAV
DRGHLLGYALIGGLDGFDASTSNPKNIAVQTAWANQAQAEYSTGQNYYESKVRKALDQNKRVRYRVTLYYASNEDL
VPSASQIEAKSSDGELEFNVLVPNVQKGLQLDYRTGEVTVTQZ

ID123 225bp

GTGCTAAGATTCAGCGGATTGAGGCAAGTGATGAAGATGAATAAGAAATCAAGCTACGTAGTCAAGCGTTTACTT
TTAGTCATCATAGTACTGATTTTAGGTACTCTGGCTCTAGGAATCGGTTTAATGGTAGGTTATGGAATCTTGGGCA
AGGGTCAAGATCCATGGGCTATCCTGTCTCCAGCAAAATGGCAGGAATTGATTCATAAATTTACAGGAAATTAG

VLRFSGLRQVMKMNKKSSYVVKRLLLVIIVLILGTLALGIGLMVGYGILGKGQDPWAILSPAKWQELIHKFTGNZ

## Claims

1. A *Streptococcus pneumoniae* protein or polypeptide having a sequence selected from those shown in table 1.

2. *A Streptococcus pneumoniae* protein or polypeptide having a sequence selected from those shown in table 2.

3. A protein or polypeptide as claimed in claim 1 or claim 2 provided in substantially pure form.

4. A protein or polypeptide which is substantially identical to one defined in any one of claims 1 to 3.

5. A homologue or derivative of a protein or polypeptide as defined in any one of claims 1 to 4.

6. An antigenic and/or immunogenic fragment of a protein or polypeptide as defined
in Tables 1-3.

7. A nucleic acid molecule comprising or consisting of a sequence which is:
(i) any of the DNA sequences set out in Table 1 or their RNA equivalents;
(ii) a sequence which is complementary to any of the sequences of (i);
(iii) a sequence which codes for the same protein or polypeptide, as those sequences of (i) or (ii) ;
(iv) a sequence which is substantially identical with any of those of (i), (ii) and (iii);
(v) a sequence which codes for a homologue, derivative or fragment of a protein as defined in Table 1.

8. A nucleic acid molecule comprising or consisting of a sequence which is:
(i) any of the DNA sequences set out in Table 2 or their RNA equivalents;
(ii) a sequence which is complementary to any of the sequences of (i);
(iii) a sequence which codes for the same protein or polypeptide, as those sequences of (i) or (ii);
(iv) a sequence which is substantially identical with any of those of (i), (ii) and (iii);
(v) a sequence which codes for a homologue, derivative or fragment of a protein as defined in Table 2.

9. The use of a protein or polypeptide having a sequence selected from those shown in Tables 1-3, or homologues, derivatives and/or fragments thereof, as an immunogen and/or antigen.

10. An immunogenic and/or antigenic composition comprising one or more proteins or polypeptides selected from those whose sequences are shown in Tables 1-3, or homologues or derivatives thereof, and/or fragments of any of these.

11. An immunogenic and/or antigenic composition as claimed in claim 10 which is a vaccine or is for use in a diagnostic assay.

12. A vaccine as claimed in claim 11 which comprises one or more additional components selected from excipients, diluents, adjuvants or the like.

13. A vaccine composition comprising one or more nucleic acid sequences as defined in Tables 1-3.

14. A method for the detection/diagnosis of *S.pneumoniae* which comprises the step of bringing into contact a sample to be tested with at least one protein or polypeptide as defined in Tables 1-3, or homologue, derivative or fragment thereof.

15. An antibody capable of binding to a protein or polypeptide as defined in Tables 1-3, or for a homologue, derivative or fragment thereof.

16. An antibody as defined in claim 15 which is a monoclonal antibody.

17. A method for the detection/diagnosis of *S.pneumoniae* which comprises the step of bringing into contact a sample to be tested and at least one antibody as define din claim 15 or claim 16.

18. A method for the detection/diagnosis of *S.pneumoniae* which comprises the step of bringing into contact a sample to be tested with at least one nucleic acid sequence as defined in claim 7 or claim 8.

19. A method of determining whether a protein or polypeptide as defined in Tables 1-3 represents a potential anti-microbial target which comprises inactivating said protein or polypeptide and determining whether *S.pneumoniae* is still viable.

20. The use of an agent capable of antagonising, inhibiting or otherwise interfering with the function or expression of a protein or polypeptide as defined in Tables 1-3 in the manufacture of a medicament for use in the treatment or prophylaxis of *S.pneumoniae* infection
